(19) Europäisches Patentamt
European Patent Office
Office européen des brevets

(11) Veröffentlichungsnummer: **0 148 128**
**A2**

(12) # EUROPÄISCHE PATENTANMELDUNG

(21) Anmeldenummer: 84810652.2

(22) Anmeldetag: 20.12.84

(51) Int. Cl.⁴: C 07 D 499/00
C 07 F 7/18, A 61 K 31/43
//C07F9/65, C07D205/08,
C07D277/16, C07D233/54

(30) Priorität: 30.12.83 CH 6989/83
08.02.84 CH 589/84
27.06.84 CH 3093/84

(43) Veröffentlichungstag der Anmeldung:
10.07.85 Patentblatt 85/28

(84) Benannte Vertragsstaaten:
AT BE CH DE FR GB IT LI LU NL SE

(71) Anmelder: CIBA-GEIGY AG
Postfach
CH-4002 Basel(CH)

(72) Erfinder: Lang, Marc, Dr.
Rue des Ormes 6
F-68170 Rixheim(FR)

(54) **Neue optisch aktive Penem-Verbindungen.**

(57) 2-Heterocyclyl-6-hydroxyniederalkyl-2-penem-Verbindungen der Formel

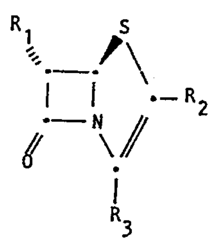

(I),

worin $R_1$ durch Hydroxy oder geschütztes Hydroxy substituiertes Niederalkyl ist, $R_2$ einen über ein Ringstickstoffatom an den Penemrest gebundenen ungesättigten Azaheterocyclylrest darstellt, und $R_3$ Carboxyl oder funktionell abgewandeltes Carboxyl ist, optische Isomere von Verbindungen der Formel I, Mischungen dieser optischen Isomere, und Salze von solchen Verbindungen der Formel I, die eine salzbildende Gruppe aufweisen, besitzen antibiotische Eigenschaften. Die Verbindungen werden nach an sich bekannten Verfahren hergestellt.

EP 0 148 128 A2

Croydon Printing Company Ltd

0148128

CIBA-GEIGY AG

Basel (Schweiz)

4-14723/1-3/+

Neue optisch aktive Penem-Verbindungen

Die Erfindung betrifft neue optisch aktive Penem-Verbindungen, Verfahren zu ihrer Herstellung, pharmazeutische Präparate, die solche Verbindungen enthalten, und ihre Verwendung zur Herstellung von pharmazeutischen Präparaten oder als pharmakologisch wirksame Verbindungen.

Die Erfindung betrifft insbesondere 2-Heterocyclyl-6-hydroxyniederalkyl-2-penem-Verbindungen der Formel

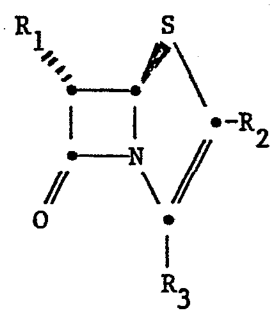

(I),

worin $R_1$ durch Hydroxy oder geschütztes Hydroxy substituiertes Niederalkyl ist, $R_2$ einen über ein Ringstickstoffatom an den Penemrest gebundenen ungesättigten Azaheterocyclylrest darstellt, und $R_3$ Carboxyl oder funktionell abgewandeltes Carboxyl ist, optische Isomere von Verbindungen der Formel I, Mischungen dieser optischen Isomere, und Salze von solchen Verbindungen der Formel I, die eine salzbildende Gruppe aufweisen.

Die vor- und nachstehend verwendeten Definitionen haben im Rahmen der vorliegenden Beschreibung vorzugsweise die folgenden Bedeutungen:

Ein über ein Ringstickstoffatom an den Penem-Rest gebundener, ungesättigter Azaheterocyclyl-Rest $R_2$ ist insbesondere ein entsprechender monocyclischer oder polycyclischer, insbesondere monocyclischer oder bicyclischer, ferner auch tricyclischer, Azaheterocyclylrest, wie ein gegebenenfalls partiell gesättigter monocyclischer 5-gliedriger Heteroaryl-Rest mit 1 bis 4 Ringstickstoffatomen, z.B. ein entsprechender aza-, diaza-, triaza- oder tetraza-cyclischer Rest aromatischen Charakters oder ein entsprechender Dihydro-Rest, oder ein entsprechender partiell gesättigter monocyclischer 6-gliedriger Heteroaryl-Rest mit 1 bis 3 Ringstickstoffatomen, wie ein entsprechender aza-, diaza- oder triaza-cyclischer Rest, z.B. ein entsprechender Dihydro- oder Tetrahydro-Rest, ferner gegebenenfalls partiell gesättigte Benzo-, Dibenzo-, Pyrido- oder Pyrimido-Derivate solcher 5- oder 6-gliedrigen Reste. Entsprechende Azaheterocyclylreste $R_2$ sind beispielsweise gegebenenfalls partiell gesättigtes Pyrrolyl, Diazolyl, Triazolyl oder Tetrazolyl, ferner partiell gesättigtes Pyridyl, Pyrimidyl, Pyridazinyl, Pyrazinyl oder Triazinyl, sowie gegebenenfalls im Pyrrolyl-, Diazolyl- bzw. Triazolylteil und/oder im Benzo-, Pyrido- bzw. Pyrimidoteil partiell gesättigtes Benzopyrrolyl, Benzodiazolyl, Benzotriazolyl, Pyridopyrrolyl, Pyridodiazolyl, Pyridotriazolyl, Pyrimidopyrrolyl, Pyrimidodiazolyl, Pyrimidotriazolyl oder Dibenzopyrrolyl, ferner im Pyridyl-, Pyrimidyl-, Pyridazinyl-, Pyrazinyl- bzw. Triazinylteil partiell gesättigtes und im Benzo-, Pyrido- bzw. Pyrimidoteil gegebenenfalls partiell gesättigtes Benzopyridyl, Benzopyrimidyl, Benzopyridazinyl, Benzopyrazinyl, Benzotriazinyl, Pyridopyridyl, Pyridopyrimidyl, Pyridopyridazinyl, Pyridopyrazinyl, Pyridotriazinyl, Pyrimidopyridyl, Pyrimidopyrimidyl, Pyrimidopyridazinyl, Pyrimidopyrazinyl, Pyrimidotriazinyl, Dibenzopyridyl oder Dibenzopyrazinyl.

Reste $R_2$ sind unsubstituiert oder können durch gegebenenfalls veräthertes oder verestertes Hydroxy, z.B. Hydroxy, Niederalkoxy, Niederalkanoyloxy oder Halogen, gegebenenfalls veräthertes Mercapto, z.B. Mercapto, Niederalkylthio oder Phenylthio, Niederalkyl, Hydroxyniederalkyl, Niederalkanoyloxyniederalkyl, Niederalkoxyniederalkyl, Carboxyniederalkyl, Niederalkoxycarbonylniederalkyl, Carbamoylniederalkyl, Carbamoyloxyniederalkyl, Halogenniederalkyl, Niederalkylthioniederalkyl, gegebenenfalls N-niederalkyliertes Aminoniederalkyl, z.B. Aminoniederalkyl, Niederalkylaminoniederalkyl oder Diniederalkylaminoniederalkyl, Niederalkanoylaminoniederalkyl, Amino-carboxy-niederalkyl, Amino-niederalkoxycarbonyl-niederalkyl, Sulfoniederalkyl, gegebenenfalls substituiertes Amino, z.B. Amino, Niederalkylamino, Diniederalkylamino, Niederalkylenamino oder Acylamino, wie Niederalkanoylamino, gegebenenfalls funktionell abgewandeltes Carboxyl oder Sulfo, z.B. Carboxyl, verestertes Carboxyl, wie Niederalkoxycarbonyl, gegebenenfalls substituiertes Carbamoyl, wie Carbamoyl oder N-mono- oder N,N-diniederalkyliertes Carbamoyl, Cyano, Sulfo oder Sulfamoyl, gegebenenfalls durch Niederalkyl, Nitro, Niederalkoxy und/oder Halogen substituiertes Phenyl, Cycloalkyl, Nitro, Imino, Oxo und/oder Oxido substituiert, wie insbesondere mono- oder auch poly-, in erster Linie mono- oder disubstituiert, sein. Reste $R_2$ sind in erster Linie an den Ringkohlenstoffatomen substituiert, können jedoch auch an den Ringstickstoffatomen substituiert sein.

Funktionell abgewandeltes Carboxyl $R_3$ ist insbesondere unter physiologischen Bedingungen spaltbares, verestertes Carboxyl oder geschütztes Carboxyl $R_3'$.

Eine unter physiologischen Bedingungen spaltbare veresterte Carboxylgruppe $R_3$ schützt die Verbindungen der Formel I vor Salzbildung im Magen-Darm-Trakt bei oraler Verabreichung, womit die vorzeitige Exkretion verhindert wird, und ist in erster Linie eine Acyloxymethoxycarbonylgruppe, worin Acyl z.B. den Rest einer organischen Carbonsäure, in erster Linie einer gegebenenfalls substituierten Niederalkancarbonsäure, bedeutet oder worin Acyloxymethyl den Rest eines Lactons bildet. Solche Gruppen sind z.B. Niederalkanoyloxymethoxycarbonyl, Aminoniederalkanoyloxymethoxycarbonyl, insbesondere α-Aminoniederalkanoyloxymethoxycarbonyl, 4-Crotonolactonyl und 4-Butyrolacton-4-yl. Weitere unter physiologischen Bedingungen spaltbare veresterte Carboxylgruppen $R_3$ sind z.B. 5-Indanyloxycar-

bonyl, Phthalidyloxycarbonyl, 1-Niederalkoxycarbonyloxyniederalkoxycarbonyl, 1-Niederalkoxyniederalkoxycarbonyl oder 2-Oxo-1,3-dioxolen-4-ylmethoxycarbonyl, welches in 5-Stellung des Dioxolenringes gegebenenfalls durch Niederalkyl oder Phenyl substituiert ist.

In der vorliegenden Beschreibung bedeutet der im Zusammenhang mit Definitionen von Gruppen bzw. Verbindungen verwendete Ausdruck "Nieder", dass die entsprechenden Gruppen bzw. Verbindungen, sofern nicht ausdrücklich anders definiert, 1 bis 7, bevorzugt 1 bis 4, Kohlenstoffatome enthalten.

Durch Hydroxy substituiertes Niederalkyl $R_1$ ist insbesondere in α-Stellung zum Penem-Ringgerüst durch Hydroxy substituiertes Niederalkyl und bedeutet z.B. 1-Hydroxyprop-1-yl, 2-Hydroxyprop-2-yl, 1-Hydroxybut-1-yl oder 2-Hydroxybut-2-yl und insbesondere Hydroxymethyl oder 1-Hydroxyäthyl.

Niederalkoxy ist z.B. Methoxy, ferner Aethoxy, n-Propyloxy, Isopropyloxy, n-Butyloxy, Isobutyloxy oder tert.-Butyloxy, sowie n-Pentyloxy, n-Hexyloxy oder n-Heptyloxy.

Niederalkanoyloxy ist z.B. Acetyloxy oder Propionyloxy.

Halogen ist z.B. Fluor, Chlor, Brom oder Jod.

Niederalkylthio ist z.B. Methylthio, Aethylthio, n-Propylthio, Isopropylthio oder n-Butylthio.

Niederalkyl ist z.B. Methyl, Aethyl, n-Propyl, Isopropyl, n-Butyl, Isobutyl, sek.-Butyl oder tert.-Butyl, ferner n-Pentyl, n-Hexyl oder n-Heptyl.

Hydroxyniederalkyl als Substituent eines Azaheterocyclyl-Restes $R_2$ ist z.B. Hydroxymethyl, 2-Hydroxyäthyl oder 2,3-Dihydroxypropyl.

Niederalkanoyloxyniederalkyl ist z.B. Acetoxymethyl oder 2-Acetoxyäthyl.

Niederalkoxyniederalkyl ist z.B. Methoxymethyl, 2-Methoxyäthyl, Aethoxymethyl oder 2-Aethoxyäthyl.

Carboxyniederalkyl ist z.B. Carboxymethyl, 1-Carboxy-, 2-Carboxy- oder 1,2-Dicarboxyäthyl.

Niederalkoxycarbonylniederalkyl ist z.B. Methoxycarbonylmethyl, Aethoxycarbonylmethyl oder 2-Methoxycarbonyläthyl.

Carbamoylniederalkyl ist z.B. Carbamoylmethyl oder 2-Carbamoyläthyl, während Carbamoyloxyniederalkyl z.B. Carbamoyloxymethyl oder 2-Carbamoyloxyäthyl ist.

Halogenniederalkyl ist z.B. Chlormethyl, Brommethyl, 2-Chloräthyl oder 2,2-Dichloräthyl.

Niederalkylthioniederalkyl ist z.B. Methylthiomethyl, Aethylthiomethyl, n-Propylthiomethyl oder 2-Methylthioäthyl.

Aminoniederalkyl ist z.B. Aminomethyl oder 2-Aminoäthyl, während Niederalkylaminoniederalkyl z.B. Methylaminomethyl, Aethylaminomethyl, 2-Methylaminoäthyl oder 2-Aethylaminoäthyl und Diniederalkylaminoniederalkyl z.B. Dimethylaminomethyl, 2-Dimethylaminoäthyl oder 2-Diäthylaminoäthyl ist.

Niederalkanoylaminoniederalkyl ist z.B. Acetaminomethyl, 2-Acetaminoäthyl oder Formylaminomethyl.

Amino-carboxy-niederalkyl ist z.B. 2-Amino-2-carboxy-äthyl oder auch 1-Amino-1-carboxymethyl, während Amino-niederalkoxycarbonyl-niederalkyl z.B. 2-Amino-2-methoxy-(oder 2-äthoxy)-carbonyläthyl ist.

Sulfoniederalkyl ist z.B. Sulfomethyl oder 2-Sulfoäthyl.

Niederalkylamino ist z.B. Methylamino, Aethylamino, n-Propylamino, Isopropylamino oder n-Butylamino, während Diniederalkylamino z.B. Dimethylamino, Diäthylamino, Di-n-propylamino oder Diisopropylamino bedeutet.

Niederalkylenamino weist insbesondere 4 bis 6 Kohlenstoffkettenglieder auf und bedeutet z.B. Pyrrolidino oder Piperidino.

Niederalkanoylamino ist z.B. Acetylamino oder Propionylamino.

Niederalkoxycarbonyl ist z.B. Methoxycarbonyl oder Aethoxycarbonyl.

N-Mono-niederalkyliertes Carbamoyl ist z.B. N-Methyl-, N-Aethyl-oder N-Propylcarbamoyl, während N,N-di-niederalkyliertes Carbamoyl z.B. N,N-Dimethyl- oder N,N-Diäthylcarbamoyl bedeutet.

Cycloalkyl enthält vorzugsweise 3 bis 8, in erster Linie 5 oder 6 Ringglieder und ist z.B. Cyclopentyl oder Cyclohexyl, ferner Cyclopropyl sowie Cycloheptyl.

Niederalkanoyloxymethoxycarbonyl ist z.B. Acetoxymethoxycarbonyl oder Pivaloyloxymethoxycarbonyl.

α-Aminoniederalkanoyloxymethoxycarbonyl ist z.B. Glycyloxymethoxycarbonyl, Valyloxymethoxycarbonyl oder Leucyloxymethoxycarbonyl.

1-Niederalkoxycarbonyloxyniederalkoxycarbonyl ist z.B. Aethoxycarbonyloxymethoxycarbonyl oder 1-Aethoxycarbonyloxyäthoxycarbonyl.

1-Niederalkoxyniederalkoxycarbonyl ist z.B. Methoxymethoxycarbonyl oder 1-Methoxyäthoxycarbonyl.

Bei einer 2-Oxo-1,3-dioxolen-4-ylmethoxy-Gruppe, welche in 5-Stellung des Dioxolenringes gegebenenfalls durch Niederalkyl oder Phenyl substituiert ist, handelt es sich vor allem um eine 5-Phenyl-und in erster Linie um eine 5-Methyl-2-oxo-1,3-dioxolen-4-ylmethoxy-Gruppe.

Entsprechende 5-gliedrige gegebenenfalls partiell gesättigte monocyclische Heteroarylreste $R_2$ sind z.B. gegebenenfalls z.B. durch Niederalkyl oder Halogen substituiertes Pyrrolyl oder Dihydropyrrolyl, z.B. 1-Pyrrolyl, 3-Methyl-1-pyrrolyl, 3,4-Dichlor-1-pyrrolyl, ferner 2,3- oder 2,5-Dihydro-1-pyrrolyl, gegebenenfalls z.B. durch Niederalkyl, Niederalkoxy, Aminoniederalkyl,N-niederalkyliertes Aminoniederalkyl, Niederalkanoylaminoniederalkyl, Amino-carboxyniederalkyl, Amino-niederalkoxycarbonyl-niederalkyl, Carboxyniederalkyl, Niederalkoxycarbonylniederalkyl, Carbamoylniederalkyl, Carbamoyloxyniederalkyl, Hydroxyniederalkyl, Niederalkanoyloxyniederalkyl, Halogenniederalkyl, Sulfoniederalkyl, Niederalkylthioniederalkyl, Amino, Niederalkylamino, Diniederalkylamino, Niederalkanoylamino oder Nitro substituiertes Diazolyl, wie Imidazolyl oder Pyrazolyl, z.B. 1-Imidazolyl, 1-Pyrazolyl, 4-Niederalkyl-1-pyrazolyl, 2-Amino-, 4-Niederalkoxy-, 4-Niederalkyl-, 4,5-Diniederalkyl-, 2-Aminoniederalkyl-, 4-Aminoniederalkyl-, 4-Niederalkanoylaminoniederalkyl-, 2-Hydroxyniederalkyl-, 4-Hydroxyniederalkyl-, 4-Niederalkanoyloxyniederalkyl-, 2- oder 4-Amino-carboxy-niederalkyl-, 4-Carbamoylniederalkyl-, 4-Carbamoyloxyniederalkyl-, 4-Halogenniederalkyl-, 4-Niederalkylthioniederalkyl-oder 2-Nitro-1-imidazolyl, 2-Niederalkylpyrazolio oder 3-Niederalkyl-imidazolio, gegebenenfalls z.B. durch Niederalkyl, Carboxyniederalkyl, Amino oder Phenyl substituiertes Triazolyl, wie 1H-1,2,3-Triazol-1-yl, 1H-1,2,3-Triazol-2-yl, 1H-1,2,4-Triazol-1-yl oder 1H-1,3,4-Triazol-1-yl, z.B. die entsprechenden unsubstituierten Reste, 4-oder 5-Methyl-1,2,3-triazol-1-yl, 3-Methyl- oder 3-Phenyl-1H-1,2,4-triazol-1-yl oder 1-(2-Methyl- oder 4-Methyl-1H-1,2,4-triazolio), oder gegebenenfalls z.B. durch Niederalkyl, Carboxyniederalkyl, Sulfoniederalkyl, Diniederalkylaminoniederalkyl, Amino oder gegebenenfalls durch Halogen substituiertes Phenyl substituiertes Tetrazolyl, wie

1H-Tetrazol-1-yl oder 2H-Tetrazol-2-yl, z.B. die entsprechenden unsubstituierten Reste, 5-Amino-, 5-Methyl-, 5-Carboxymethyl-, 5-Sulfomethyl-, 5-(2-Dimethylaminoäthyl)- oder 5-Phenyl-1H-tetrazol-1-yl, oder 5-Amino-, 5-Methyl-, 5-Carboxymethyl-, 5-Sulfomethyl-, 5-(2-Dimethylaminoäthyl)-oder 5-Phenyl-2H-tetrazol-2-yl.

Entsprechende 6-gliedrige partiell gesättigte monocyclische Heteroarylreste $R_2$ sind z.B. unsubstituiertes oder insbesondere z.B. durch Oxo und gegebenenfalls zusätzlich z.B. durch Halogen substituiertes Dihydro-1-pyridyl, wie 2H-1,2-Dihydro- oder 4H-1,4-Dihydro-1-pyridyl, z.B. 2-Oxo-2H-1,2-dihydro-1-pyridyl oder 4-Oxo-4H-1,4-dihydro-1-pyridyl, gegebenenfalls, insbesondere z.B. durch Oxo und gegebenenfalls zusätzlich z.B. durch Niederalkyl, Amino, Diniederalkylamino und/oder Carboxy substituiertes Dihydro- oder Tetrahydro-1-pyrimidyl, wie 2H-1,2-Dihydro-, 4H-1,4-Dihydro- oder 1,2,3,4-Tetrahydro-1-pyrimidyl, z.B. 2-Oxo-1,2-dihydro-1-pyrimidyl, 6-Methyl-, 5-Methyl-, 5-Carboxy- oder 6-Carboxy-2-oxo-1,2-dihydro-1-pyrimidyl, 4-Amino-2-oxo-1,2-dihydro-1-pyrimidyl (Cytosyl), 4-Oxo-1,4-dihydro-1-pyrimidyl, 2,4-Dioxo-1,2,3,4-tetrahydro-1-pyrimidyl oder 5-Methyl-2,4-dioxo-1,2,3,4-tetrahydro-1-pyrimidyl, oder gegebenenfalls z.B. durch Niederalkyl, Niederalkoxy, Amino und/oder bis zu 2 Oxo substituiertes Dihydro-oder Tetrahydrotriazinyl, wie 2H-1,2-Dihydro-1,3,5-triazin-1-yl, 2H-1,2-Dihydro-1,2,4-triazin-1-yl, 1,2,5,6-Tetrahydro-1,2,4-triazin-1-yl, oder 1,2,3,4-Tetrahydro-1,3,6-triazin-1-yl, z.B. 4-Amino-2-oxo-1,2-dihydro-1,3,5-triazin-1-yl (5-Azacytosyl), 4-Niederalkyl-1,4,5,6-tetrahydro-5,6-dioxo-1,2,4-triazin-1-yl, z.B. 4-Methyl-1,4,5,6-tetrahydro-5,6-dioxo-1,2,4-triazin-1-yl, 2,4-Dioxo-1,2,3,4-tetrahydro-1,3,6-triazin-1-yl (6-Azauracyl) oder 2,4-Dioxo-5-methyl-1,2,3,4-tetrahydro-1,3,6-triazin-1-yl (4-Azathymyl).

Gegebenenfalls substituierte und gegebenenfalls partiell gesättigte Benzo-, Dibenzo-, Pyrido- und Pyrimidoderivate der genannten monocyclischen Azaheterocyclylreste $R_2$ sind insbesondere solche der Pyrrol-1-yl-, Imidazol-1-yl- und Triazol-1-yl-Reste, vor allem Indol-1-yl, Carbazol-9-yl, Benzoimidazol-1-yl, Benzotriazol-1-yl,

Pyrido-pyrrol-1-yl, z.B. 1H-Pyrrolo(2,3-b)pyrid-1-yl, Pyrido-imidazol-1-yl, z.B. 1H-Imidazo(4,5-b)pyrid-1-yl, gegebenenfalls z.B. durch Amino, Imino und/oder Oxo substituiertes Pyrimido-, Dihydropyrimido-oder Tetrahydropyrimido-imidazol-1-yl, z.B. Purin-1-yl, Hypoxanthyl, Adenyl oder Guanyl, oder gegebenenfalls z.B. durch Amino, Imino und/oder Oxo substituiertes Pyrimido-, Dihydropyrimido- oder Tetrahydropyrimido-1,2,3-triazol-1-yl, z.B. 8-Azahypoxanthyl, 8-Azaadenyl oder 8-Azaguanyl.

Bevorzugte Reste $R_2$ sind unsubstituiertes oder z.B. wie angegeben substituiertes Pyrrol-1-yl, Pyrazol-1-yl, 1,2,4-Triazol-1-yl, Tetrazol-1-yl, Benzoimidazol-1-yl, Indol-1-yl, Benzotriazol-1-yl, 1H-Pyrrolo (2,3-b)pyrid-1-yl, 1H-Imidazo(4,5-b) pyrid-1-yl und Purin-1-yl und in erster Linie unsubstituiertes oder z.B. wie angegeben substituiertes Imidazol-1-yl.

Bevorzugte, unter physiologischen Bedingungen spaltbare veresterte Carboxylgruppen $R_3$ sind z.B. Phthalidyloxycarbonyl, Niederalkanoyloxymethoxycarbonyl, z.B. Acetoxymethoxycarbonyl oder Pivaloyloxymethoxycarbonyl, und 1-Niederalkoxycarbonyloxy-niederalkoxycarbonyl, z.B. 1-Aethoxycarbonyloxyäthoxycarbonyl.

Die in den Verbindungen der Formel I vorhandenen funktionellen Gruppen, wie Hydroxy-, Carboxy-, Amino- oder Sulfogruppen, insbesondere die Hydroxygruppe im Rest $R_1$ und die Carboxylgruppe $R_3$, sind gegebenenfalls durch Schutzgruppen geschützt, die in der Penem-, Penicillin-, Cephalosporin- und Peptidchemie verwendet werden. Solche Schutzgruppen schützen die betreffenden funktionellen Gruppen vor unerwünschten Kondensationsreaktionen, Substitutionsreaktionen und dergleichen während der Synthese der Verbindung der Formel I aus ihren Vorstufen.

Solche Schutzgruppen sind leicht, d.h. ohne dass unerwünschte Nebenreaktionen stattfinden, beispielsweise solvolytisch, reduktiv oder auch unter physiologischen Bedingungen, abspaltbar.

Schutzgruppen dieser Art sowie ihre Einführung und Abspaltung sind beispielsweise beschrieben in

J.F.W. McOmie, "Protective Groups in Organic Chemistry", Plenum Press, London, New York, 1973,
T.W. Greene, "Protective Groups in Organic Synthesis", Wiley, New York, 1981,
"The Peptides", Vol. I, Schroeder und Luebke, Academic Press, London, New York, 1965 und
Houben-Weyl, "Methoden der Organischen Chemie", Band 15/1, Georg Thieme Verlag, Stuttgart, 1974.

In Verbindungen der Formel (I) kann eine Hydroxygruppe im Rest $R_1$, ferner eine im Rest $R_2$ vorhandene Hydroxygruppe, beispielsweise durch Acylreste geschützt sein. Geeignete Acylreste sind z.B. gegebenenfalls durch Halogen substituiertes Niederalkanoyl, z.B. Acetyl oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromäthoxycarbonyl oder 2,2,2-Trichloräthoxycarbonyl, Niederalkenyloxycarbonyl, z.B. Allyloxycarbonyl, oder gegebenenfalls durch Nitro substituiertes Phenylniederalkoxycarbonyl, z.B. 4-Nitrobenzyloxycarbonyl. Weitere geeignete Hydroxyschutzgruppen sind z.B. trisubstituiertes Silyl, wie Triniederalkylsilyl, z.B. Trimethylsilyl oder tert.-Butyl-dimethylsilyl, 2-Halogenniederalkylgruppen, z.B. 2-Chlor-, 2-Brom-, 2-Jod- und 2,2,2-Trichloräthyl, und gegebenenfalls durch Halogen, z.B. Chlor, Niederalkoxy, z.B. Methoxy, und/oder Nitro substituiertes Phenylniederalkyl, wie entsprechendes Benzyl. Bevorzugt als Hydroxyschutzgruppe ist Triniederalkylsilyl und Niederalkenyloxycarbonyl.

Eine Carboxylgruppe $R_3$, ferner auch eine im Rest $R_2$ vorhandene Carboxylgruppe, ist üblicherweise in veresterter Form geschützt, wobei die Estergruppe unter schonenden Bedingungen, z.B. unter schonend reduktiven, wie hydrogenolytischen, oder schonend solvolytischen, wie acidolytischen oder insbesondere basisch oder neutral

hydrolytischen Bedingungen, leicht spaltbar ist. Eine geschützte Carboxylgruppe kann ferner eine leicht in eine andere funktionell abgewandelte Carboxylgruppe, wie in eine andere veresterte Carboxylgruppe umwandelbare, veresterte Carboxylgruppe darstellen.

Solche veresterten Carboxylgruppen enthalten als veresternde Gruppen in erster Linie in 1-Stellung verzweigte oder in 1- oder 2-Stellung geeignet substituierte Niederalkylgruppen. Bevorzugte in veresterter Form vorliegende Carboxylgruppen sind unter anderen Niederalkoxycarbonyl, z.B. Methoxycarbonyl, Aethoxycarbonyl, Isopropoxycarbonyl oder tert.-Butoxycarbonyl, und (Hetero-)Arylmethoxycarbonyl mit 1 bis 3 Arylresten oder einem monocyclischen Heteroarylrest, wobei diese gegebenenfalls z.B. durch Niederalkyl, wie tert.-Niederalkyl, z.B. tert.-Butyl, Halogen, z.B. Chlor, und/oder Nitro mono- oder polysubstituiert sind. Beispiele für solche Gruppen sind gegebenenfalls z.B. wie oben erwähnt, substituiertes Benzyloxycarbonyl, z.B. 4-Nitrobenzyloxycarbonyl, gegebenenfalls, z.B. wie oben erwähnt, substituiertes Diphenylmethoxycarbonyl, z.B. Diphenylmethoxycarbonyl, oder Triphenylmethoxycarbonyl, oder gegebenenfalls, z.B. wie oben erwähnt, substituiertes Picolyloxycarbonyl, z.B. 4-Picolyloxycarbonyl, oder Furfuryloxycarbonyl, wie 2-Furfuryloxycarbonyl. Weitere geeignete Gruppen sind Niederalkanoylmethoxycarbonyl, wie Acetonyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, Halogenniederalkoxycarbonyl, wie 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder ω-Halogenniederalkoxycarbonyl, worin Niederalkoxy 4-7 Kohlenstoffatome enthält, z.B. 4-Chlorbutoxycarbonyl, Phthalimidomethoxycarbonyl, Niederalkenyloxycarbonyl, z.B. Allyloxycarbonyl, oder in 2-Stellung durch Niederalkylsulfonyl, Cyano oder trisubstituiertes Silyl, wie Triniederalkylsilyl oder Triphenylsilyl, substituiertes Aethoxycarbonyl, z.B. 2-Methylsulfonyläthoxycarbonyl, 2-Cyanoäthoxycarbonyl, 2-Trimethylsilyläthoxycarbonyl oder 2-(Di-n-butyl-methyl-silyl)-äthoxycarbonyl.

Weitere in veresterter Form vorliegende geschützte Carboxylgruppen sind entsprechende organische Silyloxycarbonyl-, ferner entsprechende organische Stannyloxycarbonylgruppen. In diesen enthält das Silicium- bzw. Zinnatom vorzugsweise Niederalkyl, insbesondere Methyl oder Aethyl, ferner Niederalkoxy, z.B. Methoxy, als Substituenten. Geeignete Silyl- bzw. Stannylgruppen sind in erster Linie Triniederalkylsilyl, insbesondere Trimethylsilyl oder Dimethyl-tert.-butylsilyl, oder entsprechend substituierte Stannylgruppen, z.B. Tri-n-butylstannyl.

Bevorzugte geschützte Carboxylgruppen $R_3'$ sind die 4-Nitrobenzyloxycarbonyl-, Niederalkenyloxycarbonyl-, insbesondere Allyloxycarbonyl-, und die in 2-Stellung durch Niederalkylsulfonyl, Cyano oder Triniederalkylsilyl, z.B. Trimethylsilyl oder Di-n-butylmethylsilyl, substituierte Aethoxycarbonylgruppe.

Eine geschützte Aminogruppe im Rest $R_2$ kann beispielsweise in Form einer leicht spaltbaren Acylamino-, Acylimino-, verätherten Mercaptoamino-, Silyl- oder Stannylaminogruppe oder als Enamino-, Nitro- oder Azidogruppe vorliegen.

In einer entsprechenden Acylaminogruppe ist Acyl beispielsweise der Acylrest einer organischen Säure mit z.B. bis zu 18 Kohlenstoffatomen, insbesondere einer gegebenenfalls z.B. durch Halogen oder Phenyl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Fluor-, 2-Brom-, 2-Jod-, 2,2,2-Trifluor- oder 2,2,2-Trichloracetyl, gegebenenfalls substituiertes Benzoyl, z.B. Benzoyl, Halogenbenzoyl, wie 4-Chlorbenzoyl, Niederalkoxybenzoyl, wie 4-Methoxybenzoyl, oder Nitrobenzoyl, wie 4-Nitrobenzoyl. Insbesondere ist auch Niederalkenyloxycarbonyl, z.B. Allyloxycarbonyl, oder gegebenenfalls in 1- oder 2-Stellung substituiertes Niederalkoxycarbonyl geeignet, wie Niederalkoxycarbonyl, z.B. Methoxy- oder Aethoxycarbonyl, gegebenenfalls substituiertes Benzyloxycarbonyl, z.B. Benzyloxycarbonyl oder 4-Nitrobenzyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder 2-(trisubstituiertes Silyl)-äthoxycarbonyl, wie 2-Triniederalkylsilyläthoxycarbonyl, z.B. 2-Trimethylsilyläthoxycarbonyl oder 2-(Di-n-butylmethyl-silyl)-äthoxycarbonyl, oder 2-Triarylsilyläthoxycarbonyl, wie 2-Triphenylsilyläthoxycarbonyl.

In einer Acyliminogruppe ist Acyl beispielsweise der Acylrest einer organischen Dicarbonsäure mit z.B. bis zu 12 Kohlenstoffatomen, insbesondere einer entsprechenden aromatischen Dicarbonsäure, wie Phthalsäure. Eine solche Gruppe ist in erster Linie Phthalimino.

Eine verätherte Mercaptoaminogruppe ist in erster Linie eine gegebenenfalls durch Niederalkyl, wie Methyl oder tert.-Butyl, Niederalkoxy, wie Methoxy, Halogen, wie Chlor oder Brom, und/oder Nitro substituierte Phenylthioaminogruppe oder eine Pyridylthioaminogruppe. Entsprechende Gruppen sind beispielsweise 2- oder 4-Nitrophenylthioamino oder 2-Pyridylthioamino.

Eine Silyl- oder Stannylaminogruppe ist in erster Linie eine organische Silyl- bzw. Stannylaminogruppe, worin das Silicium- bzw. Zinnatom vorzugsweise Niederalkyl, z.B. Methyl, Aethyl, n-Butyl oder tert.-Butyl, ferner Niederalkoxy, z.B. Methoxy, als Substituenten enthält. Entsprechende Silyl- oder Stannylgruppen sind in erster Linie Triniederalkysilyl, insbesondere Trimethylsilyl, ferner Dimethyl-tert.-butylsilyl, oder entsprechend substituiertes Stannyl, z.B. Tri-n-butylstannyl.

Weitere geschützte Aminogruppen sind z.B. Enaminogruppen, die an der Doppelbindung in 2-Stellung einen elektronenziehenden Substituenten, beispielsweise eine Carbonylgruppe, enthalten. Schutzgruppen dieser Art sind beispielsweise 1-Acyl-niederalk-1-en-2-yl-reste, worin Acyl

z.B. der entsprechende Rest einer Niederalkancarbonsäure, z.B. Essigsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters, z.B. -methylhalbesters oder -äthylhalbesters, und Niederalk-1-en insbesondere 1-Propen bedeutet. Entsprechende Schutzgruppen sind in erster Linie 1-Niederalkanoylprop-1-en-2-yl, z.B. 1-Acetyl-prop-1-en-2-yl, oder 1-Niederalkoxycarbonyl-prop-1-en-2-yl, z.B. 1-Aethoxycarbonyl-prop-1-en-2-yl.

Bevorzugte geschützte Aminogruppen sind z.B. Azido, Phthalimido, Nitro, Niederalkenyloxycarbonylamino, z.B. Allyloxycarbonylamino, und gegebenenfalls durch Nitro substituiertes Benzyloxycarbonylamino.

Eine geschützte Sulfogruppe im Rest $R_2$ ist in erster Linie eine veresterte, wie mit einem aliphatischen, cycloaliphatischen, cycloaliphatisch-aliphatischen, aromatischen oder araliphatischen Alkohol, z.B. einem Niederalkanol, oder mit einem Silyl- oder Stannylrest, wie Triniederalkysilyl, veresterte Sulfogruppe. In einer Sulfogruppe kann die Hydroxygruppe beispielsweise wie die Hydroxygruppe in einer veresterten Carboxylgruppe veräthert sein.

Salze von erfindungsgemässen Verbindungen sind in erster Linie pharmazeutisch annehmbare, nichttoxische Salze von Verbindungen der Formel I. Solche Salze werden beispielsweise von den in Verbindungen der Formel I vorhandenen sauren Gruppen, z.B. Carboxyl- und Sulfogruppen, gebildet und sind in erster Linie Metall- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin oder Tris-(2-hydroxyäthyl)-amin, basischen aliphatischen Estern von Carbonsäuren, z.B. 4-Aminobenzoesäure-2-diäthylaminoäthylester, Niederalkylenaminen, z.B. 1-Aethylpiperidin, Cycloalkylaminen, z.B. Dicyclohexylamin, oder Benzylaminen, z.B. N,N'-Dibenzyläthylendiamin, Dibenzylamin oder N-Benzyl-ß-phenäthylamin. Verbindungen der Formel I mit einer basischen Gruppe, z.B. mit einer Aminogruppe, können Säureadditionssalze, z.B. mit anorganischen Säuren, wie Salzsäure, Schwefelsäure oder Phosphorsäure, oder mit geeigneten organischen Carbon- oder Sulfonsäure, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe können auch in Form von inneren Salze, d.h. in zwitterionischer Form, vorliegen.

Zur Isolierung oder Reinigung können auch pharmazeutisch ungeeignete Salze Verwendung finden. Zur therapeutischen Anwendung gelangen nur die pharmazeutisch annehmbaren, nicht-toxischen Salze, die deshalb bevorzugt sind.

Die Penem-Verbindungen der Formel I können im Substituenten $R_1$ ein zusätzliches Chiralitätszentrum besitzen. Beispielsweise kann 1-Hydroxyäthyl als Substituent $R_1$ in der R-, in der S- oder in der racemischen R,S-Konfiguration vorliegen. In bevorzugten Penem-Verbindungen der Formel I weist ein Rest $R_1$, welcher ein asymmetrisches Kohlenstoffatom besitzt, insbesondere 1-Hydroxyäthyl, die R-Konfiguration auf.

Die Erfindung betrifft insbesondere Verbindungen der Formel I, worin $R_1$ durch Hydroxy oder geschütztes Hydroxy substituiertes Niederalkyl ist, $R_2$ ein über ein Ringstickstoffatom an den Penem-Rest gebundener, gegebenenfalls partiell gesättigter monocyclischer 5-gliedriger Heteroaryl-Rest mit 1-4 Ringstickstoffatomen, z.B. ein entsprechender aza-, diaza-, triaza-oder tetraza-cyclischer Rest aromatischen Charakters oder ein entsprechender Dihydrorest, ein entsprechender partiell gesättigter monocyclischer 6-gliedriger Heteoaryl-Rest mit 1-3 Ringstickstoffatomen, wie ein entsprechender aza-, diaza- oder triaza-cyclischer Rest, oder ein entsprechendes

gegebenenfalls partiell gesättigtes Benzo-, Dibenzo-, Pyrido- oder Pyrimido-Derivat eines solchen 5- oder 6-gliedrigen Restes ist, wobei diese Reste unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkanoyloxyniederalkyl, Niederalkoxyniederalkyl, Carboxyniederalkyl, Niederalkoxycarbonylniederalkyl, Carbamoylniederalkyl, Carbamoyloxyniederalkyl, Halogenniederalkyl, Niederalkylthioniederalkyl, gegebenenfalls N-niederalkyliertes Aminoniederalkyl, Niederalkanoylaminoniederalkyl, Amino-carboxy-niederalkyl, Amino-niederalkoxycarbonyl-niederalkyl, Sulfoniederalkyl, Amino, Niederalkylamino, Diniederalkylamino, Niederalkylenamino, Niederalkanoylamino, Carboxyl, Niederalkoxycarbonyl, Carbamoyl, N-mono- oder N,N-diniederalkyliertes Carbamoyl, Cyano, Sulfo, Sulfamoyl, gegebenenfalls durch Niederalkyl, Nitro, Niederalkoxy und/oder Halogen substituiertes Phenyl, Cycloalkyl, Nitro, Imino, Oxo und/oder Oxido substituiert sind, $R_3$ Carboxyl, unter physiologischen Bedingungen spaltbares verestertes Carboxyl oder geschütztes Carboxyl $R_3'$ bedeutet, optische Isomere von Verbindungen der Formel I, Mischungen dieser optischen Isomere und Salze von Verbindungen der Formel I, die eine salzbildende Gruppe aufweisen.

Die Erfindung betrifft vor allem Verbindungen der Formel I, worin $R_1$ durch Hydroxy oder Triniederalkylsilyloxy substituiertes Niederalkyl ist, $R_2$ gegebenenfalls durch Niederalkyl oder Halogen substituiertes 1-Pyrrolyl oder Dihydro-1-pyrrolyl, gegebenenfalls durch Niederalkyl, Niederalkoxy, Aminoniederalkyl, N-niederalkyliertes Aminoniederalkyl, Niederalkanoylaminoniederalkyl, Amino-carboxy-niederalkyl, Amino-niederalkoxycarbonyl-niederalkyl, Carboxyniederalkyl, Niederalkoxycarbonylniederalkyl, Carbamoylniederalkyl, Carbamoyloxyniederalkyl, Hydroxyniederalkyl, Niederalkanoyloxyniederalkyl, Halogenniederalkyl, Sulfoniederalkyl, Niederalkylthioniederalkyl, Amino, Niederalkylamino, Diniederalkylamino, Niederalkanoylamino oder Nitro substituiertes Imidazol-1-yl, gegebenenfalls durch Niederalkyl, Aminoniederalkyl, Amino-carboxy-niederalkyl, Amino oder Nitro substituiertes Pyrazol-1-yl, gegebenenfalls durch Niederalkyl,

Carboxyniederalkyl oder Phenyl substituiertes 1,2,3-, 1,2,4- oder 1,3,4-Triazol-1-yl, gegebenenfalls durch Niederalkyl, Carboxyniederalkyl, Sulfoniederalkyl, Diniederalkylaminoniederalkyl, Amino oder gegebenenfalls durch Halogen substituiertes Phenyl substituiertes 1- oder 2-Tetrazolyl, unsubstituiertes oder durch Oxo und gegebenenfalls zusätzlich durch Halogen substituiertes Dihydro-1-pyridyl, unsubstituiertes oder durch Oxo und gegebenenfalls zusätzlich durch Niederalkyl, Amino, Diniederalkylamino und/oder Carboxy substituiertes Dihydro- oder Tetrahydro-1-pyrimidyl, gegebenenfalls durch Niederalkyl, Niederalkoxy, Amino und/oder bis zu 2 Oxogruppen substituiertes Dihydro- oder Tetrahydro-1,2,4- oder -1,3,6-triazin-1-yl; Indol-1-yl, Benzimidazol-1-yl, Benzotriazol-1-yl, Pyrido-pyrrol-1-yl, Pyrido-imidazol-1-yl, gegebenenfalls durch Amino, Imino und/oder Oxo substituiertes Pyrimido-, Dihydropyrimido- oder Tetrahydropyrimido-imidazol-1-yl, oder gegebenenfalls durch Amino, Imino und/oder Oxo substituiertes Pyrimido-, Dihydropyrimido- oder Tetrahydropyrimido-1,2,3-triazol-1-yl ist, und $R_3$ Carboxyl, 4-Nitrobenzyloxycarbonyl, Niederalkenyloxycarbonyl, in 2-Stellung durch Niederalkylsulfonyl, Cyano oder Triniederalkylsilyl substituiertes Aethoxycarbonyl oder eine unter physiologischen Bedingungen spaltbare veresterte Carboxygruppe darstellt, optische Isomere von Verbindungen der Formel I, Mischungen dieser optischen Isomere und Salze von solchen Verbindungen der Formel I, die eine salzbildende Gruppe aufweisen.

Die Erfindung betrifft besonders Verbindungen der Formel I, worin $R_1$ in α-Stellung durch Hydroxy substituiertes Niederalkyl ist, $R_2$ unsubstituiertes oder durch Niederalkyl oder Halogen substituiertes Pyrrol-1-yl, unsubstituiertes oder durch Niederalkyl oder Aminoniederalkyl substituiertes Pyrazol-1-yl, unsubstituiertes oder durch Niederalkyl, Niederalkoxy, Aminoniederalkyl, Niederalkanoylaminoniederalkyl, Amino-carboxy-niederalkyl, Amino-niederalkoxycarbonyl-niederalkyl, Carboxyniederalkyl, Niederalkoxycarbonylniederalkyl, Carbamoylniederalkyl, Carbamoyloxyniederalkyl, Hydroxyniederalkyl, Niederalkanoyloxyniederalkyl, Halogenniederalkyl, Niederalkylthioniederalkyl, Amino oder Nitro substituiertes Imidazol-1-yl, unsubstituiertes oder durch Niederalkyl substituiertes 1,2,4- oder 1,3,4-Triazol-1-yl, unsubstituiertes oder durch Niederalkyl, Amino oder Phenyl substituiertes 1- oder 2-Tetrazolyl; Indol-1-yl, Benzimidazol-1-yl, Benzotriazol-1-yl, 1H-Pyrrolo(2,3-b)pyrid-1-yl, 1H-Imidazo(4,5-b)pyrid-1-yl oder Purin-1-yl ist, und $R_3$ Carboxyl oder unter physiologischen Bedingungen spaltbares verestertes Carboxyl, wie Niederalkanoyloxymethoxycarbonyl oder 1-Niederalkoxycarbonyloxy-niederalkoxycarbonyl, bedeutet, optische Isomere von Verbindungen der Formel I, Mischungen dieser optischen Isomere und Salze, insbesondere pharmazeutisch annehmbare Salze von solchen Verbindungen der Formel I, die eine salzbildende Gruppe enthalten.

Die Erfindung betrifft in erster Linie Verbindungen der Formel I, worin $R_1$ Hydroxymethyl oder 1-Hydroxyäthyl ist, $R_2$ Pyrrol-1-yl, unsubstituiertes oder durch Niederalkyl, Niederalkoxy, Amino oder Aminoniederalkyl substituiertes Imidazol-1-yl, unsubstituiertes oder durch Niederalkyl oder Aminoniederalkyl substituiertes Pyrazol-1-yl, 1,2,4-Triazol-1-yl oder unsubstituiertes oder durch Amino substituiertes Tetrazol-1-yl bedeutet, und $R_3$ Carboxyl oder unter physiologischen Bedingungen spaltbares verestertes Carboxyl, wie Niederalkanoyloxymethoxycarbonyl oder 1-Niederalkoxycarbonyloxy-niederalkoxycarbonyl, bedeutet, optische Isomere, insbesondere das (1R)-Isomere von Verbindungen der Formel I, worin $R_1$ 1-Hydroxyäthyl ist, Mischungen dieser optischen Isomere und Salze, insbesondere pharmazeutisch annehmbare Salze von Verbindungen der Formel I.

Die Erfindung betrifft hauptsächlich Verbindungen der Formel I, worin $R_1$ Hydroxymethyl oder (1R)-1-Hydroxyäthyl ist, $R_2$ unsubstituiertes oder durch Niederalkyl oder Aminoniederalkyl substituiertes Imidazol-1-yl bedeutet, und $R_3$ Carboxyl oder unter physiologischen Bedingungen spaltbares verestertes Carboxyl, wie Niederalkanoyloxymethoxycarbonyl oder 1-Niederalkoxycarbonyloxy-niederalkoxycarbonyl, ist, und Salze, insbesondere pharmazeutisch annehmbare Salze von Verbindungen der Formel I.

Die Erfindung betrifft vor allem die in den Beispielen genannten Verbindungen der Formel I und deren Salze, insbesondere pharmazeutisch annehmbare Salze.

Die Verbindungen der vorliegenden Erfindung können nach an sich bekannten Verfahren hergestellt werden.

Die neuen Verbindungen werden z.B. hergestellt, indem man

a. eine Ylid-Verbindung der Formel

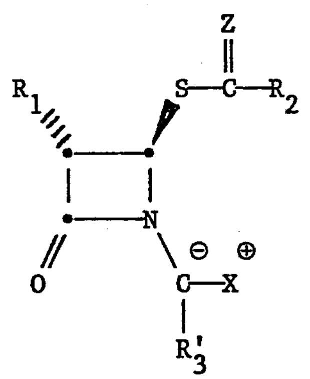

(II),

worin $R_1$ und $R_2$ die unter Formel I angegebenen Bedeutungen haben und $R_3'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b. eine Verbindung der Formel

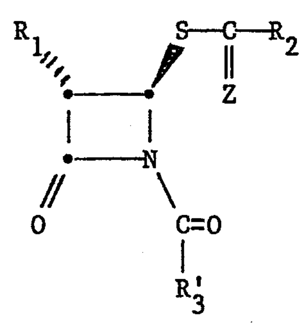

(III),

worin $R_1$ und $R_2$ die unter Formel I angegebenen Bedeutungen haben, Z die unter Formel II angegebene Bedeutung hat und $R_3'$ eine geschützte Carboxylgruppe darstellt, mit einer organischen Verbindung des dreiwertigen Phosphors behandelt, oder

c. eine Verbindung der Formel

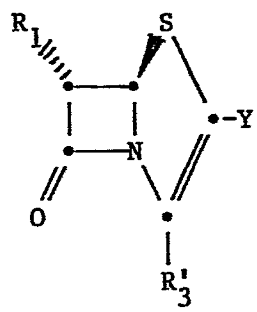

(X),

worin $R_1$ die unter Formel I angegebene Bedeutung hat, $R_3'$ eine geschützte Carboxylgruppe darstellt und Y eine durch nucleophile Reaktion austauschbare Gruppe darstellt, mit einer den Rest $R_2$ einführenden Verbindung umsetzt

und, wenn erwünscht oder notwendig, in einer erhältlichen Verbindung der Formel I eine geschützte Hydroxygruppe im Rest $R_1$ in die freie Hydroxygruppe überführt, und/oder, wenn erwünscht, in einer erhältlichen Verbindung der Formel I eine geschützte Carboxylgruppe $R_3'$ in die freie, in eine unter physiologischen Bedingungen spaltbare veresterte Carboxylgruppe oder in eine andere geschützte Carboxylgruppe $R_3'$ oder eine freie Carboxylgruppe $R_3$ in eine unter physiologischen Bedingungen spaltbare veresterte Carboxylgruppe oder in eine geschützte Carboxylgruppe $R_3'$ überführt, und/oder, wenn erwünscht weitere im Rest $R_2$ enthaltene geschützte funktionelle Gruppen in die freien funktionellen Gruppen überführt, und/oder, wenn erwünscht in einer erhältlichen Verbindung der Formel I einen Rest $R_2$ in einen anderen Rest $R_2$ überführt, und/oder, wenn erwünscht; eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

In den Ausgangsverbindungen der Formeln II, III und X sind funktionelle Gruppen, wie eine freie Hydroxygruppe im Rest $R_1$ sowie weitere im Rest $R_2$ enthaltende funktionelle Gruppen, vorzugsweise durch konventionelle Schutzgruppen, z.B. durch die oben genannten, geschützt.

a) Cyclisierung der Verbindung der Formel II

Die Gruppe $X^{\oplus}$ im Ausgangsmaterial der Formel II ist eine der bei Wittig-Kondensationsreaktionen gebräuchlichen Phosphonio- oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetallion, z.B. das Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z.B. dem Natriumion.

In Phosphonio-Verbindungen der Formel II wird die negative Ladung durch die positiv geladene Phosphoniogruppe neutralisiert. In Phosphono-Verbindungen der Formel II wird die negative Ladung durch das Kation einer starken Base neutralisiert, das je nach Herstellungsweise des Phosphono-Ausgangsmaterials z.B. ein Alkalimetall-, z.B. Natrium-, Lithium- oder Kaliumion, sein kann. Die Phosphono-Ausgangsstoffe werden daher als Salze in die Reaktion eingesetzt.

Der Ringschluss kann spontan, d.h. bei der Herstellung der Ausgangsstoffe, oder durch Erwärmen, z.B. in einem Temperaturbereich von etwa 30°C bis 160°C, vorzugsweise von etwa 50°C bis etwa 100°C, erfolgen. Die Reaktion wird vorzugsweise in einem geeigneten inerten Lösungsmittel, wie in einem aliphatischen, cycloaliphatischen oder aromatischen Kohlenwasserstoff, z.B. Cyclohexan, Benzol oder Toluol, einem halogenierten Kohlenwasserstoff, z.B. Methylenchlorid, einem Aether, z.B. Diäthyläther, einem cyclischen Aether, z.B. Dioxan oder

Tetrahydrofuran, einem Carbonsäureamid, z.B. Dimethylformamid, einem Diniederalkylsulfoxid, z.B. Dimethylsulfoxid, oder einem Niederalkanol, z.B. Aethanol, oder in einem Gemisch davon, und, falls notwendig, in einer Inertgas-, z.B. Stickstoffatmosphäre, durchgeführt.

b. Cyclisierung der Verbindung der Formel III

Eine organische Verbindung des dreiwertigen Phosphors leitet sich z.B. von phosphoriger Säure ab und ist insbesondere ein Ester derselben mit einem Niederalkanol, z.B. Methanol oder Aethanol, und/oder einer gegebenenfalls substituierten aromatischen Hydroxyverbindung, z.B. Phenol oder Brenzcatechin, oder ein Amidester derselben der Formel $P(OR_a)-N(R_b)_2$, worin $R_a$ und $R_b$ unabhängig voneinander Niederalkyl, z.B. Methyl, oder Aryl, z.B. Phenyl, bedeuten. Bevorzugte Verbindungen des dreiwertigen Phosphors sind Trialkylphosphite, z.B. Trimethylphosphit oder Triäthylphosphit.

Die Reaktion wird vorzugsweise in einem inerten Lösungsmittel, wie einem aromatischen Kohlenwasserstoff, z.B. Benzol oder Toluol, einem Aether, z.B. Dioxan oder Tetrahydrofuran, oder einem halogenierten Kohlenwasserstoff, z.B. Methylenchlorid oder Chloroform, bei einer Temperatur von etwa 20° bis 80°C, bevorzugt von etwa 40° bis etwa 60°C, durchgeführt, wobei man 1 Moläquivalent einer Verbindung der Formel III mit 2 Moläquivalent der Phosphorverbindung umsetzt. Vorzugsweise legt man die Verbindung der Formel III in einem inerten Lösungsmittel vor und tropft die Phosphorverbindung, vorzugsweise in dem gleichen inerten Lösungsmittel gelöst, über einen längeren Zeitraum, z.B. während eines Zeitraums von 2 bis 4 Stunden, hinzu.

In einer bevorzugten Ausführungsform des Verfahrens stellt man das Ausgangsmaterial der Formel III wie weiter unten angegeben her und setzt es ohne Isolierung aus dem Reaktionsgemisch mit der organischen Verbindung des dreiwertigen Phosphors um, wobei die Endprodukte der Formel I entstehen.

c. Einführung des Restes $R_2$

In einer Verbindung der Formel X stellt eine durch nucleophile Reaktion austauschbare Gruppe Y z.B. einen Rest der Formel $-S(O)-R_4$, worin $R_4$ z.B. für Niederalkyl, z.B. Methyl oder Aethyl, Cycloalkyl, z.B. Cyclopentyl oder Cyclohexyl, oder Aralkyl, z.B. Benzyl, steht, oder reaktionsfähiges verestertes Hydroxy, wie gegebenenfalls durch Halogen substituiertes Niederalkanoyloxy, z.B. Acetoxy, Niederalkansulfonyloxy, z.B. Methansulfonyloxy, gegebenenfalls durch Niederalkyl, wie Methyl, oder Halogen, wie Brom, substituiertes Benzolsulfonyloxy, z.B. Benzolsulfonyloxy, p-Methyl- oder p-Brombenzolsulfonyloxy, oder einen Rest der Formel $-O-PO(OR_5)_2$, worin $R_5$ z.B. Niederalkyl, wie Methyl oder Aethyl bedeutet, dar.

Geeignete den Rest $R_2$ einführende Verbindungen sind z.B. Verbindungen der Formel $R_2-H$, worin das Wasserstoffatom an dem die Bindung knüpfenden Stickstoffatom des Azaheterocyclylrestes $R_2$ gebunden ist, oder ein Salz, wie ein Alkalimetallsalz, z.B. das Lithium-. Natrium- oder Kaliumsalz davon, oder Verbindungen der Formel $R_2-Si(R_6)_3$, worin $R_6$ insbesondere für Niederalkyl, z.B. Methyl, steht.

Die Umsetzung der Verbindung der Formel X mit der den Rest $R_2$ einführenden Verbindung wird vorzugsweise in einem inerten Lösungsmittel, z.B. Dimethylformamid, Dimethylsulfoxid, Acetonitril, Wasser oder Mischungen davon, bei einer Temperatur von etwa -50°C bis etwa +50°C, insbesondere bei etwa -20°C bis etwa +20°C, durchgeführt, wobei man bei der Umsetzung mit Verbindungen der Formel $R_2-H$ zweckmässigerweise in Gegenwart äquimolarer Mengen einer Base, wie einer organischen Base, z.B. Diisopropyläthylamin, Triäthylamin oder Pyridin, oder einer anorganischen Base, z.B. Natriumhydrogencarbonat oder Kaliumcarbonat, arbeitet.

Die Ausgangsverbindungen der Formel X sind bekannt oder können nach bekannten Verfahren hergestellt werden.

Bevorzugt werden solche Ausgangsmaterialien der Formeln II, III und X verwendet, die zu den eingangs als besonders bevorzugt genannten Verbindungen der Formel I führen.

In einer erhältlichen Verbindung der Formel I, worin eine oder mehrere funktionelle Gruppen geschützt sind, können diese, z.B. geschützte Carboxyl-, Hydroxy-, Amino-, und/oder Sulfogruppen, in an sich bekannter Weise mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

In einer verfahrensgemäss erhältlichen Verbindung der Formel I, worin $R_3$ eine geschützte Carboxylgruppe bedeutet und/oder worin der Rest $R_2$ geschütztes Carboxyl als Substituenten enthält, kann die geschützte Carboxylgruppe in an sich bekannter Weise freigesetzt werden. So kann man tert.-Niederalkoxycarbonyl oder in 2-Stellung durch eine trisubstituierte Silylgruppe oder in 1-Stellung durch Niederalkoxy substituiertes Niederalkoxycarbonyl oder gegebenenfalls substituiertes Diphenylmethoxyarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, oder Metallsalz, wie einem Chrom-II-Salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer geeigneten Carbonsäure, z.B. einer gegebenenfalls, z.B. durch Hydroxy, substituierten Niederalkancarbonsäure, z.B. Essigsäure, Ameisensäure oder Glykolsäure, oder eines Alkohols oder Thiols,

wobei man vorzugsweise Wasser zugibt, in freies Carboxyl überführen. Die Abspaltung einer Allylschutzgruppe kann z.B. durch Umsetzung mit einer Verbindung des Palladiums, z.B. Tetrakis-(triphenylphosphin)-palladium, in Gegenwart von Triphenylphosphin und unter Zusatz einer Carbonsäure, z.B. 2-Aethylhexansäure, oder eines Salzes davon erfolgen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Aroylmethoxycarbonyl in freies Carboxyl umwandeln, während Aroylmethoxycarbonyl ebenfalls durch Behandeln mit einem nucleophilen, vorzugsweise salzbildenden Reagenz, wie Natriumthiophenolat oder Natriumjodid, gespalten werden kann. Substituiertes 2-Silyläthoxycarbonyl kann auch durch Behandeln mit einem das Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetrabutylammoniumfluorid, in freies Carboxyl überführt werden. Mit einer organischen Silyl- oder Stannylgruppe, wie Triniederalkylsilyl oder -stannyl, verestertes Carboxyl kann in üblicher Weise solvolytisch, z.B. durch Behandeln mit Wasser oder einem Alkohol, freigesetzt werden. Eine in 2-Stellung durch Niederalkylsulfonyl oder Cyano substituierte Niederalkoxycarbonylgruppe kann z.B. durch Behandeln mit einem basischen Mittel, wie einem Alkalimetall- oder Erdalkalimetallhydroxid oder -carbonat, z.B. Natrium- oder Kaliumhydroxid oder Natrium- oder Kaliumcarbonat, in freies Carboxyl übergeführt werden.

Andererseits können auch Verbindungen der Formel I, worin $R_3$ Carboxy, bedeutet, in Verbindungen der Formel I überführt werden, worin $R_3$ eine geschützte Carboxylgruppe, insbesondere eine veresterte Carboxylgruppe, oder eine unter physiologischen Bedingungen spaltbare veresterte Carboxylgruppe darstellt. So kann man die freie Carboxylgruppe z.B. durch Behandeln mit einer geeigneten Diazoverbindung, wie einem Diazoniederalkan, z.B. Diazomethan, oder einem

Phenyldiazoniederalkan, z.B. Diphenyldiazomethan, wenn notwendig, in Gegenwart einer Lewis-Säure, wie z.B. Bortrifluorid, oder durch Umsetzen mit einem zur Veresterung geeigneten Alkohol in Gegenwart eines Veresterungsmittels, wie eines Carbodiimids, z.B. Dicyclohexylcarbodiimid, sowie Carbonyldiimidazol, verestern. Ester können auch durch Umsetzung eines gegebenenfalls in situ hergestellten Salzes der Säure mit einem reaktionsfähigen Ester eines Alkohols und einer starken anorganischen Säure, wie Schwefelsäure, oder einer starken organischen Sulfonsäure, wie 4-Toluolsulfonsäure, hergestellt werden. Ferner können Säurehalogenide, wie Chloride (hergestellt z.B. durch Behandeln mit Oxalylchlorid), aktivierte Ester, (gebildet z.B. mit N-Hydroxystickstoffverbindungen, wie N-Hydroxysuccinimid) oder gemischte Anhydride (erhalten z.B. mit Halogenameisensäure-niederalkylester, wie Chlorameisensäureäthyl- oder Chlorameisensäureisobutylester, oder mit Halogenessigsäurehalogeniden, wie Trichloressigsäurechlorid) durch Umsetzen mit geeigneten Alkoholen, gegebenenfalls in Gegenwart einer Base, wie Pyridin, in eine veresterte Carboxylgruppe übergeführt werden.

In einer Verbindung der Formel I mit einer veresterten Carboxylgruppe kann diese in eine andere veresterte Carboxylgruppe übergeführt werden, z.B. 2-Chloräthoxycarbonyl oder 2-Bromäthoxycarbonyl durch Behandeln mit einem Jodsalz, z.B. Natriumjodid, in 2-Jodäthoxycarbonyl. Ferner kann man in Verbindungen der Formel I, die eine in veresterter Form geschützte Carboxylgruppe enthalten, die Carboxylschutzgruppe wie oben beschrieben abspalten und eine entstandene Verbindung der Formel I mit einer freien Carboxylgruppe oder ein Salz davon durch Umsetzen mit dem reaktionsfähige Ester eines entsprechenden Alkohols in eine Verbindung der Formel I überführen, worin $R_3$ eine unter physiologischen Bedingungen spaltbare veresterte Carboxylgruppe darstellt.

In verfahrensgemäss erhältlichen Verbindungen der Formel I, worin der Rest $R_1$ und/oder gegebenenfalls der Rest $R_2$ geschütztes Hydroxyl als Substituenten enthält, kann die geschützte Hydroxygruppe in an sich bekannter Weise in die freie Hydroxygruppe überführt werden.

Beispielsweise wird eine durch eine geeignete Acylgruppe oder eine organische Silyl- oder Stannylgruppe geschützte Hydroxygruppe wie eine entsprechend geschützte Aminogruppe freigesetzt (s.u.); eine Triniederalkylsilylgruppe z.B. auch mit Tetrabutylammoniumfluorid und Essigsäure (unter diesen Bedingungen werden durch trisubstituiertes Silyläthoxy geschützte Carboxygruppen nicht gespalten). Eine 2-Halogenniederalkylgruppe und eine gegebenenfalls substituierte Benzylgruppe werden reduktiv abgespalten.

In einer erfindungsgemäss erhältlichen Verbindung der Formel I mit einer geschützten Aminogruppe kann diese in an sich bekannter Weise, z.B. je nach Art der Schutzgruppe, vorzugsweise mittels Solvolyse oder Reduktion, in die freie Aminogruppe übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe), Aroylmethoxycarbonylamino oder 4-Nitrobenzyloxycarbonylamino durch Behandeln mit einem geeigneten chemischen Reduktionsmittel, wie Zink in Gegenwart einer geeigneten Carbonsäure, wie wässriger Essigsäure, oder katalytisch mit Wasserstoff in Gegenwart eines Palladiumkatalysators gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z.B. Natriumdithionit gespalten werden. Gegebenenfalls substituiertes Benzyloxycarbonylamino kann z.B. mittels Hydrogenolyse, d.h. durch Behandeln mit Wasserstoff in Gegenwart eines geeigneten Hydrierkatalysators, wie eines Palladiumkatalysators, und Allyloxycarbonylamino durch Umsetzen mit einer Verbindung des Palladiums, z.B. Tetrakis(triphenylphosphin)palladium, in Gegenwart von Triphenylphosphin und Behandeln mit einer Carbonsäure, z.B. 2-Aethylhexansäure, oder eines Salzes davon, gespalten werden. Eine mit einer organischen Silyl- oder Stannylgruppe geschützte Aminogruppe kann z.B. mittels Hydrolyse oder Alkoholyse, eine durch 2-Halogenniederalkanoyl, z.B. 2-Chloracetyl, geschützte Aminogruppe durch Behandeln mit Thioharnstoff in Gegenwart einer Base oder mit einem Thiolatsalz, wie einem Alkalimetallthiolat, des Thioharnstoffs und anschliessender Solvolyse, wie Alkoholyse oder Hydrolyse des entstandenen Kondensationsproduktes freigesetzt werden. Eine durch 2-substituiertes Silyläthoxycarbonyl geschützte Aminogruppe kann durch Behandeln mit einem Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetraäthylammoniumfluorid, in die freie Aminogruppe übergeführt werden. Eine in Form einer Azido- oder Nitrogruppe geschützte Aminogruppe wird z.B. durch Reduktion in freies Amino überführt, beispielsweise durch katalytische Hydrierung mit Wasserstoff in Gegenwart eines Hydrierkatalysators wie Platinoxid, Palladium oder Raney-Nickel, oder durch Behandeln mit Zink in Gegenwart einer Säure, wie Essigsäure. Eine in Form einer Phthalimidogruppe geschützte Aminogruppe kann durch Umsetzen mit Hydrazin in die freie Aminogruppe überführt werden. Weiterhin kann eine Arylthioaminogruppe durch Behandeln mit einem nucleophilen Reagenz, wie schwefliger Säure, in Amino umgewandelt werden.

Eine geschützte, insbesondere veresterte, Sulfogruppe wird analog einer geschützten Carboxylgruppe freigesetzt.

In Verbindungen der Formel I kann man weiterhin einen Rest $R_2$ in einen anderen Rest $R_2$ überführen.

So kann beispielsweise in Verbindungen der Formel I, worin der Rest $R_2$ durch eine Carboxylgruppe substituiert ist, diese Carboxylgruppe nach an sich bekannten Verfahren in eine funktionell abgewandelte Carboxylgruppe, wie in eine veresterte Carboxylgruppe oder in gegebenenfalls substituiertes Carbamoyl, überführt werden. Beispielsweise erhält man durch Umsetzen einer Verbindung der Formel I, worin $R_2$ durch Carboxyl substituiertes Azaheterocyclyl ist, mit einem Alkohol, insbesondere einem Niederalkanol, eine Verbindung der Formel I, worin $R_2$ durch verestertes Carboxyl, insbesondere Niederalkoxycarbonyl, substituiertes Azaheterocylyl ist, wobei man

vorzugsweise in Gegenwart eines geeigneten Kondensationsmittels, z.B. eines Carbodiimids, arbeitet oder das entstehende Wasser durch azeotrope Destillation entfernt. Andererseits kann man Carboxylgruppen an Resten $R_2$ auch in reaktionsfähige funktionelle Derivate, wie gemischte Anhydride, z.B. Säurehalogenide, oder aktivierte Ester, überführen und diese durch Umsetzen mit einem Alkohol, z.B. Niederalkanol, Ammoniak oder einem primären oder sekundären Amin, z.B. einem Niederalkyl- oder Diniederalkylamin, in entsprechend veresterte oder amidierte Carboxylgruppen umwandeln, wobei man bei Verwendung von gemischten Anhydriden vorzugsweise in Gegenwart eines säurebindenden Mittels, wie eines aromatischen oder tertiären Amins oder eines Alkalimetall- oder Erdalkalimetallcarbonats, arbeitet.

Weist ein Heteroaryl-Rest $R_2$ eine Hydroxygruppe auf, so lässt sich diese auf üblichem Wege veräthern. Die Umsetzung zu den entsprechenden Niederalkyl-heteroaryl-äthern erfolgt beispielsweise in Gegenwart von Basen, wie Alkalimetallhydroxiden oder -carbonaten, z.B. Natriumhydroxid oder Kaliumcarbonat, mit Hilfe von Diniederalkylsulfaten oder Niederalkylhalogeniden, oder mit Diazoniederalkanen, oder, in Gegenwart eines Dehydratisierungsmittels, beispielsweise Dicyclohexylcarbodiimid, mit Hilfe von Niederalkanolen. Weiterhin lässt sich Hydroxy in verestertes Hydroxy, z.B. Niederalkanoyloxy, umwandeln, beispielsweise durch Umsetzung mit dem reaktionsfähigen Derivat einer entsprechenden Niederalkancarbonsäure, z.B. Essigsäure, wie einem Anhydrid davon, z.B. dem symmetrischen Anhydrid davon oder einem gemischten Anhydrid mit einer Halogenwasserstoffsäure, erforderlichenfalls in Gegenwart eines basischen Kondensationsmittels, wie eines Alkalimetallhydroxids oder -carbonats, oder einer Stickstoffbase, z.B. Pyridin. Die Umwandlung von Niederalkanoyloxy zu Hydroxy erfolgt beispielsweise durch Alkoholyse oder, vorzugsweise, Hydrolyse, z.B. durch basenkatalysierte Hydrolyse, z.B. in Gegenwart von Natriumhydroxid.

In Verbindungen der Formel I, worin $R_2$ durch Amino substituiertes Azaheterocylyl bedeutet, kann die Aminogruppe in eine substituierte Aminogruppe, z.B. eine Niederalkylamino-, Diniederalkylamino-,

Niederalkylenamino- oder Niederalkanoylaminogruppe, überführt werden. Die Ueberführung in eine Niederalkylamino- oder Diniederalkylaminogruppe erfolgt beispielsweise durch Reaktion mit einem reaktionsfähigen veresterten Niederalkanol, beispielsweise einem Niederalkylhalogenid oder -sulfonat, in Gegenwart eines basischen Kondensationsmittels, wie eines Hydroxids oder Carbonats eines Alkali- oder Erdalkalimetalls oder einer heteroaromatischen Stickstoffbase, z.B. Pyridin. In analoger Weise kann Amino durch Behandeln mit einem Niederalkylendihalogenid oder -disulfonat in Niederalkylenamino und durch Behandeln mit dem reaktionsfähigen funktionellen Derivat einer Niederalkancarbonsäure, z.B. dem entsprechenden Carbonsäurehalogenid, in Niederalkanoylamino umgewandelt werden. Verbindungen der Formel I mit einem substituierbaren Ringstickstoffatom im Rest $R_2$ können auf die gleiche Weise in Verbindungen der Formel I überführt werden, welche im Rest $R_2$ ein durch einen gegebenenfalls substituierten Niederalkylrest substituiertes Ringstickstoffatom enthalten. Die mit Hilfe der genannten Umsetzungen herstellbaren neuen Verbindungen der Formel I können daher im Rest $R_2$ ein entsprechend substituiertes sekundäres, tertiäres oder auch quaternäres, d.h. positiv geladenes, Stickstoffatom enthalten.

Salze von Verbindungen der Formel I mit salzbildenden Gruppen können in an sich bekannter Weise hergestellt werden. So kann man Salze von Verbindungen der Formel I mit einer freien Carboxyl- oder Sulfogruppe z.B. durch Behandeln mit Metallverbindungen, wie Alkalimetallsalzen von geeigneten organischen Carbonsäure, z.B. dem Natriumsalz der α-Aethylcapronsäure, oder mit anorganischen Alkali- oder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel

I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze z.B. durch Behandeln mit geeigneten Säuren und Säureadditionssalze z.B. durch Behandeln mit einem geeigneten basischen Mittel.

Erhaltene Gemische von isomeren Verbindungen können nach an sich bekannten Methoden in die einzelnen Isomere aufgetrennt werden. Beispielsweise kann man ein erhaltenes Racemat mit einem optisch aktiven Hilfsstoff reagieren lassen, das dabei entstandene Gemisch zweier diastereomerer Verbindungen mit Hilfe von geeigneten physikalisch-chemischen Methoden (z.B. fraktioniertes Kristallisieren, Adsorptionschromatographie) trennen und die einzelnen diastereomeren Verbindungen dann in die optisch aktiven Verbindungen spalten.
Zur Trennung in Antipoden besonders geeignete Racemate sind solche, die eine saure Gruppe besitzen, wie z.B. Racemate von Verbindungen der Formel I, worin $R_3$ Carboxy ist. Diese sauren Racemate können mit optisch aktiven Basen, z.B. Estern von optisch aktiven Aminosäuren, oder (-)-Brucin, (+)-Chinidin, (-)-Chinin, (+)-Cinchonin, (+)-Dehydroabietylamin, (+)- und (-)-Ephedrin, (+)- und (-)-1-Phenyläthylamin oder deren N-mono- oder N,N-dialkylierten Derivaten zu Gemischen, bestehend aus zwei diastereomeren Salzen, umgesetzt werden.

In Carboxylgruppen enthaltenden Racematen kann diese Carboxylgruppe auch durch einen optisch aktiven Alkohol, wie (-)-Menthol, (+)-Borneol, (+)- oder (-()-2-Octanol verestert werden, worauf nach erfolgter Isolierung des gewünschten Diastereomeren die Carboxylgruppe freigesetzt wird.

Zur Racemattrennung kann auch eine vorhandene Hydroxygruppe mit optisch aktiven Säuren oder deren reaktionsfähigen, funktionellen Derivaten verestert werden, wobei sich diastereomere Ester bilden.

Solche Säuren sind beispielsweise (-)-Abietinsäure, D(+)- und L(-)-Aepfelsäure, N-acylierte optisch aktive Aminosäuren, (+)- und (-)-Camphansäure, (+)- und (-)-Ketopinsäure, L(+)-Ascorbinsäure, (+)-Camphersäure, (+)-Campher-10-sulfonsäure(ß), (+)- oder (-)-α-Bromcampher-π-sulfonsäure, D(-)-Chinasäure, D(-)-Isoascorbinsäure, D(-)- und L(+)-Mandelsäure, (+)-1-Menthoxyessigsäure, D(-)- und L(+)-Weinsäure und deren Di-O-Benzoyl- und Di-O-p-Tolylderivate.

Durch Umsetzung mit optisch aktiven Isocyanaten, wie mit (+)- oder (-)-1-Phenyläthylisocyanat, können Verbindungen der Formel (I), worin $R_3$ geschütztes Carboxy und $R_1$ durch Hydroxy substituiertes Niederalkyl bedeutet, in ein Gemisch diastereomerer Urethane umgewandelt werden.

Basische Racemate, z.B. Verbindungen der Formel I, worin der Rest $R_2$ durch Amino substituiert ist, können mit den genannten optischen aktiven Säuren diastereomere Salze bilden.

Die Spaltung der aufgetrennten Diastereomeren in die optisch aktiven Verbindungen der Formel I erfolgt ebenfalls nach üblichen Methoden. Aus den Salzen befreit man die Säuren oder die Basen z.B. durch Behandeln mit stärkeren Säuren bzw. Basen als die ursprünglich eingesetzten. Aus den Estern und Urethanen erhält man die gewünschten optisch aktiven Verbindungen beispielsweise nach alkalischer Hydrolyse oder nach Reduktion mit einem komplexen Hydrid, wie Lithiumaluminiumhydrid.

Eine weitere Methode zur Auftrennung der Racemate besteht in der Chromatographie an optisch aktiven Adsorptionsschichten, beispielsweise an Rohrzucker.

Nach einer dritten Methode können die Racemate in optisch aktiven Lösungsmitteln gelöst und der schwerer lösliche optische Antipode auskristallisiert werden.

Bei einer vierten Methode benützt man die verschiedene Reaktionsfähigkeit der optischen Antipoden gegenüber biologischem Material, wie Mikroorganismen oder isolierten Enzymen.

Nach einer fünften Methode löst man die Racemate und kristallisiert einen der optischen Antipoden durch Animpfen mit einer kleinen Menge eines nach den obigen Methoden erhaltenen optisch aktiven Produktes aus.

Die Auftrennung der Racemate in die optischen Antipoden kann auf einer beliebigen Verfahrensstufe, d.h. z.B. auch auf der Stufe der Ausgangsverbindungen der Formeln II oder III oder auf einer beliebigen Stufe der nachstehend beschriebenen Verfahren zur Herstellung der Ausgangsverbindungen der Formel II oder III, durchgeführt werden.

Bei allen nachträglichen Umwandlungen erhaltener Verbindungen der Formel I werden solche Reaktionen bevorzugt, die unter alkalischen oder insbesondere neutralen Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird. Ferner können Ausgangsstoffe in Form von Derivaten verwendet oder in situ, gegebenenfalls unter den Reaktionsbedingungen, hergestellt werden.

Die Ausgangsverbindungen der Formeln II und III können, wie in dem folgenden Reaktionsschema I angegeben, hergestellt werden:

Reaktionsschema I

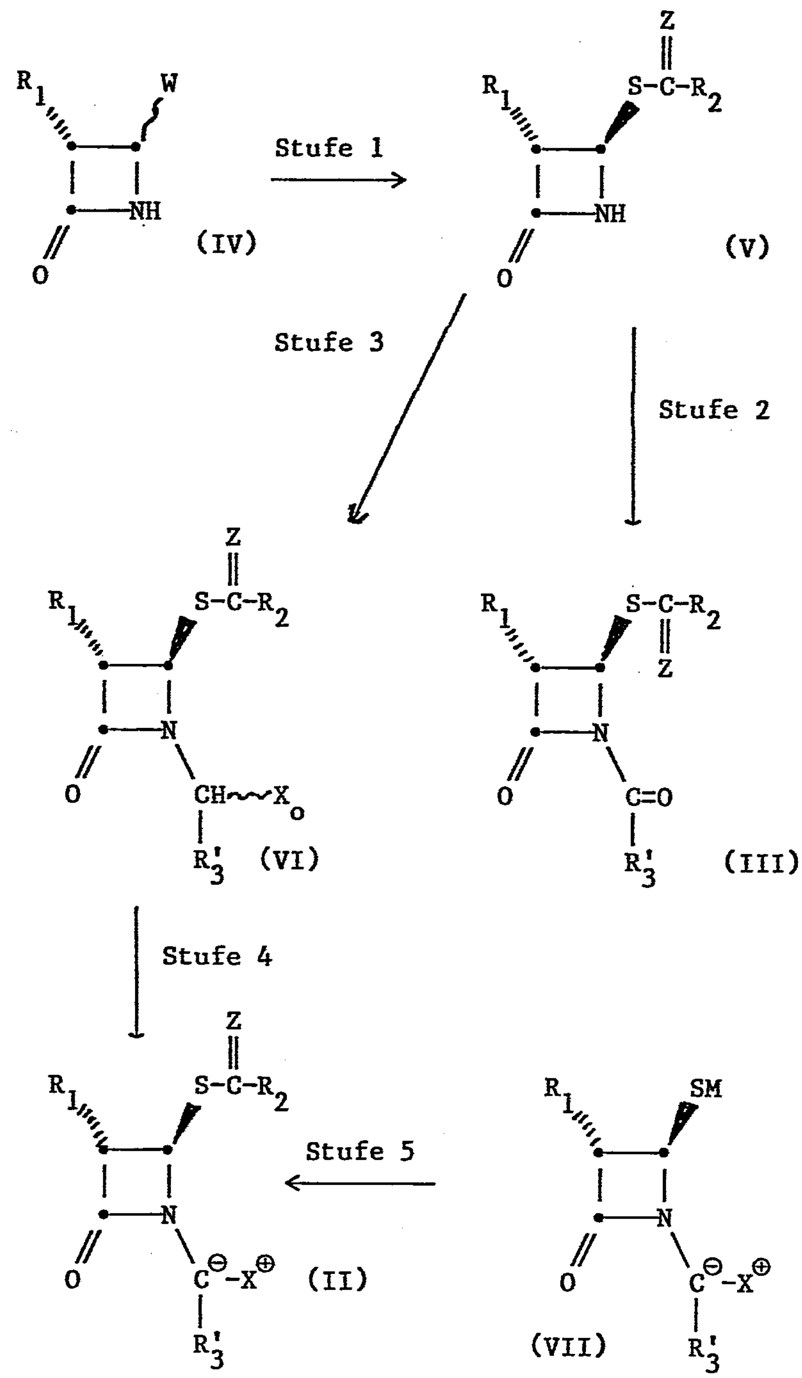


(IV) (V) (VI) (III) (II) (VII)

Stufe 1

Ein Thioazetidinon der Formel V wird erhalten, indem man eine Verbindung der Formel IV mit einer den Rest $-S-C(=Z)-R_2$ einführenden Verbindung umsetzt.

In einem Ausgangsmaterial der Formel IV ist W ein nucleofuger, durch die Gruppe $-S-C(=Z)-R_2$ ersetzbarer Rest. Solche Reste W sind beispielsweise Acyloxyreste, Sulfonylreste $R_o-SO_2-$, worin $R_o$ ein organischer Rest ist, Azido oder Halogen. In einem Acyloxyrest W ist Acyl z.B. der Rest einer organischen Carbonsäure und bedeutet beispielsweise Niederalkanoyl, z.B. Acetyl oder Propionyl, gegebenenfalls substituiertes Benzoyl, z.B. Benzoyl oder 2,4-Dinitrobenzoyl, oder Phenylniederalkanoyl, z.B. Phenylacetyl. In einem Sulfonylrest $R_o-SO_2-$ ist $R_o$ beispielsweise gegebenenfalls durch Hydroxy substituiertes Niederalkyl, wie Methyl, Aethyl, 2-Hydroxyäthyl, 1-Hydroxyprop-2-yl oder 1-Hydroxy-2-methyl-prop-2-yl, Benzyl oder gegebenenfalls substituiertes Phenyl, wie Phenyl, 4-Bromphenyl oder 4-Methylphenyl. Ein Halogenrest W ist z.B. Brom, Jod oder insbesondere Chlor. W ist bevorzugt Methyl- oder 2-Hydroxyäthylsulfonyl, Acetoxy oder Chlor.

Eine den Rest $-S-C(=Z)-R_2$ einführende Verbindung ist beispielsweise eine Säure der Formel $R_2-C(=Z)-SH$ oder insbesondere ein Salz, z.B. ein Alkalimetallsalz, wie das Natrium- oder Kaliumsalz, davon. Die Substitution kann in einem organischen Lösungsmittel, wie in einem Niederalkanol, z.B. Methanol oder Aethanol, einem Niederalkanon, z.B. Aceton, einem Niederalkancarbonsäureamid, z.B. Dimethylformamid, einem cyclischen Aether, z.B. Tetrahydrofuran oder Dioxan, oder in einem ähnlichen inerten Lösungsmittel durchgeführt werden. Die Reaktion wird üblicherweise bei Raumtemperatur durchgeführt, kann aber auch bei erhöhter oder erniedrigter Temperatur, z.B. bei etwa 0° bis etwa 40°C, durchgeführt werden. Durch Zugabe eines Salzes der Jodwasserstoffsäure oder der Thiocyansäure, z.B. eines Alkalimetall-, wie Natriumsalzes, kann die Reaktion beschleunigt werden.

Die eintretende Gruppe $-S-C(=Z)-R_2$ wird von dem Rest $R_1$ bevorzugt in die trans-Stellung dirigiert. Daher können sowohl (3S,4R)- als auch (3S,4RS)-konfigurierte Ausgangsverbindungen der Formel IV eingesetzt werden. Obwohl die trans-Isomeren überwiegend gebildet werden, können doch gelegentlich auch geringe Mengen des cis-Isomeren entstehen. Die Abtrennung der cis-Isomeren erfolgt, wie oben beschrieben, nach konventionellen Methoden, insbesondere durch Chromatographie und/oder durch Kristallisation.

Geeignete Ausgangsverbindungen der Formel IV sind beispielsweise aus der Europäischen Patentanmeldung Nr. 82113, der Deutschen Offenlegungsschrift Nr. 3 224 055 oder der Deutschen Offenlegungsschrift 3 013 997 bekannt oder können in analoger Weise hergestellt werden. Sie können auch nach den in den Beispielen beschriebenen Verfahren hergestellt worden.

Stufe 2

Eine Ausgangsverbindung der Formel (III) wird erhalten, indem man ein Azetidinon der Formel (V) mit einer Säure der Formel $R_3'$-COOH oder insbesondere einem reaktionsfähigen Derivat, wie einem Ester oder Säurehalogenid, z.B. dem Säurechlorid, davon bei einer Temperatur von 20° bis 80°C, bevorzugt bei 40° bis 60°C, in einem inerten Lösungsmittel, wie einem der bei der Umsetzung von Verbindungen der Formel III zu Verbindungen der Formel I genannten, behandelt. Bei Verwendung eines Säurehalogenids arbeitet man vorzugsweise in Gegenwart eines säurebindenden Mittels, wie eines tertiären aliphatischen Amins, z.B. Triäthylamin, eines aromatischen Amins, z.B. Pyridin, oder insbesondere eines Alkalimetall- oder Erdalkalimetallcarbonats oder -hydrogencarbonats, z.B. Kaliumcarbonat oder Calciumcarbonat.

Stufe 3

Verbindungen der Formel VI, worin $X_o$ für eine reaktionsfähige veresterte Hydroxygruppe, insbesondere für Halogen, z.B. Chlor oder Brom, oder organisches Sulfonyloxy, z.B. Niederalkansulfonyloxy, wie Methansulfonyloxy, oder Arensulfonyloxy, z.B. Benzol- oder 4-Methylbenzolsulfonyloxy, steht, werden hergestellt, indem man eine Verbindung der Formel V mit einer Glyoxylsäure-Verbindung der Formel $OHC-R_3'$ oder einem geeigneten Derivat davon, wie einem Hydrat, Hemihydrat oder Halbacetal, z.B. einem Halbacetal mit einem Niederalkanol, z.B. Methanol oder Aethanol, umsetzt, und in einer erhaltenen Verbindung der Formel VI, worin $X_o$ für Hydroxy steht, die Hydroxygruppe in eine reaktionsfähige veresterte Hydroxygruppe umwandelt.

Die Verbindungen der Formel VI werden üblicherweise als Gemisch der beiden Isomeren [bezüglich der Gruppierung $-CH(R_3')\sim X_o$] erhalten. Man kann aber auch die reinen Isomeren davon isolieren, z.B. durch Chromatographie.

Die Anlagerung der Glyoxylsäureester-Verbindung an das Stickstoffatom des Lactamrings in der Verbindung der Formel V findet bei Raumtemperatur oder, falls notwendig, unter Erwärmen, z.B. bei etwa 100°C, und zwar in Abwesenheit eines eigentlichen Kondensationsmittels statt. Bei Verwendung des Hydrats der Glyoxylsäure-Verbindung wird Wasser gebildet, das, wenn notwendig, durch Destillation, z.B. azeotrop, oder durch Verwendung eines geeigneten Dehydratationsmittels, wie eines Molekularsiebs, enfernt wird. Vorzugsweise arbeitet man in Gegenwart eines geeigneten Lösungsmittels, wie z.B. Dioxan, Toluol oder Dimethylformamid, oder Lösungsmittelgemisches, und, wenn erwünscht oder notwendig in der Atmosphäre eines Inertgases, wie Stickstoff.

Die Ueberführung einer Hydroxygruppe $X_o$ in eine reaktionsfähige veresterte Hydroxygruppe $X_o$ in einer Verbindung der Formel VI wird durch Behandeln mit einem geeigneten Veresterungsmittel durchgeführt, z.B. mit einem Thionylhalogenid, z.B. -chlorid, einem Phosphoroxyhalogenid, besonders -chlorid, einem Halogenphosphonium-halogenid, wie Triphenylphosphoniumdibromid oder -dichlorid oder einem geeigneten organischen Sulfonsäurehalogenid, wie -chlorid, vorzugsweise in Gegenwart eines basischen, in erster Linie organischen basischen Mittels, wie eines aliphatischen tertiären Amins, z.B. Triäthylamin oder Diisopropylamin, oder einer heterocyclischen Base vom Pyridintyp, z.B. Pyridin oder Collidin. Vorzugsweise arbeitet man in Gegenwart eines geeigneten Lösungsmittels, z.B. Dioxan oder Tetrahydrofuran, oder eines Lösungsmittelgemisches, wenn notwendig unter Kühlen, z.B. bei etwa -30° bis etwa 30°C, und gegebenenfalls in der Atmosphäre eines Inertgases, wie Stickstoff.

Stufe 4

Das Ausgangsmaterial der Formel (II) wird erhalten, indem man eine Verbindung der Formel (VI) mit einer geeigneten Phosphin-Verbindung, wie einem Triniederalkylphosphin, z.B. Tri-n-butyl-phosphin, oder einem Triaryl-phosphin, z.B. Triphenylphosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt, und in einer erhältlichen Verbindung der Formel (II) den Rest $R_2$ gegebenenfalls gegen einen anderen Rest $R_2$ austauscht.

Die Umwandlung der Verbindung der Formel (VI) in die Verbindung der Formel (II) wird vorzugsweise in Gegenwart eines geeigneten inerten Lösungsmittels, wie eines Kohlenwasserstoffs, z.B. Cyclohexan oder Benzol, oder eines Aethers, z.B. Dioxan, oder eines Lösungsmittelgemisches vorgenommen. Je nach Reaktionsfähigkeit arbeitet man unter Kühlen oder bei erhöhter Temperatur, etwa zwischen -10° und +100°C, bevorzugt bei etwa 20° bis 80°C, und/oder in der Atmosphäre eines inerten Gases, wie Stickstoff. Zur Verhinderung oxidativer Prozesse können katalytische Mengen eines Antioxidans, z.B. Hydrochinon, zugesetzt werden.

Dabei arbeitet man üblicherweise in Gegenwart eines basischen Mittels, wie einer organischen Base, z.B. eines Amins, wie Triäthylamin, Diisopropyläthylamin, Pyridin, Lutidin, oder "Polystyrol-Hünigbase", oder einer anorganischen Base, z.B. eines Alkalimetallcarbonats, z.B. Natrium- oder Kaliumcarbonat, wobei das primär entstandene Phosphoniumsalz der Formel

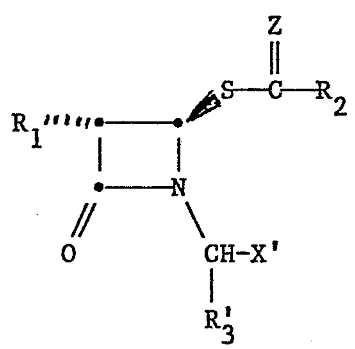

(IIa),

worin X' eine Phosphonogruppe oder eine Phosphoniogruppe zusammen mit einem Anion, je nach Bedeutung des Restes $X_o$ z.B. Chlorid, bedeutet, in das Ylid-Ausgangsmaterial der Formel II umgewandelt wird. Man kann aber auch in Abwesenheit einer Base arbeiten und eine Verbindung der Formel (IIa), insbesondere eine entsprechende Phosphono-Verbindung, isolieren und diese bei der Herstellung der Endprodukte der Formel (I) in situ in das Ausgangsmaterial der Formel II überführen.

In einer erhältlichen Verbindung der Formel (II) kann ein Rest $R_2$ gegen einen anderen Rest $R_2$ ausgetauscht werden. Dazu wird eine Verbindung der Formel (II), worin $R_2$ ein leicht austauschbarer Azaheterocyclyl-Rest ist, z.B. gegebenenfalls substituiertes Imidazol-1-yl, 1,2,3-Triazol-1-yl, 1,3,4-Triazol-1-yl, 1,2,4-Triazol-1-yl, Tetrazol-1-yl oder Tetrazol-2-yl, mit einem Ueberschuss, insbesondere dem drei- bis zehnfachen Ueberschuss, einer Verbindung der Formel $R_2'$-H umgesetzt, worin $R_2'$ ein von $R_2$ verschiedener ungesättigter Azaheterocyclylrest gemäss der vorliegenden Erfindung ist. Die Reaktion wird in einem inerten Lösungsmittel oder Lösungsmittelgemisch, beispielsweise in einem cyclischen Aether, z.B.

Dioxan, in Acetonitril oder in Dimethylformamid, bei Raumtemperatur oder leicht erhöhter oder erniedrigter Temperatur, z.B. bei etwa 10° bis etwa 50°C, durchgeführt.

Stufe 5

Eine Verbindung der Formel (II) kann weiterhin erhalten werden, indem man ein Mercaptid der Formel (VII), worin M für ein Metallkation steht, mit einem den Rest $R_2$-C(=Z)- einführenden Acylierungsmittel behandelt.

In dem Ausgangsmaterial der Formel (VII) ist das Metallkation M beispielsweise ein Kation der Formel $M^+$ oder $M^{2+}/2$, wobei $M^+$ insbesondere für ein Silberkation und $M^{2+}$ insbesondere für das zweiwertige Kation eines geeigneten Uebergangmetalls, z.B. Kupfer, Blei oder Quecksilber, steht.

Ein den Rest $R_2$-C(=Z)- einführendes Acylierungsmittel ist z.B. die Säure $R_2$-C(=Z)-OH oder insbesondere ein reaktionsfähiges funktionelles Derivat davon, wie ein Säurehalogenid, z.B. Chlorid oder Bromid, Azid oder Anhydrid davon, oder eine Verbindung der Formel $S=C(R_2)_2$, worin der Azaheterocyclyl-Rest $R_2$ über ein Ringstickstoffatom an die Thiocarbonylgruppe gebunden ist.

Die Acylierung erfolgt, wenn ein reaktionsfähiges funktionelles Derivat der Säure der Formel $R_2$-C(=Z)-OH, z.B. das Säurechlorid, eingesetzt wird, in einem inerten Lösungsmittel, wie einem chlorierten Kohlenwasserstoff, z.B. Methylenchlorid, oder einem Aether, z.B. Diäthyläther oder Dioxan, bei Raumtemperatur oder unter Erhitzen oder Kühlen, z.B. in einem Temperaturbereich von ca. -50° bis ca. +60°C, insbesondere bei ca. -30° bis ca. +20°C.

Die Acylierung mit Hilfe der Thiocarbonyl-Verbindung der Formel $S=C(R_2)$ erfolgt in einem inerten Lösungsmittel, z.B. einem halogenierten Kohlenwasserstoff, wie Methylenchlorid, einem cyclischen

Aether, wie Tetrahydrofuran, oder Dimethylformamid, bei Raumtemperatur oder erniedrigter Temperatur, z.B. bei ca. -30° bis ca. 30°C, insbesondere bei ca. 0° bis 20°C.

Die Ausgangsverbindungen der Formel (VII) können beispielsweise hergestellt werden, indem man ein Azetidinon der Formel

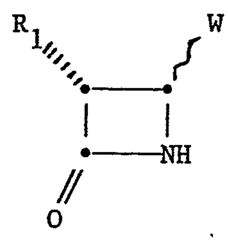

(IV)

durch Umsetzen mit einem Alkalimetallsalz, z.B. dem Natriumsalz, einer Thioniederalkancarbonsäure, z.B. Thioessigsäure, oder des Triphenylmethylmercaptans in eine Verbindung der Formel

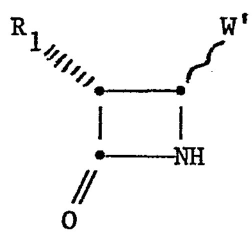

(VIII)

überführt, worin W' Triphenylmethylthio oder Niederalkanoylthio, z.B. Acetylthio, bedeutet, diese analog dem in den Reaktionsstufen 3 und 4 beschriebenen Verfahren in eine Verbindung der Formel

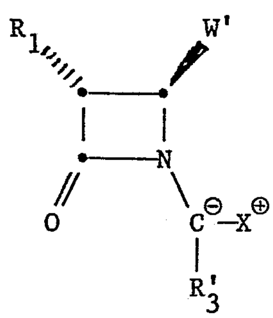

(IX)

überführt und diese in Gegenwart einer Base, z.B. Pyridin oder Tri-n-butylamin, in einem geeigneten Lösungsmittel, z.B. Diäthyläther oder Methanol, mit einem Salz der Formel MA, worin M die obige

Bedeutung hat, insbesondere aber für ein Silber-Kation steht, und A ein gebräuchliches Anion darstellt, welches die Löslichkeit des Salzes MA in dem gewählten Lösungsmittel begünstigt, z.B. das Nitrat-, Acetat- oder Fluorid-Anion, umsetzt.

Thiocarbonylverbindungen der Formel $S=C(R_2)_2$, worin der Rest $R_2$ über ein Stickstoffatom an die Thiocarbonylgruppe gebunden ist, sind bekannt oder können hergestellt werden, indem man eine Verbindung der Formel $R_2$-H, worin das Wasserstoffatom an ein Ringstickstoffatom des Restes $R_2$ gebunden ist, mit einer starken anorganischen Base, z.B. Natriumhydrid, in ein Salz, z.B. in das Natriumsalz der Formel $Na^{\oplus}R_2^{\ominus}$, oder mit einem Triniederalkylhalogensilan, z.B. Trimethylchlorsilan, in Gegenwart einer Base in das Triniederalkylsilyl-Derivat überführt, und dieses mit Thiophosgen umsetzt.

Die Ylide der Formel II können direkt in die Cyclisierungsreaktion zur Herstellung der Endprodukte der Formel I eingesetzt werden. Man kann aber auch in Verbindungen der Formel II, worin $R_1$ durch eine geschützte Hydroxygruppe, z.B. eine hydrolytisch leicht spaltbare geschützte Hydroxygruppe, wie trisubstituiertes Silyloxy, substituiertes Niederalkyl ist, zuerst die Hydroxyschutzgruppe abspalten und dann die erhaltene Verbindung der Formel II, worin $R_1$ durch Hydroxy substituiertes Niederalkyl ist, in die Cyclisierungsreaktion einsetzen.

Beim Austausch eines leicht austauschbaren Restes $R_2$ durch einen unsymmetrisch substituierten Azaheterocyclylrest $R_2'$ mit mindestens zwei Stickstoffatomen oder einen unsubstituierten Azaheterocyclylrest mit mindestens 3 Stickstoffatomen, von denen zwei benachbart sind, in einer Verbindung der Formel II (vgl. Erläuterungen zu Stufe 4 des Reaktionsschema I) bzw. bei der Einführung eines Restes $R_2$ in eine Verbindung der Formel X (vgl. Verfahren c) können zwei isomere Produkte entstehen. So kann beispielsweise bei der Umsetzung einer Verbindung der Formel II oder einer Verbindung der Formel X mit 4-Methylimidazol oder einem Salz davon ein Produkt mit einem 4-Methyl- oder mit einem 5-Methylimidazol-1-yl-Rest entstehen. Die

erfindungsgemäss durch Umsetzung eines entsprechenden Zwischenproduktes mit einem in 4-Stellung substituierten Imidazol hergestellten Imidazol-1-yl-penem-Verbindungen sind laut Dünnschichtchromatogramm homogen und besitzen auf Grund der spektroskopischen Daten wahrscheinlich einen in 4-Stellung (und nicht in 5-Stellung) substituierten Imidazol-1-yl-Rest. Für die bevorzugte Bildung der in 4-Stellung substituierten Imidazol-1-yl-penem-Verbindungen sprechen auch sterische Gründe. Bis zum Vorliegen eindeutiger Strukturbeweise, z.B. durch Röntgenstrukturanalyse, ist aber die Möglichkeit nicht vollständig auszuschliessen, dass die genannten Verbindungen einen in 5-Stellung substituierten Imidazol-1-yl-Rest aufweisen.

In den Verbindungen der Formeln (II)-(VII) können vorhandene funktionelle Gruppen in geschützte funktionelle Gruppen oder vorhandene geschützte funktionelle Gruppen in die freien oder in anders geschützte Gruppen überführt werden. Weiterhin kann man in Verbindungen der Formeln (II), (III), (V) und (VI) einen Rest $R_2$ in einen anderen Rest $R_2$ umwandeln. Bei diesen Umwandlungen können unter Berücksichtigung der weiteren in den Molekülen enthaltenen Substituenten die gleichen Methoden zur Anwendung gelangen, wie bei den entsprechenden Umwandlungen in die Verbindungen der Formel (I) angegeben ist.

Man kann das in Reaktionschema 1 beschriebene Verfahren zur Herstellung der Verbindungen der Formeln (II), (III) und (V)-(VII), sowie die angegebenen Verfahren zur Herstellung der Endprodukte der Formel (I) auch mit optisch inaktiven Verbindungen durchführen und auf einer beliebigen Verfahrensstufe aus einem erhaltenen Diastereomerengemisch oder Racemat, wie oben beschrieben, die optisch aktiven Verbindungen gemäss der vorliegenden Erfindung isolieren.

Die Erfindung betrifft ebenfalls die neuen Ausgangsverbindungen sowie verfahrensgemäss erhältliche neue Zwischenprodukte, wie diejenigen der Formeln (II), (III), (V)-(VII) und (IX), sowie die angegebenen Verfahren zu ihrer Herstellung.

Vorzugsweise werden solche Ausgangsverbindungen verwendet und die Reaktionsbedingungen so gewählt, dass man zu den vorstehend als besonders bevorzugt aufgeführten Verbindungen gelangt.

Die Verbindungen der Formel I weisen wertvolle pharmakologische Eigenschaften auf oder können als Zwischenprodukte zur Herstellung von solchen Verbindungen mit wertvollen pharmakologischen Eigenschaften verwendet werden. Verbindungen der Formel I, worin $R_1$ durch Hydroxy substituiertes Niederalkyl ist, $R_2$ die unter Formel I angegebene Bedeutung hat und $R_3$ Carboxyl oder eine unter physiologischen Bedingungen spaltbare veresterte Carboxylgruppe darstellt und pharmakologisch annehmbare Salze von solchen Verbindungen mit salzbildenden Gruppen haben antibakterielle Wirkungen. Beispielsweise sind sie in vitro gegen grampositive und gramnegative Kokken, z.B. Staphylococcus aureus, Streptococcus pyogenes, Streptococcus pneumoniae und Streptococcus faecalis, und gegen gramnegative Stäbchenbakterien, wie Enterobacteriaceae, Haemophilus influenzae und Pseudomonas aeruginosa, und Anaerobier, z.B. Bacteroides sp., in minimalen Konzentrationen von ca. 0,02 bis ca. 8 µg/ml wirksam. In vivo, bei der systemischen Infektion der Maus, z.B. durch Staphylococcus aureus, ergeben sich bei subkutaner Applikation erfindungsgemässer Verbindungen $ED_{50}$-Werte von ca. 4,5 bis ca. 100 mg/kg.

Die neuen Verbindungen können als oral oder parenteral applizierbare antibakterielle Antibiotika, z.B. in Form von entsprechenden pharmazeutischen Präparaten, zur Behandlung von Infektionen Verwendung finden.

Verbindungen der Formel I, worin mindestens eine der vorhandenen funktionellen Gruppen in geschützter Form vorliegt, können als Zwischenprodukte zur Herstellung der oben genannten pharmakologisch wirksamen Verbindungen der Formel I verwendet werden.

Die pharmakologisch verwendbaren Verbindungen der vorliegenden Erfindung können z.B. zur Herstellung von pharmazeutischen Präparaten verwendet werden, welche eine therapeutisch wirksame Menge der

Aktivsubstanz zusammen oder im Gemisch mit anorganischen oder organischen, festen oder flüssigen, pharmazeutisch annehmbaren Trägerstoffen enthalten, die sich zur oralen oder zur parenteralen, d.h. intramuskulären, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen. Tabletten enthalten ebenfalls Bindemittel, z.B. Magnesiumaluminiumsilikat, Stärken, wie Mais-, Weizen-, Reis-oder Pfeilwurzstärke, Gelatine, Tragacant, Methylcellulose, Natriumcarboxymethylcellulose und/oder Polyvinylpyrrolidon, und, wenn erwünscht, Sprengmittel, z.B. Stärken, Agar, Alginsäure oder Salze davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventionellen Mischungs-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffs.

Je nach Art der Infektion und Zustand des infizierten Organismus verwendet man tägliche Dosen (oral oder parenteral) von etwa 100 mg bis etwa 1 g zur Behandlung von Warmblütern (Menschen und Tiere) von etwa 70 kg Gewicht.

Die folgenden Beispiele dienen zur Illustration der Erfindung. Temperaturen werden in Celsiusgraden angegeben.

In den Beispielen werden folgende Abkürzungen verwendet:

DC: Dünnschichtchromatogramm
IR: Infrarotspektrum
UV: Ultraviolettspektrum
THF: Tetrahydrofuran

Beispiel 1: (5R,6S)-2-(Imidazol-1-yl)-6-(tert.butyldimethylsilyloxymethyl)-2-penem-3-carbonsäure-allylester

3 g 2-[(3S,4R)-3-(tert.Butyldimethylsilyloxymethyl)-4-[(imidazol-1-yl)-thiocarbonylthio]-2-oxo-azetidin-1-yl]-2-triphenylphosphoranylidenessigsäure-allylester werden in 500 ml abs. Toluol während 5 Stunden bei Rückflusstemperatur und unter Argonatmosphäre gerührt. Nach Abkühlen wird das Lösungsmittel abdestilliert und der Rückstand wird an Silicagel chromatographiert (Laufmittel: Toluol-Aethylacetat 4:1); DC (Silicagel-Essigester): $R_f$ = 0,4; IR ($CH_2Cl_2$): 1790; 1715; 1590 $cm^{-1}$.

Das Ausgangsmaterial kann wie folgt hergestellt werden:

a) (3S,4R)-3-(tert.Butyl-dimethylsilyloxymethyl)-4-triphenylmethylthio-azetidin-2-on

12,5 g Triphenylmethylmercaptan werden in 70 ml Methanol bei 0° suspendiert, und über 10 Minuten portionsweise mit insgesamt 2,2 g einer 55%igen Natriumhydrid-Suspension in Oel versetzt. Anschliessend wird eine Emulsion von 11,_ g (3S,4R)-3-(tert.Butyldimethylsilyloxymethyl)-4-methylsulfonyl-azetidin-2-on (Europäische Patentanmeldung Nr. 82113) in 70 ml Aceton und 70 ml Wasser über 30 Minuten zugetropft. Nach 30 Minuten Rühren bei 0° und 1 Stunde bei Raumtemperatur wird das Reaktionsgemisch am Rotationsverdampfer eingeengt, mit Methylenchlorid versetzt, und die wässrige Phase wird abgetrennt. Die organische Lösung wird mit Sole gewaschen und über Natriumsulfat getrocknet. Nach Einengen wird die rohe Titelverbindung durch Chromatographie auf Silicagel (Laufmittel Toluol/Aethylacetat 19:1) gereinigt. DC (Toluol-Essigester 19:1): $R_f$ = 0,64; IR (Methylenchlorid): 3390; 1761; 1118, 835 $cm^{-1}$.

b) 2-[(3S,4R)-3-(tert.Butyl-dimethylsilyloxymethyl)-4-triphenylmethylthio-2-oxo-azetidin-1-yl]-2-hydroxyessigsäureallylester

8,4 g (3S,4R)-3-(tert.Butyldimethylsilyloxymethyl)-4-triphenylmethylthio-azetidin-2-on und 8,23 g Glyoxylsäure-allylester-äthylhemiacetal in 170 ml abs. Toluol werden mit 27 g Molekularsiebe (4Å) versetzt und während 10 Stunden bei 55° gerührt. Nach Abfiltrieren und Einengen am Rotationsverdampfer unter vermindertem Druck wird das Rohprodukt durch Chromatographie an Silicagel gereinigt (Laufmittel Toluol/Aethylacetat 95:5); DC (Silicagel, Toluol/Aethylacetat 10:1); $R_f$ = 0,37 und 0,27; IR ($CH_2Cl_2$): 3520, 1760, 1745 $cm^{-1}$.

c) 2-[(3S,4R)-3-(tert.Butyl-dimethylsilyloxymethyl)-4-triphenylmethylthio-2-oxo-azetidin-1-yl]-2-triphenylphosphoranylidenessigsäure-allylester

Zu einer Lösung von 604 mg 2-[(3S,4R)-3-(tert.Butyl-dimethylsilyloxymethyl)-4-triphenylmethylthio-2-oxo-azetidin-1-yl]-2-hydroxy essigsäure-allylester in 5 ml Tetrahydrofuran werden unter Rühren bei -15° nacheinander 80 µl Thionylchlorid und 88 µl Pyridin innert 5 Minuten zugegeben. Die weisse Suspension wird 1 Stunde bei -10° nachgerührt und über Hyflo filtriert. Nach dem Waschen des Rückstandes mit Toluol wird am Rotationsverdampfer eingeengt. Der Rückstand wird in 3 ml Dioxan gelöst, mit 293 mg Triphenylphosphin und 0,13 ml Lutidin versetzt und während 2 Stunden bei 115° Badtemperatur gerührt. Das Gemisch wird über Hyflo filtriert und dieser Rückstand mit Toluol nachgewaschen. Die vereinigten Filtrate werden eingedampft. Die Chromatographie des Rückstandes an Silicagel ergibt

das reine Produkt (Laufmittel Toluol/Aethylacetat 95:5); DC (Silicagel, Toluol-Aethylacetat 1:1): $R_f$ = 0,18; IR ($CH_2Cl_2$): 1745, 1605 $cm^{-1}$.

d) 2-[(3S,4R)-3-(tert.Butyldimethylsilyloxymethyl)-4-mercapto-2-oxo-azetidin-1-yl]-2-triphenylphosphoranylidenessigsäure-allylester-Silbersalz

7,5 g 2-[(3S,4R)-3-(tert.Butyl-dimethylsilyloxymethyl)-4-triphenylmethylthio-2-oxo-azetidin-1-yl]-2-triphenylphosphoranylidenessigsäure-allylester werden in 87 ml Aether vorgelegt und bei Raumtemperatur mit 70 ml einer 0,5 M wässrigen Silbernitrat-Lösung versetzt. Danach gibt man tropfenweise ein Gemisch aus 3,6 ml Tributylamin, 0,18 ml Trifluoressigsäure und 25 ml Aether dazu und rührt das Reaktionsgemisch weitere 20 Minuten. Dann wird der Feststoff abgenutscht und mit Aether, Wasser und Aether gewaschen. Der Feststoff wird zur Reinigung in 40 ml Aether und 40 ml Wasser aufgeschlämmt, abgenutscht und getrocknet. IR ($CH_2Cl_2$): 1760, 1620 $cm^{-1}$.

e) 2-[(3S,4R)-3-(tert.Butyldimethylsilyloxymethyl)-4-[(imidazol-1-yl)-thiocarbonylthio]-2-oxo-azetidin-1-yl]-2-triphenylphosphoranylidenessigsäure-allylester

4,27 g Silbersalz des 2-[(3S,4R)-3-(tert.Butyldimethylsilyloxymethyl)-4-mercapto-2-oxoazetidin-1-yl]-2-triphenylphosphoranylidenessigsäure-2-allylesters werden in 40 ml abs. Methylenchlorid gelöst und einer auf 0° gekühlten Lösung von 2,14 g 1,1'-Thiocarbonyldiimidazol in 40 ml abs. Methylenchlorid zugegeben. Die Suspension wird noch weitere 2,5 Stunden bei 0° gerührt, dann über Hyflo abfiltriert. Die gelbe Lösung wird am Wasserstrahl-Vakuum eingeengt und der erhaltene Rückstand durch Säulenchromatographie an Silicagel (Laufmittel Toluol/Aethylacetat 95:5 bis 80:20) gereinigt. DC (Silicagel, Toluol/Aethylacetat): $R_f$ = 0,45; IR ($CH_2Cl_2$): 1760; 1620; 1105 $cm^{-1}$.

Beispiel 2: (5R,6S)-2-(Imidazol-1-yl)-6-hydroxymethyl-2-penem-3-carbonsäure-allylester

Eine Lösung von 105 mg (5R,6S)-2-(Imidazol-1-yl)-6-(tert.butyldimethylsilyloxymethyl)-2-penem-3-carbonsäure-allylester in 2,7 ml abs. THF wird auf -70° abgekühlt und nacheinander mit 0,11 ml Essigsäure und tropfenweise während 10 Minuten mit 5,9 ml einer 0,1 M Tetrabutylammoniumfluorid-Lösung in THF versetzt. Das Kühlbad wird entfernt und das Reaktionsgemisch wird langsam auf Raumtemperatur gebracht. Nach 2,25 Stunden bei Raumtemperatur wird das Reaktionsgemisch am Rotationsverdampfer konzentriert und in Aethylacetat und wässriger $NaHCO_3$-Lösung aufgenommen. Die organische Phase wird abgetrennt, mit Sole gewaschen, über $Na_2SO_4$ getrocknet und eingeengt. Das Rohprodukt wird durch Chromatographie auf Silicagel gereinigt (Laufmittel Toluol/Aethylacetat 3:1 bis 1:3); DC (Silicagel, Aethylacetat): $R_f$ = 0,14; IR ($CH_2Cl_2$): 3600; 1790; 1715; 1590 $cm^{-1}$.

Beispiel 3: (5R,6S)-2-(Imidazol-1-yl)-6-hydroxymethyl-2-penem-3-carbonsäure, Natriumsalz

0,38 g (5R,6S)-2-(Imidazol-1-yl)-6-hydroxymethyl-2-penem-3-carbonsäure-allylester werden in 16 ml abs. THF gelöst, auf -10° abgekühlt und mit 40 mg Tetrakis-(triphenylphosphin)-palladium und 0,4 ml Tributylzinnhydrid versetzt. Nach 20 Minuten Rühren bei -10° werden 86 µl Essigsäure zugegeben und das Reaktionsgemisch wird bei -10° 10 Minuten weitergerührt. Nach Konzentration am Rotationsverdampfer wird der Rückstand in Wasser-Aethylacetat aufgenommen, gekühlt und mit $NaHCO_3$ basisch gestellt. Die wässrige Phase wird abgetrennt, zweimal mit Aethylacetat gewaschen und nach Konzentrieren am Rotationsverdampfer auf einer XAD-2 Säule gereinigt (Laufmittel: Wasser). Die vereinigten Fraktionen werden lyophilisiert. DC (Reverse Phase Opti $UCP_{12}$, $H_2O$/Acetonitril): $R_f$ = 0,57; UV (Wasser): $\lambda_{max}$ = 310 nm.

Beispiel 4: (5R,6S)-2-(Pyrazol-1-yl)-6-(tert.butyldimethylsilyloxymethyl)-2-penem-3-carbonsäure-allylester

Analog Beispiel 1 werden 84 mg 2-[(3S,4R)-3-(tert.Butyldimethylsilyloxymethyl)-4-[(pyrazol-1-yl)-thiocarbonylthio]-2-oxoazetidin-1-yl]-2-triphenylphosphoranylidenessigsäure-allylester zur Titelverbindung umgesetzt. IR (Methylenchlorid): 1785; 1705; 1580 $cm^{-1}$.

Das Ausgangsmaterial kann wie folgt hergestellt werden:

Analog Beispiel 1 e) werden 0,21 g Silbersalz des 2-[(3S,4R)-3-(tert.Butyldimethylsilyloxymethyl)-4-mercapto-2-oxoazetidin-1-yl]-2-triphenylphosphoranylidenessigsäure-allylesters durch Reaktion mit 1,1'-Thiocarbonyl-dipyrazol zur Titelverbindung umgesetzt. IR (Methylenchlorid): 1750; 1615 $cm^{-1}$.

Beispiel 5: (5R,6S)-2-(Pyrazol-1-yl)-6-hydroxymethyl-2-penem-3-carbonsäure-allylester

Analog Beispiel 2 werden 50 mg (5R,6S)-2-(Pyrazol-1-yl)-6-(tert-butyldimethylsilyloxymethyl)-2-penem-3-carbonsäure-allylester in die Titelverbindung überführt. IR (Methylenchlorid): 3600; 1785; 1710; 1580 $cm^{-1}$.

Beispiel 6: (5R,6S)-2-(Pyrazol-1-yl)-6-hydroxymethyl-2-penem-3-carbonsäure, Natriumsalz

Analog Beispiel 3 werden 130 mg (5R,6S)-2-(pyrazol-1-yl)-6-hydroxymethyl-2-penem-3-carbonsäure-allylester zur Titelverbindung umgesetzt. UV (Wasser): $\lambda_{max}$ = 312 nm.

Beispiel 7: (5R,6S)-2-(1,2,4-Triazol-1-yl)-6-(tert.butyldimethylsilyloxymethyl)-2-penem-3-carbonsäure-allylester

Analog Beispiel 1 werden 102 mg 2-[(3S,4R)-3-(tert.Butyldimethylsilyloxymethyl)-4-[(1,2,4-triazol-1-yl)-thiocarbonylthio]-2-oxo-azetidin-1-yl]-2-triphenylphosphoranylidenessigsäure-allylester zur Titelverbindung umgesetzt. IR (Methylenchlorid): 1795; 1710; 1585 $cm^{-1}$.

Das Ausgangsmaterial kann wie folgt hergestellt werden:

Analog Beispiel 1 e) werden 0,21 g Silbersalz des 2-[(3S,4R)-3-(tert.Butyldimethylsilyloxymethyl)-4-mercapto-2-oxoazetidin-1-yl]-2-triphenylphosphoranylidenessigsäure-2-allylesters durch Reaktion mit 1,1'-Thiocarbonyl-di-(1,2,4-triazol) zur Titelverbindung umgesetzt. IR (Methylenchlorid): 1755; 1620 $cm^{-1}$.

Beispiel 8: (5R,6S)-2-(1,2,4-Triazol-1-yl)-6-hydroxymethyl-2-penem-3-carbonsäure-allylester

Analog Beispiel 2 werden 850 mg (5R,6S)-2-(1,2,4-Triazol-1-yl)-6-(tert.butyldimethylsilyloxymethyl)-2-penem-3-carbonsäure-allylester in die Titelverbindung überführt. IR (Methylenchlorid): 3610; 1790; 1710; 1585 $cm^{-1}$.

Beispiel 9: (5R,6S)-2-(1,2,4-Triazol-1-yl)-6-hydroxymethyl-2-penem-3-carbonsäure, Natriumsalz

Analog Beispiel 3 werden 510 mg (5R,6S)-2-(1,2,4-Triazol-1-yl)-6-(tert.butyldimethylsilyloxymethyl)-2-penem-3-carbonsäure-allylester zur Titelverbindung umgesetzt. UV (Wasser) $\lambda_{max}$ = 310 nm.

Beispiel 10: (5R,6S)-2-(Imidazol-1-yl)-6-[(1'R)-1-(tert.butyl-dimethylsilyloxyäthyl)]-2-penem-3-carbonsäure-allylester.

Analog Beispiel 1 werden 0,76 g 2-[(3S,4R)-3-[(1'R)-1-tert.Butyldimethylsilyloxyäthyl]-4-(imidazol-1-yl-thiocarbonylthio)-2-oxo-azetidin-1-yl-]-2-triphenylphosphoranylidenessigsäure-allylester zur Titelverbindung umgesetzt. DC (Silicagel; Essigester): $R_f$ = 0,74; IR ($CH_2Cl_2$): 1790; 1715; 1587 $cm^{-1}$

Das Ausgangsmaterial kann wie folgt hergestellt werden:

a). N-p-Methoxybenzyl-N-tert.butylthiomethylammoniumchlorid

Eine Lösung von 10,69 g (23,9 mMol) 1,3,5-Tris-(p-methoxybenzyl)-hexahydro-1,3,5-triazin, welches analog den Vorschriften in der Deutschen Offenlegungsschrift DE-A-2,431,862 hergestellt werden kann, in 170 ml Acetonitril wird bei Raumtemperatur nacheinander mit einer Lösung von 2,88 g (78,8 mMol) Chlorwasserstoff in 20 ml Acetonitril und 6,45 g (71,66 mMol) tert-Butylmercaptan versetzt. Das Gemisch wird 22 Stunden gerührt. Man nutscht ungelöstes Material ab und engt das Filtrat unter vermindertem Druck ein. Man erhält einen kristallinen Rückstand, der mit Aether verrührt und abgesaugt wird. F. 142°.

b). (2S,3R)-N-p-Methoxybenzyl-N-tert.butylthiomethyl-2-brom-3-hydroxybutyramid

Eine Lösung von 1,83 g (10 mMol) (2S,3R)-2-Brom-3-hydroxybuttersäure, hergestellt analog nach einer Vorschrift von Shimohigashi Y. et al., Bull. Chem. Soc. Japan 52, 949 (1979), wird bei Raumtemperatur nacheinander mit 2,76 g (10 mMol) N-p-Methoxybenzyl-N-tert-butylthiomethylammoniumchlorid, 2,06 g (10 mMol) Dicyclohexylcarbodiimid und tropfenweise mit 1,40 ml (10 mMol) Triäthylamin versetzt. Das erhaltene Reaktionsgemisch wird bei Raumtemperatur zwei Stunden lang gerührt. Man nutscht den ausgeschiedenen Dicyclohexylharnstoff ab, verdünnt das Filtrat mit Methylenchlorid und wäscht mit Wasser und Phosphatpufferlösung vom pH 8. Man trocknet die organische Phase über Natriumsulfat, dampft ein und chromatographiert den öligen Rückstand an Kieselgel mit Toluol-Aethylacetat. Man erhält die Titelverbindung als farbloses, dickflüssiges Oel. Rf (Toluol-Aethylacetat 1:1): 0,55. IR (in Methylenchlorid): 3550-3200, 2950-2850, 1632, 1608, 1508, 1457, 1438, 1407, 1360, 1242, 1202, 1175, 1150, 1028.

c). (2S,3R)-N-p-Methoxybenzyl-N-tert.butylsulfonylmethyl-2-brom-3-hydroxybutyramid

Eine Lösung von 1,97 g (4,89 mMol) (2S,3R)-N-p-Methoxybenzyl-N-tert.butylthiomethyl-2-brom-3-hydroxybutyramid in 50 ml Methylen chlorid wird bei -14° unter Rühren mit 2,06 g (ca. 2,2 Aequivalente) 90 %iger m-Chlorperbenzoesäure versetzt. Das Reaktionsgemisch wird 80 Minuten bei 0° gerührt. Man filtriert die ausgeschiedene m-Chlorbenzoesäure ab, verdünnt das Filtrat mit Methylenchlorid und schüttelt dieses nacheinander mit 3 %iger wässriger Natriumhydrogensulfit- und 8 %iger wässriger Natriumhydrogencarbonat-Lösung. Man trocknet die organische Phase über Natriumsulfat, dampft unter vermindertem Druck ein und chromatographiert den Rückstand an Kieselgel mit Toluol-Aethylacetat (7:1) und (6:1). Man erhält die Titelverbindung als farbloses, dickflüssiges Oel. Rf (Toluol-Aethylacetat 1:1): 0,43; [α] = +88 ± 1° (1,01 % in Chloroform). Das $^1$H-NMR-Spektrum (400 MHz in $CDCl_3$) weist auf die Existenz von zwei Rotameren im Verhältnis von 1,3:1 hin.

d). N-p-Methoxybenzyl-N-tert.butylsulfonylmethyl-(2R,3R)-2,3-epoxybutyramid

Zu einer Lösung von 486 mg (1,1 mMol) (2S,3R)-N-p-Methoxybenzyl-N-tert.butylsulfonylmethyl-2-brom-3-hydroxybutyramid in 8 ml Tetrahydrofuran werden bei -14° und Feuchtigkeitsausschluss 340 ml 1,5-Diazabicyclo[5.4.0]undec-5-en in 1 ml Tetrahydrofuran gegeben. Die Lösung wird 75 Minuten bei Raumtemperatur gerührt. Nach Zugabe von Methylenchlorid wird die organische Phase mit 15 %iger wässriger Zitronensäure-Lösung und 8 %iger wässriger Natriumhydrogencarbonat-Lösung ausgeschüttelt. Die organische Phase wird über Natriumsulfat getrocknet und unter vermindertem Druck eingedampft. Nach Chromatographieren des Rückstandes an Kieselgel mit Toluol-Aethylacetat (4:1) erhält man die Titelverbindung als farbloses, dickflüssiges Oel. Rf (Toluol-Aethylacetat 1:1): 0,29; [α] = + 45 ± 1° (1,065 % in $CHCl_3$). Das $^1$H-NMR-Spektrum (400 MHz in $CDCl_3$) weist auf die Existenz von zwei Rotameren im Verhältnis 1:2,8 hin.

e). (3S,4R)-1-p-Methoxybenzyl-3-[(1'R)-1-hydroxyäthyl]-4-tert.butylsulfonyl-2-azetidinon

Eine Lösung von 398 mg (1,12 mMol) N-p-Methoxybenzyl-N-tert.butylsulfonylmethyl-(2R,3R)-2,3-epoxybutyramid in 2,5 ml Tetrahydrofuran wird bei 0° unter Rühren und Feuchtigkeitsausschluss tropfenweise mit 7 ml einer Lösung von entwässertem Tetra-n-butylammoniumfluorid in THF, hergestellt durch Entwässern von 5 g Tetra-n-butylammoniumfluorid-Trihydrat bei 55° und 0,1 Torr und Auffüllen auf 20 ml mit Tetrahydrofuran, versetzt. Man versetzt das Reaktionsgemisch mit aktiviertem Molekularsieb von 4 Å und rührt zwei Stunden. Man nutscht das Molekularsieb ab und wäscht dieses viermal mit je 20 ml Methylenchlorid nach. Jedes Filtrat wird separat mit 5 Teilen Diäthyläther versetzt und nacheinander mit wässriger Phosphatpufferlösung vom pH 8 gewaschen. Man tocknet die vereinigten organischen Phasen über Magnesiumsulfat und dampft unter vermindertem Druck ein. Man chromatographiert den Rückstand an 20 g Kieselgel mit Toluol-Aethylacetat (3:1) und erhält die kristalline Titelverbindung. F. 112°-113° (Kofler, aus Methylenchlorid, Diäthyläther, Pentan); Rf (Toluol-Aethylacetat 1:1): 0,27; [α] = +9 ± 1° (1,105 % in Chloroform); $^1$H-NMR-Spektrum (400 Mhz in $CDCl_3$): δ = 4,65 für Proton (a) am 4(R)-C-Atom, δ = 3,61 für Proton (b) am 3(S)-C-Atom und δ = 4,09 für Proton (c) am 1'(R)-C-Atom der Hydroxyäthylgruppe; J a-b: ca. 2, J b-c: ca. 7.

f). (3S,4R)-3-[(1'R)-1-Hydroxyäthyl)-4-tert.butylsulfonyl-2-azetidinon

Eine Lösung von 71 mg (0,2 mMol) (3S,4R)-1-p-Methoxybenzyl-3-[(1'R)-1-hydroxyäthyl]-4-tert.butylsulfonyl-2-azetidinon in 4 ml Acetonitril wird zu einer auf 65° erwärmten, gut gerührten Lösung von 428 mg (1,8 mMol) Natriumperoxodisulfat, 174 mg (1 mMol) Dikaliumhydrogenphosphat, 1 mg Eisen(II)-sulfatheptahydrat und 2 mg Kupfer-(II)-acetathydrat in 4 ml Wasser gegeben. Man rührt das Reaktionsgemisch zwei Stunden lang bei 65°. Man destilliert das organische Lösungsmittel unter vermindertem Druck ab und extrahiert den wässrigen Rückstand mit Aethylacetat. Nach Einengen der organischen

Phase im Vakuum kristallisiert man den Rückstand aus Methylenchlorid-Diäthyläther und erhält die Titelverbindung. F. 194°; Rf (Ethylacetat): [α] =+13 ± 1° (0,75 % in Methanol); $^1$H-NMR-Spektrum (400 MHz in $CD_3OD$): δ = 5,04 für Proton (a) am 4(R)-C-Atom, δ = 3,58 für Proton (b) am 3(S)-C-Atom und δ = 4,18 für Proton (c) am 1'(R)-C-Atom der Hydroxyäthylgruppe; J a-b; ca. 2, J b-c: ca. 4,5.

g). <u>(3S,4R)-3-[(1'R)-1-(tert.Butyldimethylsilyloxyäthyl)]-4-tert.-butylsulfonyl-2-azetidinon</u>

135 mg (0,574 mMol) (3S,4R)-3-[(1'R)-1-Hydroxyäthyl]-4-tert.butylsulfonyl-2-azetidinon werden mit 173 mg (1,15 mMol) tert.Butyldimethylchlorsilan und 78 mg (1,15 mMol) Imidazol in 3 ml Dimethylformamid zwei Stunden lang bei Raumtemperatur gerührt. Das Reaktionsgemisch wird im Vakuum eingedampft. Der Rückstand wird in Aethylacetat aufgenommen und mit 8 %iger wässriger Natriumhydrogencarbonat-Lösung gewaschen. Nach Trocknen der organischen Phase über Natriumsulfat und Eindampfen unter vermindertem Druck erhält man die Titelverbindung als kristallinen Rückstand. Durch Chromatographieren an Kieselgel mit Toluol-Aethylacetat (4:1) befreit man diesen von Spuren Imidazol und erhält weisse Kristalle. F. 196°; Rf (Toluol-Aethylacetat 1:1): 0,48; [α] = +11 ± 1°.

h) <u>(3S,4R)-3-[(1'R)-1-(tert.Butyldimethylsilyloxyäthyl)]-4-triphenylmethylthio-azetidin-2-on</u>

Analog Beispiel 1a) werden 6,99 g (3S,4R)-3-[(1'R)-1-(tert.Butyldimethylsilyloxyäthyl)]-4-tert.butylsulfonyl-azetidin-2-on in die Titelverbindung überführt. DC (Toluol/Aethylacetat 4:1) $R_f$ = 0,57, IR ($CH_2Cl_2$) 3400; 1765 $cm^{-1}$.

i) <u>2-[(3S,4R)-3-[(1'R)-1-(tert.Butyldimethylsilyloxyäthyl)]-4-triphenylmethylthio-2-oxoazetidin-1-yl]-2-hydroxessigsäure-allylester.</u>

Analog Beispiel 1b) werden 9,55 g (3S,4R)-3-[(1'R)-1-(tert.Butyldimethylsilyloxyäthyl)]-4-triphenylmethylthio-azetidin-2-on in die Titelverbindung überführt. DC (Toluol-Aethylacetat 19:1) $R_f$ = 0,32 und 0,2, IR ($CH_2Cl_2$) 3520; 1765; 1750 $cm^{-1}$.

j) 2-[(3S,4R)-3-[(1'R)-1-(tert.Butyldimethylsilyloxyäthyl)]-4-triphenylmethylthio-2-oxo-azetidin-1-yl-]-2-triphenylphosphoranyliden-essigsäure-allylester.

Analog Beispiel 1c) werden 12,5 g 2-[(3S,4R)-3-[(1'R)-1-(tert.-Butyldimethylsilyloxyäthyl)]-4-triphenylmethylthio-2-oxo-azetidin-1-yl]-2-hydroxyessigsäureallylester in die Titelverbindung überführt. DC (Toluol/Aethylacetat 4:1) $R_f$ = 0,38, IR ($CH_2Cl_2$) 1740; 1615 $cm^{-1}$.

k) 2-[(3S,4R)-3-[(1'R)-1-(tert.Butyldimethylsilyloxyäthyl)]-4-mercapto-2-oxo-azetidin-1-1]-2-triphenylphosphoranylidenessigsäure-allylester, Silbersalz

Analog Beispiel 1d) werden 8,47 g 2-[(3S,4R)-3-[(1'R)-1-(tert.-Butyldimethylsilyloxyäthyl)]-4-triphenylmethylthio-2-oxo-azetidin-1-yl-]-2-triphenylphosphoranylidenessigsäure-allylester in die Titelverbindung umgesetzt. IR ($CH_2Cl_2$) 1760; 1615 $cm^{-1}$.

l) 2-[(3S,4R)-3-[(1'R)-1-(tert.Butyldimethylsilyloxyäthyl)]-4-(imidazol-1-yl-thiocarbonylthio)-2-oxoazetidin-1-yl-]-2-triphenylphosphoranylidenessigsäure-allylester

Analog Beispiel 1e) werden 5,09 g Silbersalz des 2-[(3S,4R)-3-[(1'R)-1-(tert.Butyldimethylsilyloxyäthyl)]-4-mercapto-2-oxoazetidin-1-yl]-2-triphenylphosphoranylidenessigsäure-allylesters mit 2,5 g Thiocarbonyldiimidazol umgesetzt. Nach Reinigung durch Säulenchromatographie (Laufmittel Toluol-Aethylacetat 1:1) erhält man die Titelverbindung. DC (Silicagel, Essigester): $R_f$ = 0,42; IR ($CH_2Cl_2$): 1755; 1620; 1110 cm-1.

Beispiel 11: (5R,6S)-2-(Imidazol-1-yl)-6-[(1'R)-1-hydroxyäthyl]-2-penem-3-carbonsäure-allylester.

Analog Beispiel 2 werden 0,33 g (5R,6S)-2-(Imidazol-1-yl)-6-[(1'R)-1-tert.butyldimethylsilyloxyäthyl]-2-penem-3-carbonsäure-allylester in die Titelverbindung überführt. DC (Silicagel; Essigester): $R_f$ = 0,13; IR ($CH_2Cl_2$) 3600; 1790; 1715; 1587 $cm^{-1}$.

Beispiel 12: (5R,6S)-2-(Imidazol-1-yl)-6-[(1'R)-1-hydroxyäthyl]-2-penem-3-carbonsäure, Natriumsalz.

Analog Beispiel 3 werden 132 mg (5R,6S)-2-(Imidazol-1-yl)-6-[(1'R)-1-hydroxyäthyl]-2-penem-3-carbonsäure-allylester in die Titelverbindung überführt. DC (Reverse Phase Opti $UCP_{12}$, Wasser): $R_f$ = 0,14; UV (Phosphatpuffer pH 7.4): $\lambda_{max}$ = 309,5 nm.

Beispiel 13: (5R,6S)-2-(Pyrrol-1-yl)-6-(tert.butyldimethylsilyloxymethyl)-2-penem -3-carbonsäure-allylester.

5,4 g (3S,4R)-3-(tert.Butyl-dimethylsilyloxymethyl)-4-[(pyrrol-1-yl)-thiocarbonylthio]-azetidin-2-on werden in 60 ml abs. $CH_2Cl_2$ gelöst und nacheinander mit 6 g $CaCO_3$ und bei +10° mit 2,7 Allyloxalylchlorid versetzt. Nach Zutropfen über 5 Minuten von 3,3 ml Hünig-Base in 10 ml Methylenchlorid wird das Gemisch weitere 30 Minuten bei 10° gerührt.

Dar Reaktionsgemisch wird mit Chloroform (abs.) verdünnt, zweimal mit Eiswasser gewaschen, über $Na_2SO_4$ getrocknet, filtriert und eingeengt auf ein Volumen von 25 ml. Nach Verdünnen mit 400 ml abs. Chloroform wird die Badtemperatur auf 70° erhöht, und das Gemisch wird während 2,5 Stunden mit insgesamt 6 ml Triäthylphosphit in 100 ml Chloroform versetzt. Das Reaktionsgemisch wird während 3,25 Stunden bei leichtem Rückfluss gehalten, abgekühlt und eingeengt. Das Rohprodukt wir durch Chromatographie an Silicagel (Laufmittel Toluol und Toluol-Aethylacetat 97,5:2,5) gereinigt. DC (Silicagel; Toluol-Aethylacetat 4:1) $R_f$ = 0,67; IR ($CH_2Cl_2$) 1790; 1710; 1575 $cm^{-}$.

Das Ausgangsmaterial kann wie folgt hergestellt werden:

(3S,4R)-3-(tert.Butyl-dimethylsilyloxymethyl)-4-[(pyrrol-1-yl)-thiocarbonylthio]-azetidin-2-on.

11,72 g (3S,4R)-3-tert.Butyldimethylsilyloxymethyl-4-methylsulfonyl-azetidin-2-on (Europäische Patentanmeldung Nr. 82113) werden in 150 ml abs. Aethanol gelöst und tropfenweise mit einer Lösung von

10,88 g Kalium N-Pyrroldithiocarboxylat [Herstellung siehe R.D. Bereman, D. Nalewajek, Inorganic Chemistry 16, 2687 (1977)] in 150 ml abs. Aethanol versetzt. Nach einstündigem Nachrühren bei Raumtemperatur wird das Reaktionsgemisch am Rotationsverdampfer konzentriert und der Rückstand zwischen Aethylacetat und Wasser aufgetrennt. Nach Waschen der organischen Phase mit Sole und Trocknen derselben über Natriumsulfat wird das Lösungsmittel im Vakuum entfernt. Das Rohprodukt wird durch Chromatographie an Silicagel gereinigt. DC (Silicagel; Toluol/Aethylacetat; 4:1) $R_f$ = 0,5 IR ($CH_2Cl_2$) 3410; 1780; 1310; 1110 $cm^{-1}$.

Beispiel 14: (5R,6S)-2-(Pyrrol-1-yl)-6-hydroxymethyl-2-penem-3-carbonsäureallylester.

Analog Beispiel 2 werden 1,1 g (5R,6S)-2-(Pyrrol-1-yl)-6-tert.butyldimethylsilyloxymethyl-2-penem-3-carbonsäureallylester in die Titelverbindung überführt. DC (Essigester) $R_f$ = 0,5; IR ($CH_2Cl_2$) 3600; 1785; 1705; 1575 $cm^{-1}$.

Beispiel 15: (5R,6S)-2-(Pyrrol-1-yl)-6-hydroxymethyl-2-penem-3-carbonsäure-Natriumsalz

Analog Beispiel 3 werden 0,36 g (5R,6S)-2-(Pyrrol-1-yl)-6-hydroxymethyl-2-penem-3-carbonsäureallylester in die Titelverbindung überführt. UV (Wasser) $\lambda_{max}$ = 310 nm.

Beispiel 16: 2-[(3S,4R)-3-(tert.Butyldimethylsilyloxymethyl)-4-[(pyrazol-1-yl-)-thiocarbonylthio]-2-oxo-azetidin-1-yl]-2-triphenylphosphoranylidenessigsäure-allylester.

108 mg 2-[(3S,4R)-3-tert.Butyldimethylsilyloxymethyl)-4-[(imidazol-1-yl)-thiocarbonylthio]-2-oxo-azetidin-1-yl]-2-triphenylphosphoranylidenessigsäure-allylester werden in 0,6 ml abs. Dimethylformamid gelöst und mit 51 mg Pyrazol versetzt. Nach 6 Stunden Rühren bei 40° wird das Reaktionsgemisch auf Eiswasser gegossen. Der Niederschlag wird abfiltriert und mit Wasser gewaschen. Der Rückstand wird in Aethylacetat aufgenommen, mit Sole gewaschen, über $Na_2SO_4$ getrocknet, filtriert und eingeengt. Das rohprodukt wird durch Chromatographie an Silicagel (Laufmittel Toluol bis Toluol/Aethylacetat 85:15) gereinigt. IR (Methylenchlorid) 1750; 1615 $cm^{-1}$; DC (Toluol/Essigester 1/1) $R_f$ = 0,55.

Das Produkt ist mit dem in Beispiel 4 beschriebenen identisch.

Beispiel 17: (5R,6S)-2-(4-Methyl-pyrazol-1-yl)-6-(tert.butyl-dimethylsilyoxymethyl)-2-penem-3-carbonsäure-allylester

Analog Beispiel 1 werden 1,15 g 2-[(3S,4R)-3-(tert.Butyldimethylsilyloxymethyl)-4-[(4-methyl-pyrazol-1-yl)-thiocarbonylthio]-2-oxoazetidin-1-yl]-2-triphenylphosphoranylidenessigsäure-allylester in die Titelverbindung überführt. IR ($CH_2Cl_2$): 1780, 1700, 1590; 1565 $cm^{-1}$.

Das Ausgangsmaterial kann wie folgt hergestellt werden:

2-[(3S,4R)-3-(tert.Butyldimethylsilyloxymethyl)-4-[(4-methyl-pyrazol-1-yl)-thiocarbonylthio]-2-oxo-azetidin-1-yl]-2-triphenylphosphoranylidenessigsäure-allylester.

Analog Beispiel 16 werden 1,43 g 2-[(3S,4R)-3-(tert.Butyldimethylsilyloxymethyl)-4-[(imidazol-1-yl)-thiocarbonylthio]-2-oxo-azetidin-1-yl]-2-triphenylphosphoranylidenessigsäureallylester in 10 ml abs. DMF mit 0,49 ml 4-Methylpyrazol bei Raumtemperatur während 18,5 Stunden zur Titelverbindung umgesetzt. DC (Toluol-Aethylacetat 1:1) $R_f$ = 0,48; IR ($CH_2Cl_2$) 1750; 1615 $cm^{-1}$.

Beispiel 18: (5R,6S)-2-(4-Methyl-pyrazol-1-yl)-6-(hydroxy methyl-2-penem-3-carbonsäure-allylester

Analog Beispiel 2 werden 0,44 g (5R,6S)-2-(4-Methyl-pyrazol-1-yl)-6-(tert.butyl-dimethylsilyloxymethyl)-2-penem-3-carbonsäure-allylester in die Titelverbindung überführt. IR ($CH_2Cl_2$): 3600; 1785; 1705; 1590; 1570 $cm^{-1}$.

Beispiel 19: (5R,6S)-2-(4-Methyl-pyrazol-1-yl)-6-hydroxymethyl-2-penem-3-carbonsäure, Natriumsalz.

Analog Beispiel 3 werden 0,19 g (5R,6S)-2-(4-Methyl-pyrazol-1-yl)-6-hydroxymethyl-2-penem-3-carbonsäure-allylester in die Titelverbindung überführt. UV (Wasser) $\lambda_{max}$ = 318 nm

Beispiel 20: (5R,6S)-2-(4-Methyl-imidazol-1-yl)-6-(tert.butyldimethylsilyloxymethyl)-2-penem-3-carbonsäureallylester.

Analog Beispiel 1 werden 2,4 g 2-[(3S,4R)-3-(tert.Butyldimethylsilyloxymethyl)-4-[(4-methyl-imidazol-1-yl)-thiocarbonylthio]-2-ozoazetidin-1-yl]-2-triphenylphosphoranylidenessigsäure-allylester in die Titelverbindung überführt, IR ($CH_2Cl_2$): 1790; 1710; 1585 $cm^{-1}$.

Das Ausgangsmaterial kann wie folgt hergestellt werden:

2-[(3S,4R)-3-(tert.Butyldimethylsilyloxymethyl)-4-[(4-methyl-imidazol-1-yl)-thiocarbonylthio]-2-oxoazetidin-1-yl]-2-triphenylphosphoranylidenessigsäure-allylester.

Analog Beispiel 3 werden 2,5 g 2-[(3S,4R)-3-tert.Butyldimethylsilyloxymethyl-4-[(imidazol-1-yl)-thiocarbonylthio]-2-oxoazetidin-1-yl]-2-triphenylphosphoranylidenessigsäureallylester in 18 ml abs. DMF mit 1,44 g 4-Methylimidazol bei Raumtemperatur während 1,5 Stunden zur Titelverbindung umgesetzt. DC (Aceton-Hexan 1/1) $R_f$ = 0,5; IR ($CH_2Cl_2$): 1750, 1615 $cm^{-1}$.

Beispiel 21: (5R,6S)-2-(4-Methyl-imidazol-1-yl)-6-hydroxymethyl-2-penem-3-carbonsäure-allylester

Analog Beispiel 2 werden 0,75 g (5R,6S)-2-(4-Methyl-imidazol-1-yl)-6-(tert.butyldimethylsilyloxymethyl)-2-penem-3-carbonsäure-allylester in die Titelverbindung überführt. IR ($CH_2Cl_2$): 3600; 1790, 1710; 1590 $cm^{-1}$.

Beispiel 22: (5R,6S)-2-(4-Methyl-imidazol-1-yl)-6-hydroxymethyl-2-penem-3-carbonsäure, Natriumsalz

Analog Beispiel 3 werden 0,36 g (5R,6S)-2-(4-Methyl-imidazol-1-yl)-6-hydroxymethyl-2-penem-3-carbonsäure-allylester in die Titelverbindung überführt. UV (Wasser) $\lambda_{max}$ = 310 nm.

Beispiel 23: (5R,6S)-2-(3-Aethylimidazolio)-6-(tert.butyldimethylsilyloxymethyl)-2-penem-3-carbonsäureallylester-tetrafluoroborat

a) Verfahren A:

2,0 g 2-[(3S,4R)-3-(tert.Butyldimethylsilyloxymethyl)-4-(imidazol-1-yl-thiocarbonylthio)-2-oxoazetidin-1-yl]-2-triphenylphosphoranylidenessigsäure-allylester werden in 50 ml abs. Aethylenchlorid gelöst und bei 0° mit 0,533 g Triäthyloxonium-tetrafluoroborat versetzt. Danach entfernt man das Kühlbad und rührt bei Raumtemperatur während 21 Stunden. Nach Zugabe von weiteren 50 mg Triäthyloxonium-tetrafluoroborat wird das Reaktionsgemisch 3 Stunden bei 40° gerührt. Das Gemisch wird dann zweimal mit Wasser gewaschen, über Natriumsulfat getrocknet, filtriert und eingeengt. Der Rückstand wird durch Chromatographie an Silicagel (Laufmittel Aethylacetat bis Aethylacetat/Aceton 2:1) gereinigt. DC (Silicagel, Aethylacetat/-Aceton 4:1) $R_f$ = 0,23, IR (Methylenchlorid): 1800; 1715; 1600 $cm^{-1}$.

b) Verfahren B:

21 mg (5R,6S)-2-(Imidazol-1-yl)-6-(tert.butyldimethylsilyloxymethyl)-2-penem-3-carbonsäure-allylester werden in 1 ml abs. Methylenchlorid gelöst, auf -10° abgekühlt, mit 9,5 mg Triäthyloxonium-tetrafluoroborat versetzt und noch weitere 1,75 Stunden bei 0° gerührt. Das Reaktionsgemisch wird dann mit Methylenchlorid verdünnt, mit Wasser gewaschen, getrocknet und eingeengt. Das Rohprodukt wird wie unter Verfahren a) gereinigt. IR (Methylenchlorid) wie oben.

Beispiel 24: (5R,6S)-2-(3-Aethylimidazolio)-6-[(1'R)-1-(tert.butyldimethylsilyloxyäthyl)]-2-penem-3-carbonsäureallylester-tetrafluoroborat

Analog Beispiel 23 b) werden 0,6 g (5R,6S)-2-(Imidazol-1-yl)-6-[(1'R)-1-tert.butyldimethylsilyloxyäthyl]-2-penem-3-carbonsäure-allylester in die Titelverbindung überführt. IR (Methylenchlorid): 1800; 1718; 1600 $cm^{-1}$.

Beispiel 25: (5R,6S)-2-(3-Aethylimidazolio)-6-hydroxymethyl-2-penem-3-carbonsäure-allylester-tetrafluoroborat

Analog Beispiel 2 werden 0,1 g (5R,6S)-2-(3-Aethylimidazolio)-6-(tert.butyldimethylsilyloxymethyl)-2-penem-3-carbonsäureallylester-tetrafluoroborat in die Titelverbindung überführt. IR (Methylenchlorid): 3600; 1800; 1715; 1600 $cm^{-1}$.

Beispiel 26: (5R,6S)-2-(3-Aethylimidazolio)-6-[(1'R)-1-hydroxyäthyl]-2-penem-3-carbonsäureallylester-tetrafluoroborat

Analog Beispiel 2 werden 0,15 g (5R,6S)-2-(3-Aethylimidazolio)-6-[(1'R)-1-(tert.butyldimethylsilyloxyäthyl)]-2-penem-3-carbonsäure-allylester-tetrafluoroborat in die Titelverbindung überführt. IR (Methylenchlorid): 3600; 1800; 1718; 1600 $cm^{-1}$.

Beispiel 27: (5R,6S)-2-(3-Aethylimidazolio)-6-hydroxymethyl-2-penem-3-carboxylat

Analog Beispiel 3 werden 80 mg (5R,6S)-2-(3-Aethylimidazolio)-6-hydroxymethyl-2-penem-3-carbonsäureallylester-tetrafluoroborat in die Titelverbindung überführt, die durch Chromatographie an Antecgel (OPTI $UPC_{12}$) gereinigt wird. UV (Phosphatpuffer pH 7,4): $\lambda_{max}$ = 312 µm.

Beispiel 28: (5R,6S)-2-(3-Aethylimidazolio)-6-[(1'R)-1-hydroxyäthyl]-2-penem-3-carboxylat

Analog Beispiel 3 werden 237 mg (5R,6S)-2-(3-Aethylimidazolio)-6-[(1'R)-1-hydroxyäthyl]-2-penem-3-carbonsäureallylester-tetrafluoroborat in die Titelverbindung überführt. UV (Phosphatpuffer pH 7,4): $\lambda_{max}$ = 319 µm.

Beispiel 29: (5R,6S)-2-(4,5-Dimethylimidazol-1-yl)-6-(tert.-butyl-dimethylsilyloxymethyl)-2-penem-3-carbonsäureallylester

Analog Beispiel 1 werden 0,15 g 2-[(3S,4R)-3-(tert.Butyldimethylsilyloxymethyl)-4-[(4,5-dimethylimidazol-1-yl)-thiocarbonylthio]-2-oxoazetidin-1-yl]-2-triphenylphosphoranylidenessigsäure-allylester in die Titelverbindung überführt. IR ($CH_2Cl_2$) 1790; 1715; 1590 $cm^{-1}$.

Das Ausgangsmaterial 2-[(3S,4R)-3-(tert.Butyldimethylsilyloxymethyl)-4-[(4,5-dimethylimidazol-1-yl)-thiocarbonylthio]-2-oxoazetidin-1-yl]-2-triphenylphosphoranylidenessigsäure-allylester kann wie folgt hergestellt werden:

Analog Beispiel 16 werden 3 g 2-[(3S,4R)-3-(tert.Butyldimethylsilyloxymethyl)-4-[(imidazol-1-yl)-thiocarbonylthio]-2-oxoazetidin-1-yl]-2-triphenylphosphoranylidenessigsäure-allylester in 20 ml DMF mit 2,1 g 4,5-Dimethylimidazol bei Raumtemperatur während 6 Stunden zur Titelverbindung umgesetzt. DC (Toluol/Aethylacetat 1:1) $R_f$ = 0,2. IR ($CH_2Cl_2$): 1750; 1615 $cm^{-1}$.

Beispiel 30: (5R,6S)-2-(4,5-Dimethylimidazol-1-yl)-6-hydroxymethyl-2-penem-3-carbonsäure-allylester

Analog Beispiel 2 werden 90 mg (5R,6S)-2-(4,5-Dimethylimidazol-1-yl)-6-(tert.butyldimethylsilyloxymethyl)-2-penem-3-carbonsäure-allylester in die Titelverbindung überführt. IR ($CH_2Cl_2$): 3590; 1790; 1710; 1590 $cm^{-1}$.

Beispiel 31: (5R,6S)-2-(4,5-Dimethylimidazol-1-yl)-6-hydroxymethyl-2-penem-3-carbonsäure, Natriumsalz

Analog Beispiel 3 werden 0,43 g (5R,6S)-2-(4,5-Dimethylimidazol-1-yl)-6-hydroxymethyl-2-penem-3-carbonsäure-allylester in die Titelverbindung überführt. UV (Wasser) $\lambda_{max}$ = 312 nm.

Beispiel 32: (5R,6S)-2-[4-Allyloxycarbonylaminoäthyl-imidazol-1-yl]-6-(tert.butyldimethylsilyloxymethyl)-2-penem-3-carbonsäureallylester

Analog Beispiel 2 werden 2,85 g 2-[(3S,4R)-3-(tert.Butyldimethylsilyloxymethyl)-4-[4-allyloxycarbonylaminoäthylimidazol-1-yl-thiocarbonylthio]-2-oxoazetidin-1-yl]-2-triphenylphosphoranylidenessigssäure-allylester in die Titelverbindung überführt.
IR ($CH_2Cl_2$): 3440; 1790; 1710; 1585 $cm^{-1}$.

Die Ausgangsverbindung kann wie folgt hergestellt werden:

a) 3-Allyloxycarbonyl-2-thiazolidin-thion

16,7 g 2-Mercaptothiazolin werden in 140 ml Methylenchlorid gelöst und nacheinander bei 0° mit 20,5 ml Triäthylamin und 14,9 ml Chlorameisensäureallylester in 35 ml Methylenchlorid über 20 Minuten versetzt. Nach weiterem Rühren bei Raumtemperatur während 75 Minuten wird das Reaktionsgemisch zuerst mit Wasser, dann mit Sole gewaschen. Nach Trocknen über Natriumsulfat und Einengen wird das Rohprodukt durch Chromatographie an Silicagel (Laufmittel Toluol-Aethylacetat 95:5) gereinigt. DC (Silicagel; Toluol/Aethylacetat 1:1); $R_f$ = 0,53; IR ($CH_2Cl_2$): 1750; 1720 $cm^{-1}$.

b) 4-(2-Allyloxycarbonylaminoäthyl)-imidazol

5,55 g 4-(2-Aminoäthyl)-imidazol (Hydrochlorid) werden mit 60 ml abs. THF versetzt und tropfenweise mit einer Lösung von 6 g 3-Allyloxycarbonyl-2-thiazolidinthion in 60 ml abs. THF umgesetzt. Nach Zugabe von 8,4 ml Triäthylamin wird das Gemisch 65 Stunden bei Raumtemperatur gerührt. Nach Verdünnen mit 100 ml Methylenchlorid und Abfiltrieren des unlöslichen Teils wird am Rotationsverdampfer

eingeengt. Der Rückstand wird nochmals in Methylenchlorid aufgenommen, erneut filtriert und nach Einengen durch Säulenchromatographie gereinigt. (Laufmittel Aethylacetat bis Aethylacetyt/Aceton 1:1); DC (Silicagel; Aethylacetat/Aceton 2:1); $R_f$ = 0,09.

c) 2-[(3S,4R)-3-(tert.Butyldimethylsilyloxymethyl)-4-[4-(2-allyloxycarbonylaminoäthyl)-imidazol-1-yl-thiocarbonylthio]-2-oxoazetidin-1-yl]-2-triphenylphosphoranyliden-essigsäure-allylester

Analog Beispiel 16 werden 3 g 2-[(3S,4R)-3-(tert.Butyldimethylsilyloxymethyl)-4-[(imidazol-1-yl)-thiocarbonylthio]-2-oxoazetidin-1-yl]-2-triphenylphosphoranylidenessigsäure-allylester in 25 ml DMF mit 3,3 g 4-Allyloxycarbonylaminoäthylimidazol zur Titelverbindung umgesetzt. IR ($CH_2Cl_2$): 3440; 1750; 1720; 1615 $cm^{-1}$.

Beispiel 33: (5R,6S)-2-[4-(2-Allyloxycarbonylaminoäthyl)-imidazol-1-yl]-6-hydroxymethyl-2-penem-3-carbonsäure-allylester

Analog Beispiel 2 werden 1,13 g (5R,6S)-2-[4-(2-Allyloxycarbonylaminoäthylimidazol-1-yl]-6-(tert.butyldimethylsilyloxymethyl)-2-penem-3-carbonsäure-allylester in die Titelverbindung überführt. IR ($CH_2Cl_2$): 3600; 3440; 1790; 1710; 1585 $cm^{-1}$.

Beispiel 34: (5R,6S)-2-[4-(2-Aminoäthyl-imidazol-1-yl]-6-hydroxymethyl-2-penem-3-carbonsäure

Analog Beispiel 3 werden 0,51 g (5R,6S)-2-[4-(2-Allyloxycarbonylaminoäthyl-imidazol-1-yl]-6-hyroxymethyl-2-penem-3-carbonsäure-allylester in die Titelverbindung überführt. UV (Phosphatpuffer pH 7,4) $\lambda_{max}$ = 310 nm.

Beispiel 35: (5R,6S)-2-(4-Methoxy-imidazol-1-yl)-6-(tert.butyldimethylsilyloxymethyl)-2-penem-3-carbonsäure-allylester

Analog Beispiel 1 werden 1,85 g 2-[(3S,4R)-3-(tert.Butyldimethylsilyloxymethyl)-4-[(4-Methoxyimidazol-1-yl)-thiocarbonylthio]-2-oxoazetidin-1-yl]-2-triphenylphosphoranyliden-essigsäure-allylester in die Titelverbindung überführt. IR ($CH_2Cl_2$): 1790; 1710; 1585 $cm^{-1}$.

Das Ausgangsmaterial 2-[(3S,4R)-3-(tert.Butyldimethylsilyloxymethyl)-4-[(4-Methoxyimidazol-1-yl)-thiocarbonylthio]-2-oxoazetidin-1-yl]-2-triphenylphosphoranyliden-essigsäure-allylester kann wie folgt hergestellt werden:

Analog Beispiel 16 werden 2,15 g 2-[(3S,4R)-3-(tert.Butyldimethylsilyloxymethyl)-4-[(imidazol-1-yl)-thiocarbonylthio]-2-oxoazetidin-1-yl]-2-triphenylphosphoranylidenessigsäure-allylester in 20 ml DMF mit 1,18 g 4-Methoxy-imidazol in die Titelverbindung überführt. IR ($CH_2Cl_2$): 1750, 1610 $cm^{-1}$.

Beispiel 36: (5R,6S)-2-[4-Methoxy-imidazol-1-yl]-6-hydroxymethyl-2-penem-3-carbonsäure-allylester

Analog Beispiel 2 werden 0,62 g (5R,6S)-2-[4-Methoxy-imidazol-1-yl]-6-(tert.butyldimethylsilyloxymethyl)-2-penem-3-carbonsäure-allylester in die Titelverbindung überführt. IR ($CH_2Cl_2$): 3600; 1790; 1710; 1582 $cm^{-1}$.

Beispiel 37: (5R,6S)-2-[4-Methoxyimidazol-1-yl]-6-hydroxymethyl-2-penem-3-carbonsäure Natriumsalz

Analog Beispiel 3 werden 0,31 g (5R,6S)-2-[4-Methoxyimidazol-1-yl]-6-hydroxymethyl-2-penem-3-carbonsäure-allylester in die Titelverbindung überführt. UV (Wasser): $\lambda_{max}$ = 310 nm.

Beispiel 38: (5R,6S)-2-[4-Allyloxycarbonylaminomethyl-imidazol-1-yl]-6-(tert.butyldimethylsilyloxymethyl)-2-penem-3-carbonsäureallylester

Analog Beispiel 2 werden 0,196 g 2-[(3S,4R)-3-(tert.Butyldimethylsilyloxymethyl)]-4-[4-allyloxycarbonylaminomethyl-imidazol-1-yl)-thiocarbonylthio]-2-oxoazetidin-1-yl]-2-triphenylphosphoranylidenessigsäureallylester in die Titelverbindung überführt. IR ($CH_2Cl_2$): 3440; 1730; 1715; 1585 $cm^{-1}$.

Das Ausgangsmaterial 2-[(3S,4R)-3-(tert.Butyldimethylsilyloxymethyl)]-4-[4-allyloxycarbonylaminomethyl-imidazol-1-yl)-thiocarbonylthio]-2-oxoazetidin-1-yl]-2-triphenylphosphoranyliden-essigsäureallylester kann wie folgt hergestellt werden:

Analog Beispiel 16 werden 0,286 g 2-[(3S,4R)-3-(tert.Butyldimethylsilyloxymethyl)-4-[(imidazol-1-yl)-thiocarbonylthio]-2-oxoazetidin-1-yl]-2-triphenylphosphoranylidenessigsäureallylester mit 0,29 g 4-Allyloxycarbonylaminomethyl-imidazol in 2 ml DMF in die Titelverbindung überführt. IR ($CH_2Cl_2$): 3450; 1755; 1715; 1615 $cm^{-1}$.

Beispiel 39: (5R,6S)-2-[4-Allyloxycarbonylaminomethyl-imidazol-1-yl]-6-hydroxymethyl-2-penem-3-carbonsäure-allylester

Analog Beispiel 2 werden 9,15 g (5R,6S)-2-[4-Allyloxycarbonylaminomethyl-imidazol-1-yl]-6-(tert.butyldimethylsilyloxymethyl)-2-penem-3-carbonsäure-allylester in die Titelverbindung überführt. IR ($CH_2Cl_2$): 3600; 3440; 1790; 1715; 1590 $cm^{-1}$.

Beispiel 40: (5R,6S)-2-[4-Aminomethyl-imidazol-1-yl]-6-hydroxymethyl-2-penem-3-carbonsäure

Analog Beispiel 3 werden 2 g (5R,6S)-2-[4-Allyloxycarbonylaminomethyl-imidazol-1-yl]-6-hydroxymethyl-2-penem-3-carbonsäure-allylester in die Titelverbindung überführt. UV (Phosphatpuffer, pH 7,4): $\lambda_{max}$ = 312 nm.

Beispiel 41: In analoger Weise, wie in den vorstehenden Beispielen beschrieben, können die folgenden Verbindungen hergestellt werden:

(5R,6S)-2-(Pyrazol-1-yl)-6-[(1R)-1-hydroxyäthyl]-2-penem-3-carbonsäure, Natriumsalz, UV (Wasser) $\lambda_{max}$ = 310 nm.
(5R,6S)-2-(1,2,4-Triazol-1-yl)-6-[(1R)-1-hydroxyäthyl]-2-penem-3-carbonsäure, Natriumsalz, UV (Wasser) $\lambda_{max}$ = 312 nm.
(5R,6S)-2-(Pyrrol-1-yl)-6-[(1R)-1-hydroxyäthyl]-2-penem-3-carbonsäure-Natriumsalz, UV (Wasser) $\lambda_{max}$ = 310 nm.
(5R,6S)-2-(Tetrazol-1-yl)-6-hydroxymethyl-2-penem-3-carbonsäure, Natriumsalz, UV (Wasser) $\lambda_{max}$ = 310 nm.
(5R,6S)-2-(Tetrazol-1-yl)-6-[(1R)-1-hydroxyäthyl]-2-penem-3-carbonsäure, Natriumsalz, UV (Wasser) $\lambda_{max}$ = 312 nm.
(5R,6S)-2-(5-Aminotetrazol-1-yl)-6-hydroxymethyl-2-penem-3-carbonsäure, Natriumsalz, UV (Wasser) $\lambda_{max}$ = 314 nm.
(5R,6S)-2-(5-Aminotetrazol-1-yl)-6-[(1R)-1-hydroxyäthyl]-2-penem-3-carbonsäure, Natriumsalz, UV (Wasser): $\lambda_{max}$ = 312 nm.
(5R,6S)-2-(Indol-1-yl)-6-hydroxymethyl-2-penem-3-carbonsäure, Natriumsalz, UV (Wasser): $\lambda_{max}$ = 311 nm.
(5R,6S)-2-(Indol-1-yl)-6-[(1R)-1-hydroxyäthyl]-2-penem-3-carbonsäure, Natriumsalz, UV (Wasser): $\lambda_{max}$ = 311 nm.
(5R,6S)-2-[1H-Pyrrolo(2,3-b)pyrid-1-yl]-6-hydroxymethyl-2-penem-3-carbonsäure, Natriumsalz, UV (Wasser) $\lambda_{max}$ = 310 nm.
(5R,6S)-2-[1H-Pyrrolo(2,3-b)pyrid-1-yl]-6-[(1R)-1-hydroxyäthyl]-2-penem-3-carbonsäure, Natriumsalz, UV (Wasser) $\lambda_{max}$ = 312 nm.
(5R,6S)-2-(Benzimidazol-1-yl)-6-hydroxymethyl-2-penem-3-carbonsäure, Natriumsalz, UV (Wasser) $\lambda_{max}$ = 313 nm.
(5R,6S)-2-(Benzimidazol-1-yl)-6-[(1R)-1-hydroxyäthyl]-2-penem-3-carbonsäure, Natriumsalz, UV (Wasser) $\lambda_{max}$ = 311 nm.
(5R,6S)-2-(Benzotriazol-1-yl)-6-hydroxymethyl-2-penem-3-carbonsäure, Natriumsalz, UV (Wasser) $\lambda_{max}$ = 312 nm.
(5R,6S)-2-(Benzotriazol-1-yl)-6-[(1R)-1-hydroxyäthyl]-2-penem-3-carbonsäure, Natriumsalz, UV (Wasser) $\lambda_{max}$ = 313 nm.
(5R,6S)-2-[1H-Imidazo(4,5-b)pyrid-1-yl]-6-hydroxymethyl-2-penem-3-carbonsäure, Natriumsalz, UV (Wasser) $\lambda_{max}$ = 312 nm.
(5R,6S)-2-[(1H-Imidazo(4,5-b)pyrid-1-yl]-6-[(1R)-1-hydroxyäthyl]-

2-penem-3-carbonsäure, Natriumsalz, UV (Wasser) $\lambda_{max}$ = 312 nm.
(5R,6S)-2-(Purin-1-yl)-6-hydroxymethyl-2-penem-3-carbonsäure, Natriumsalz, UV (Wasser) $\lambda_{max}$ = 314 nm.
(5R,6S)-2-(Purin-1-yl)-6-[(1R)-1-hydroxyäthyl)-2-penem-3-carbonsäure, Natriumsalz, UV (Wasser) $\lambda_{max}$ = 313 nm.

Beispiel 42: In analoger Weise wie in den vorangehenden Beispielen beschrieben, können die folgenden 2-(Imidazol-1-yl)-penem-Verbindungen hergestellt werden:

(5R,6S)-2-(2-Aminoimidazol-1-yl)-6-hydroxymethyl-2-penem-3-carbonsäure, UV (Wasser) $\lambda_{max}$ = 309 nm.
(5R,6S)-2-(2-Aminoimidazol-1-yl)-6-[(1R)-1-hydroxyäthyl]-2-penem-3-carbonsäure, UV (Wasser) $\lambda_{max}$ = 311 nm.
(5R,6S)-2-(2-Aminomethylimidazol-1-yl)-6-hydroxymethyl-2-penem-3-carbonsäure, UV (Wasser) $\lambda_{max}$ = 313 nm.
(5R,6S)-2-(2-Aminomethylimidazol-1-yl)-6-[(1R)-1-hydroxyäthyl]-2-penem-3-carbonsäure, UV (Wasser) $\lambda_{max}$ = 312 nm.
(5R,6S)-2-(2-Nitroimidazol-1-yl)-6-hydroxymethyl-2-penem-3-carbonsäure, Natriumsalz, UV (Wasser) $\lambda_{max}$ = 310 nm.
(5R,6S)-2-(2-Nitroimidazol-1-yl)-6-[(1R)-1-hydroxyäthyl]-2-penem-3-carbonsäure, Natriumsalz, UV (Wasser) $\lambda_{max}$ = 311 nm.
(5R,6S)-2-[4-(2-Aminoäthyl)-imidazol-1-yl]-6-[(1R)-1-hydroxyäthyl]-2-penem-3-carbonsäure, UV (Phosphatpuffer, pH 7,4) $\lambda_{max}$ = 312 nm.
(5R,6S)-2-(4-Aminomethylimidazol-1-yl)-6-[(1R)-1-hydroxyäthyl]-2-penem-3-carbonsäure, UV (Phosphatpuffer pH 7,4) $\lambda_{max}$ = 311 nm.
(5R,6S)-2-(4,5-Dimethylimidazol-1-yl)-6-[(1R)-1-hydroxyäthyl]-2-penem-3-carbonsäure Natriumsalz, UV (Wasser) $\lambda_{max}$ = 312 nm.
(5R,6S)-2-(4-Aethylimidazol-1-yl)-6-hydroxymethyl-2-penem-3-carbonsäure Natriumsalz. UV (Wasser) $\lambda_{max}$ = 310 nm.
(5R,6S)-2-(4-Aethylimidazol-1-yl)-6-[(1R)-1-hydroxymethyl]-2-penem-3-carbonsäure-Natriumsalz, UV (Wasser) $\lambda_{max}$ = 311 nm.
(5R,6S)-2-(4-Propylimidazol-1-yl)-6-hydroxymethyl-2-penem-3-carbonsäure Natriumsalz, UV (Wasser) $\lambda_{max}$ = 309 nm.
(5R,6S)-2-(4-Propylimidazol-1-yl)-6-[(1R)-1-hydroxyäthyl]-2-

penem-3-carbonsäure Natriumsalz, UV (Wasser) $\lambda_{max}$ = 311 nm.
(5R,6S)-2-(4-Methoxyimidazol-1-yl)-6-[(1R)-1-hydroxyäthyl]-2-penem-3-carbonsäure Natriumsalz, UV (Wasser) $\lambda_{max}$ = 310 nm.
(5R,6S)-2-[4-(2-Acetylaminoäthyl)-imidazol-1-yl]-6-hydroxymethyl-2-penem-3-carbonsäure Natriumsalz, UV (Phosphatpuffer pH 7,4) $\lambda_{max}$ = 309 nm.
(5R,6S)-2-[4-(2-Acetylaminoäthyl)-imidazol-1-yl]-6-[(1R)-1-hydroxyäthyl]-2-penem-3-carbonsäure, Natriumsalz, UV (Phosphatpuffer pH 7,4) $\lambda_{max}$ = 310 nm.
(5R,6S)-2-[4-(2-Amino-2-methoxycarbonyläthyl)-imidazol-1-yl]-6-hydroxymethyl-2-penem-3-carbonsäure, UV (Phosphatpuffer pH 7,4) $\lambda_{max}$ = 308 nm.
(5R,6S)-2-[4-(2-Amino-2-methoxycarbonyläthyl)-imidazol-1-yl]-6-[(1R)-1-hydroxyäthyl]-2-penem-3-carbonsäure, UV (Phosphatpuffer pH 7,4) $\lambda_{max}$ = 310 nm.
(5R,6S)-2-(4-Methylimidazol-1-yl)-6-[(1R)-1-hydroxyäthyl]-2-penem-3-carbonsäure Natriumsalz. UV (Wasser) $\lambda_{max}$ = 312 nm.
(5R,6S)-2-(4-Hydroxymethylimidazol-1-yl)-6-hydroxymethyl-2-penem-3-carbonsäure Natriumsalz, UV (Wasser) $\lambda_{max}$ = 312 nm.
(5R,6S)-2-(4-Hydroxymethylimidazol-1-yl)-6-[(1R)-1-hydroxyäthyl]-2-penem-3-carbonsäure Natriumsalz, UV (Wasser) $\lambda_{max}$ = 311 nm.
(5R,6S)-2-(4-Acetoxymethylimidazol-1-yl)-6-hydroxymethyl-2-penem-3-carbonsäure Natriumsalz, UV (Phosphatpuffer pH 7,4) $\lambda_{max}$ = 310 nm.
(5R,6S)-2-(4-Acetoxymethylimidazol-1-yl)-6-[(1R)-1-hydroxyäthyl)-2-penem-3-carbonsäure Natriumsalz, UV (Wasser) $\lambda_{max}$ = 309 nm.
(5R,6S)-2-(4-Methoxycarbonylmethylimidazol-1-yl)-6-hydroxymethyl-2-penem-3-carbonsäure Natriumsalz, UV (Wasser) $\lambda_{max}$ = 311 nm.
(5R,6S)-2-(4-Methoxycarbonylmethylimidazol-1-yl)-6-[(1R)-1-hydroxyäthyl]-2-penem-3-carbonsäure Natriumsalz, UV (Wasser) $\lambda_{max}$ = 310 nm.
(5R,6S)-2-(4-Carbamoylmethylimidazol-1-yl)-6-hydroxymethyl-2-penem-3-carbonsäure Natriumsalz, UV (Phosphatpuffer pH 7,4) $\lambda_{max}$ = 311 nm.
(5R,6S)-2-(4-Carbamoylmethylimidazol-1-yl)-6-[(1R)-1-hydroxyäthyl]-2-penem-3-carbonsäure Natriumsalz, UV (Phosphatpuffer pH 7,4) $\lambda_{max}$ = 311 nm.

(5R,6S)-2-(4-Chlormethylimidazol-1-yl)-6-hydroxymethyl-2-penem-3-carbonsäure Natriumsalz, UV (Phosphatpuffer pH 7,4) $\lambda_{max}$ = 312 nm.
(5R,6S)-2-(4-Chlormethylimidazol-1-yl)-6-[(1R)-1-hydroxyäthyl]-2-penem-3-carbosäure Natriumsalz, UV (Wasser) $\lambda_{max}$ = 310 nm.
(5R,6S)-2-(4-Carboxymethylimidazol-1-yl)-6-hydroxymethyl-2-penem-3-carbonsäure Dinatriumsalz, UV (Wasser) $\lambda_{max}$ = 309 nm.
(5R,6S)-2-(4-Carboxymethylimidazol-1-yl)-6-[(1R)-1-hydroxyäthyl]-2-penem-3-carbonsäure-Dinatriumsalz, UV (Wasser) $\lambda_{max}$ = 309 nm.
(5R,6S)-2-(4-Carbamoyloxymethyl-imidazol-1-yl)-6-hydroxymethyl-2-penem-3-carbonsäure Natriumsalz, UV (Wasser) $\lambda_{max}$ = 313 nm.
(5R,6S)-2-(4-Carbamoyloxymethyl-imidazol-1-yl)-6-[(1R)-1-hydroxyäthyl]-2-penem-3-carbonsäure Natriumsalz, UV (Wasser) $\lambda_{max}$ = 312 nm.
(5R,6S)-2-(4-Methylthiomethyl-imidazol-1-yl)-6-[(1R)-1-hydroxyäthyl]-2-penem-3-carbonsäure Natriumsalz, UV (Wasser) $\lambda_{max}$ = 310 nm.
(5R,6S)-2-(4-Aethylthiomethyl-imidazol-1-yl)-6-[(1R)-1-hydroxyäthyl]-2-penem-3-carbonsäure Nariumsalz, UV (Wasser) $\lambda_{max}$ = 309 nm.

Beispiel 43: (5R,6S)-2-(Imidazol-1-yl)-6-[(1R)-1-hydroxyäthyl]-2-penem-3-carbonsäure-acetoxymethylester

60,7 g (5R,6S)-2-(Imidazol-1-yl)-6-[(1R)-1-hydroxyäthyl]-2-penem-3-carbonsäure-Natriumsalz werden in 2 ml abs. DMF und 0,2 ml abs. DMSO gelöst und bei 0° unter Rühren tropfenweise mit einer Lösung von 39,8 mg Acetoxybrommethan in 0,3 ml abs. DMF versetzt. Nach 30 Minuten Rühren bei 0° und anschliessend 30 Minuten bei Raumtemperatur wird das Reaktionsgemisch mit Aethylacetat verdünnt und zweimal mit Sole gewaschen. Die organische Phase wird über Magnesiumsulfat getrocknet und eingedampft. Die Reinigung durch Säulenchromatographie (Laufmittel Aethylacetat) ergibt die Titelverbindung.
DC (Silicagel, Aceton) $R_f$ = 0,7; IR (Methylenchlorid): 3590; 1790; 1765; 1725; 1580 $cm^{-1}$; UV (Aethanol) $\lambda_{max}$ = 328,5 nm.

Beispiel 44: (5R,6S)-2-(Imidazol-1-yl)-6-hydroxymethyl-2-penem-3-carbonsäure-1-äthoxycarbonyloxyäthylester

1,2 g Natriumjodid werden in 3,7 ml Aceton gelöst und mit 0,275 ml Aethyl-1-chloräthylcarbonat versetzt. Das Gemisch wird bei Raumtemperatur während 3 Stunden gerührt. Anschliessend wird die Lösung auf 15 ml Methylenchlorid getropft und von den ausgefallenen anorganischen Salzen abfiltriert. Die Methylenchloridlösung wird bis auf 2 ml eingeengt und bei 0° zu einer Lösung von 0,267 g (1 mMol) (5R,6S)-2-(Imidazol-1-yl)-6-hydroxymethyl-2-penem-3-carbonsäure in 4 ml Dimethylacetamid gegeben. Dann wird während 3 Stunden bei 0° gerührt, anschliessend mit Aethylacetat verdünnt und dreimal mit Wasser gewaschen. Die organischen Phasen werden über Natriumsulfat getrocknet und am Rotationsverdampfer eingeengt. Das Rohprodukt wird an 10 g Silicagel mit dem Laufmittel Aethylacetat gereinigt. Man erhält die Titelverbindung als weissen Schaum. IR-Spektrum (Methylenchlorid): 3600; 1790; 1750; 1720; 1670 $cm^{-1}$.

Beispiel 45: (5R,6S)-2-(Imidazol-1-yl)-6-hydroxymethyl-2-penem-3-carbonsäure-pivaloyloxymethylester

0,6 g Natriumjodid werden in 2 ml Aceton gelöst und mit 0,15 ml Pivalinsäurechlormethylester versetzt. Das Gemisch wird bei Raumtemperatur während 3 Stunden gerührt und anschliessend auf 7,5 ml Methylenchlorid getropft. Die ausgefallenen anorganischen Salze werden abfiltriert. Die Methylenchloridlösung wird bis auf 1 ml eingeengt und zu einer Lösung von 0,11 g (0,4 mMol) (5R,6S)-2-(Imidazol-1-yl)-6-hydroxymethyl-2-penem-3-carbonsäure und 0,17 ml Diisopropyläthylamin in 4 ml N,N-Dimethylacetamid bei 0° gegeben. Dann wird während 3 Stunden bei 0° gerührt, anschliessend mit Aethylacetat verdünnt und dreimal mit Wasser gewaschen. Die organische Phase wird über Natriumsulfat getrocknet und am Rotationsverdampfer eingeengt. Das Rohprodukt wird an 10 g Silicagel mit dem Laufmittel Aethylacetat gereinigt. Man erhält die Titelverbindung als weissen Schaum. IR-Spektrum (Methylenchlorid): 3600; 1795; 1745; 1725; 1670 $cm^{-1}$.

Beispiel 46: In analoger Weise, wie in den Beispielen 43-45 beschrieben, können die folgenden Ester hergestellt werden:

(5R,6S)-2-(Imidazol-1-yl)-6-hydroxymethyl-2-penem-3-carbonsäure-acetoxymethylester, IR (Methylenchlorid): 3620; 1795; 1765; 1725; 1670 $cm^{-1}$.

(5R,6S)-2-(Pyrazol-1-yl)-6-hydroxymethyl-2-penem-3-carbonsäure-acetoxymethylester, IR (Methylenchlorid): 3605; 1795; 1760; 1720 $cm^{-1}$.

(5R,6S)-2-[4-Methoxyimidazol-1-yl]-6-hydroxymethyl-2-penem-3-carbonsäure-acetoxymethylester, IR (Methylenchlorid): 3625; 1790; 1755; 1730; 1680 $cm^{-1}$.

(5R,6S)-2-[4-Aminomethyl-imidazol-1-yl]-6-hydroxymethyl-2-penem-3-carbonsäure-acetoxymethylester, IR (Methylenchlorid): 1785; 1760; 1725; 1685 $cm^{-1}$.

(5R,6S)-2-[4-Aminomethyl-imidazol-1-yl]-6-hydroxymethyl-2-penem-3-carbonsäure-1-äthoxycarbonyloxyäthylester, IR (Methylenchlorid): 1790; 1755; 1720; 1675 $cm^{-1}$.

(5R,6S)-2-(4,5-Dimethylimidazol-1-yl)-6-[(1R)-1-hydroxyäthyl]-2-penem-3-carbonsäure-acetoxymethylester, IR (Mehlenchlorid): 3615; 1795; 1760; 1720; 1675 $cm^{-1}$.

(5R,6S)-2-(4,5-Dimethylimidazol-1-yl)-6-[(1R)-1-hydroxyäthyl]-2-penem-3-carbonsäure-1-äthoxycarbonyloxyäthylester, IR (Methylenchlorid): 3610; 1785; 1755; 1715; 1665 $cm^{-1}$.

(5R,6S)-2-(4,5-Dimethylimidazol-1-yl)-6-hydroxymethyl-2-penem-3-carbonsäure-acetoxymethylester, IR (Methylenchlorid): 3605; 1790; 1755; 1725; 1685 $cm^{-1}$.

(5R,6S)-2-(4-Aminomethylimidazol-1-yl)-6-[(1R)-1-hydroxyäthyl]-2-penem-3-carbonsäure-acetoxymethylester, IR (Methylenchlorid): 1785; 1760; 1720; 1630 $cm^{-1}$.

(5R,6S)-2-(4-Aminomethylimidazol-1-yl)-6-[(1R)-1-hydroxyäthyl]-2-penem-3-carbonsäure-1-äthoxycarbonyläthylester, IR (Methylenchlorid): 1785; 1755; 1720; 1670 $cm^{-1}$.

(5R,6S)-2-(Imidazol-1-yl)-6-[(1R)-1-hydroxyäthyl]-2-penem-3-carbonsäure-1-äthoxycarbonyloxyäthylester, IR (Methylenchlorid): 3600; 1790; 1760; 1720; 1675 $cm^{-1}$.

Beispiel 47: Trockenampullen oder Vials, enthaltend 0,5 g (5R,6S)-2-(Imidazol-1-yl)-6-[(1'R)-1-hydroxyäthyl]-2-penem-3-carbonsäure-Natriumsalz als Wirksubstanz, werden wie folgt hergestellt:

Zusammensetzung (für 1 Ampulle oder Vial):

| | |
|---|---|
| Wirksubstanz | 0,5 g |
| Mannit | 0,05 g |

Eine sterile wässerige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Anstelle des obengenannten Wirkstoffs kann auch dieselbe Menge eines anderen Wirkstoffs der vorangehenden Beispiele, wie z.B. (5R,6S)-2-(Pyrazol-1-yl)-6-hydroxymethyl-2-penem-3-carbonsäure-Natriumsalz, (5R,6S)-2-(1,2,4-Triazol-1-yl)-6-hydroxymethyl-2-penem-3-carbonsäure-Natriumsalz, (5R,6S)-2-(4-Aminomethylimidazol-1-yl)-6-[(1R)-1-hydroxyäthyl]-2-penem-3-carbonsäure, (5R,6S)-2-(4,5-Dimethylimidazol-1-yl)-6-[(1R)-1-hydroxyäthyl]-2-penem-3-carbonsäure-Natriumsalz oder (5R,6S)-2-[4-Methoxyimidazol-1-yl]-6-hydroxymethyl-2-penem-3-carbonsäure-Natriumsalz, verwendet werden.

Patentansprüche für die Vertragsstaaten: BE, FR, DE, CH, LI, IT, LU, NL, SE, GB.

1. 2-Heterocyclyl-6-hydroxyniederalkyl-2-penem-Verbindungen der Formel

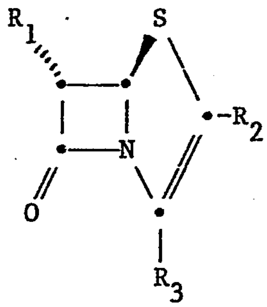

(I),

worin $R_1$ durch Hydroxy oder geschütztes Hydroxy substituiertes Niederalkyl ist, $R_2$ einen über ein Ringstickstoffatom an den Penemrest gebundenen ungesättigten Azaheterocyclylrest darstellt, und $R_3$ Carboxyl oder funktionell abgewandeltes Carboxyl ist, optische Isomere von Verbindungen der Formel I, Mischungen dieser optischen Isomere, und Salze von solchen Verbindungen der Formel I, die eine salzbildende Gruppe aufweisen.

2. Verbindungen der Formel I gemäss Anspruch 1, worin $R_1$ durch Hydroxy oder geschütztes Hydroxy substituiertes Niederalkyl ist, $R_2$ ein über ein Ringstickstoffatom an den Penem-Rest gebundener, gegebenenfalls partiell gesättigter monocyclischer 5-gliedriger Heteroaryl-Rest mit 1-4 Ringstickstoffatomen, z.B. ein entsprechender aza-, diaza-, triaza-oder tetraza-cyclischer Rest aromatischen Charakters oder ein entsprechender Dihydrorest, ein entsprechender partiell gesättigter monocyclischer 6-gliedriger Heteoaryl-Rest mit 1-3 Ringstickstoffatomen, wie ein entsprechender aza-, diaza- oder triaza-cyclischer Rest, oder ein entsprechendes gegebenenfalls partiell gesättigtes Benzo-, Dibenzo-, Pyrido- oder Pyrimido-Derivat eines solchen 5- oder 6-gliedrigen Restes ist, wobei diese Reste unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkanoyloxyniederalkyl, Niederalkoxyniederalkyl, Carboxyniederalkyl, Niederalkoxycarbonylniederalkyl, Carbamoylniederalkyl, Carbamoyloxyniederalkyl, Halogenniederalkyl, Niederalkylthioniederalkyl, gegebenenfalls N-niederalkyliertes Aminoniederalkyl, Niederalkanoylaminoniederalkyl, Amino-carboxyniederalkyl, Amino-niederalkoxycarbonyl-niederalkyl, Sulfoniederalkyl, Amino, Niederalkylamino, Diniederalkylamino, Niederalkylenamino, Niederalkanoylamino, Carboxyl, Niederalkoxycarbonyl, Carbamoyl, N-mono- oder N,N-diniederalkyliertes Carbamoyl, Cyano, Sulfo, Sulfamoyl, gegebenenfalls durch Niederalkyl, Nitro, Niederalkoxy und/oder Halogen substituiertes Phenyl, Cycloalkyl, Nitro, Imino, Oxo und/oder Oxido substituiert sind, $R_3$ Carboxyl, unter physiologischen Bedingungen spaltbares verestertes Carboxyl oder geschütztes Carboxyl $R_3'$ bedeutet, optische Isomere von Verbindungen der Formel I, Mischungen dieser optischen Isomere und Salze von Verbindungen der Formel I, die eine salzbildende Gruppe aufweisen.

3. Verbindungen der Formel I gemäss Anspruch 1, worin $R_1$ und $R_3$ die in Anspruch 2 angegebenen Bedeutungen haben und $R_2$ einer der in Anspruch 2 angegebenen Reste ist, welcher unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkoxyniederalkyl, Carboxyniederalkyl, gegebenenfalls N-niederalkyliertes Aminoniederalkyl, Sulfoniederalkyl, Amino, Niederalkylamino, Diniederalkylamino, Niederalkylenamino, Niederalkanoylamino, Carboxyl, Niederalkoxycarbonyl, Carbamoyl, N-mono- oder N,N-diniederalkyliertes Carbamoyl, Cyano, Sulfo, Sulfamoyl, gegebenenfalls durch Niederalkyl, Nitro, Niederalkoxy und/oder Halogen substituiertes Phenyl, Cycloalkyl, Nitro, Imino, Oxo und/oder Oxido substituiert ist, optische Isomere von Verbindungen der Formel I, Mischungen dieser optischen Isomere und Salze von Verbindungen der Formel I, die eine salzbildende Gruppe aufweisen.

4. Verbindungen der Formel I gemäss Anspruch 2, worin $R_1$ durch Hydroxy oder Triniederalkylsilyloxy substituiertes Niederalkyl ist, $R_2$ gegebenenfalls durch Niederalkyl oder Halogen substituiertes 1-Pyrrolyl oder Dihydro-1-pyrrolyl, gegebenenfalls durch Niederalkyl, Niederalkoxy, Aminoniederalkyl, N-niederalkyliertes Aminoniederalkyl, Niederalkanoylaminoniederalkyl, Amino-carboxy-niederalkyl, Amino-niederalkoxycarbonyl-niederalkyl, Carboxyniederalkyl, Niederalkoxycarbonylniederalkyl, Carbamoylniederalkyl, Carbamoyloxyniederalkyl, Hydroxyniederalkyl, Niederalkanoyloxyniederalkyl, Halogenniederalkyl, Sulfoniederalkyl, Niederalkylthioniederalkyl, Amino, Niederalkylamino, Diniederalkylamino, Niederalkanoylamino oder Nitro substituiertes Imidazol-1-yl, gegebenenfalls durch Niederalkyl, Aminoniederalkyl, Amino-carboxy-niederalkyl, Amino oder Nitro substituiertes Pyrazol-1-yl, gegebenenfalls durch Niederalkyl, Carboxyniederalkyl oder Phenyl substituiertes 1,2,3-, 1,2,4- oder 1,3,4-Triazol-1-yl, gegebenenfalls durch Niederalkyl, Carboxyniederalkyl, Sulfoniederalkyl, Diniederalkylaminoniederalkyl, Amino oder gegebenenfalls durch Halogen substituiertes Phenyl substituiertes 1- oder 2-Tetrazolyl, unsubstituiertes oder durch Oxo und gegebenenfalls zusätzlich durch Halogen substituiertes Dihydro-1-pyridyl, unsubstituiertes oder durch Oxo und gegebenenfalls zusätzlich durch Niederalkyl, Amino, Diniederalkylamino und/oder Carboxy substituiertes Dihydro- oder Tetrahydro-1-pyrimidyl, gegebenenfalls durch Niederalkyl, Niederalkoxy, Amino und/oder bis zu 2 Oxogruppen substituiertes Dihydro- oder Tetrahydro-1,2,4- oder -1,3,6-triazin-1-yl, Indol-1-yl, Benzimidazol-1-yl, Benzotriazol-1-yl, Pyrido-pyrrol-1-yl, Pyrido-imidazol-1-yl, gegebenenfalls durch Amino, Imino und/oder Oxo substituiertes Pyrimido-, Dihydropyrimido- oder Tetrahydropyrimido-imidazol-1-yl, oder gegebenenfalls durch Amino, Imino und/oder Oxo substituiertes Pyrimido-, Dihydropyrimido- oder Tetrahydropyrimido-1,2,3-triazol-1-yl ist, und $R_3$ Carboxyl, 4-Nitrobenzyloxycarbonyl, Niederalkenyloxycarbonyl, in 2-Stellung durch Niederalkylsulfonyl, Cyano oder Triniederalkylsilyl substituiertes Aethoxycarbonyl oder eine unter physiologischen Bedingungen spaltbare veresterte Carboxygruppe darstellt, optische Isomere von Verbindungen der Formel I, Mischungen dieser optischen Isomere und Salze von solchen Verbindungen der Formel I, die eine salzbildende Gruppe aufweisen.

5. Verbindungen der Formel I gemäss Anspruch 3, worin $R_1$ und $R_3$ die in Anspruch 4 angegebenen Bedeutungen haben und $R_2$ gegebenenfalls durch Niederalkyl oder Halogen substituiertes 1-Pyrrolyl oder

Dihydro-1-pyrrolyl, gegebenenfalls durch Niederalkyl, Aminoniederalkyl, Amino oder Nitro substituiertes Imidazol-1-yl oder Pyrazol-1-yl, gegebenenfalls durch Niederalkyl, Carboxyniederalkyl oder Phenyl substituiertes 1,2,3-, 1,2,4- oder 1,3,4-Triazol-1-yl, gegebenenfalls durch Niederalkyl, Carboxyniederalkyl, Sulfoniederalkyl, Diniederalkylaminoniederalkyl, Amino oder gegebenenfalls durch Halogen substituiertes Phenyl substituiertes 1- oder 2-Tetrazolyl, unsubstituiertes oder durch Oxo und gegebenenfalls zusätzlich durch Halogen substituiertes Dihydro-1-pyridyl, unsubstituiertes oder durch Oxo und gegebenenfalls zusätzlich durch Niederalkyl, Amino, Diniederalkylamino und/oder Carboxy substituiertes Dihydro- oder Tetrahydro-1-pyrimidyl, gegebenenfalls durch Niederalkyl, Niederalkoxy, Amino und/oder bis zu 2 Oxogruppen substituiertes Dihydro- oder Tetrahydro-1,2,4- oder -1,3,6-triazin-1-yl; Indol-1-yl, Benzimidazol-1-yl, Benzotriazol-1-yl, Pyrido-pyrrol-1-yl, Pyrido-imidazol-1-yl, gegebenenfalls durch Amino, Imino und/oder Oxo substituiertes Pyrimido-, Dihydropyrimido-oder Tetrahydropyrimido-imidazol-1-yl, oder gegebenenfalls durch Amino, Imino und/oder Oxo substituiertes Pyrimido-, Dihydropyrimido-oder Tetrahydropyrimido-1,2,3-triazol-1-yl ist, optische Isomere von Verbindungen der Formel I, Mischungen dieser optischen Isomere und Salze von solchen Verbindungen der Formel I, die eine salzbildende Gruppe aufweisen.

6. Verbindungen der Formel I gemäss Anspruch 2, worin $R_1$ in α-Stellung durch Hydroxy substituiertes Niederalkyl ist, $R_2$ unsubstituiertes oder durch Niederalkyl oder Halogen substituiertes Pyrrol-1-yl, unsubstituiertes oder durch Niederalkyl oder Aminoniederalkyl substituiertes Pyrazol-1-yl, unsubstituiertes oder durch Niederalkyl, Niederalkoxy, Aminoniederalkyl, Niederalkanoylaminoniederalkyl, Amino-carboxy-niederalkyl, Amino-niederalkoxycarbonyl-niederalkyl, Carboxyniederalkyl, Niederalkoxycarbonylniederalkyl, Carbamoylniederalkyl, Carbamoyloxyniederalkyl, Hydroxyniederalkyl, Niederalkanoyloxyniederalkyl, Halogenniederalkyl, Niederalkylthioniederalkyl, Amino oder Nitro substituiertes Imidazol-1-yl, unsubstituiertes oder durch Niederalkyl substituiertes 1,2,4- oder 1,3,4-Triazol-1-yl, unsubstituiertes oder durch Niederalkyl, Amino oder Phenyl substituiertes 1- oder 2-Tetrazolyl; Indol-1-yl,

Benzimidazol-1-yl, Benzotriazol-1-yl, 1H-Pyrrolo(2,3-b)pyrid-1-yl, 1H-Imidazo(4,5-b)pyrid-1-yl oder Purin-1-yl ist, und $R_3$ Carboxyl oder unter physiologischen Bedingungen spaltbares verestertes Carboxyl, wie Niederalkanoyloxymethoxycarbonyl oder 1-Niederalkoxycarbonyloxy-niederalkoxycarbonyl, bedeutet, optische Isomere von Verbindungen der Formel I, Mischungen dieser optischen Isomere und Salze, insbesondere pharmazeutisch annehmbare Salze von solchen Verbindungen der Formel I, die eine salzbildende Gruppe enthalten.

7. Verbindungen der Formel I gemäss Anspruch 3, worin $R_1$ in α-Stellung durch Hydroxy oder Triniederalkylsilyloxy substituiertes Niederalkyl ist, $R_2$ unsubstituiertes oder durch Niederalkyl oder Halogen substituiertes Pyrrol-1-yl, unsubstituiertes oder durch Niederalkyl, Aminoniederalkyl, Amino oder Nitro substituiertes Imidazol-1-yl, Pyrazol-1-yl, unsubstituiertes oder durch Niederalkyl substituiertes 1,2,4- oder 1,3,4-Triazol-1-yl, unsubstituiertes oder durch Niederalkyl, Amino oder Phenyl substituiertes 1- oder 2-Tetrazolyl; Indol-1-yl, Benzimidazol-1-yl, Benzotriazol-1-yl, 1H-Pyrrolo-(2,3-b)pyrid-1-yl, 1H-Imidazo(4,5-b)pyrid-1-yl oder Purin-1-yl ist, und $R_3$ Carboxyl, Niederalkenyloxycarbonyl oder unter physiologischen Bedingungen spaltbares verestertes Carboxyl, wie Niederalkanoyloxymethoxycarbonyl oder 1-Niederalkoxycarbonyloxy-niederalkoxycarbonyl, bedeutet, optische Isomere von Verbindungen der Formel I, Mischungen dieser optischen Isomere und Salze von solchen Verbindungen der Formel I, die eine salzbildende Gruppe enthalten.

8. Verbindungen der Formel I gemäss Anspruch 2, worin $R_1$ Hydroxymethyl oder 1-Hydroxyäthyl ist, $R_2$ Pyrrol-1-yl, unsubstituiertes oder durch Niederalkyl, Niederalkoxy, Amino oder Aminoniederalkyl substituiertes Imidazol-1-yl, unsubstituiertes oder durch Niederalkyl oder Aminoniederalkyl substituiertes Pyrazol-1-yl, 1,2,4-Triazol-1-yl oder unsubstituiertes oder durch Amino substituiertes Tetrazol-1-yl bedeutet, und $R_3$ Carboxyl oder unter physiologischen Bedingungen spaltbares verestertes Carboxyl, wie Niederalkanoyloxymethoxycarbonyl oder 1-Niederalkoxycarbonyloxy-niederalkoxycarbonyl, bedeutet, optische Isomere, insbesondere das (1R)-Isomere von

Verbindungen der Formel I, worin $R_1$ 1-Hydroxyäthyl ist, Mischungen dieser optischen Isomere und Salze, insbesondere pharmazeutisch annehmbare Salze von Verbindungen der Formel I.

9. Verbindungen der Formel I gemäss Anspruch 3, worin $R_1$ Hydroxymethyl oder 1-Hydroxyäthyl ist, $R_2$ Pyrrol-1-yl, unsubstituiertes oder durch Amino oder Aminoniederalkyl substituiertes Imidazol-1-yl, Pyrazol-1-yl, 1,2,4-Triazol-1-yl, unsubstituiertes oder durch Amino substituiertes Tetrazol-1-yl; Benzimidazol-1-yl oder 1H-Pyrrolo-(2,3-b)pyrid-1-yl bedeutet, und $R_3$ Carboxyl ist, optische Isomere, insbesondere das (1R)-Isomere von Verbindungen der Formel I, worin $R_1$ 1-Hydroxyäthyl ist, Mischungen dieser optischen Isomere und Salze von solchen Verbindungen der Formel I.

10. Verbindungen der Formel I gemäss Anspruch 2, worin $R_1$ Hydroxymethyl oder (1R)-1-Hydroxyäthyl ist, $R_2$ unsubstituiertes oder durch Niederalkyl oder Aminoniederalkyl substituiertes Imidazol-1-yl bedeutet, und $R_3$ Carboxyl oder unter physiologischen Bedingungen spaltbares verestertes Carboxyl, wie Niederalkanoyloxymethoxycarbonyl oder 1-Niederalkoxycarbonyloxy-niederalkoxycarbonyl, ist, und Salze, insbesondere pharmazeutisch annehmbare Salze von Verbindungen der Formel I.

11. Verbindungen der Formel I gemäss Anspruch 3, worin $R_1$ Hydroxymethyl oder (1R)-1-Hydroxyäthyl ist, $R_2$ Imidazol-1-yl, Pyrazol-1-yl oder 1,2,4-Triazol-1-yl bedeutet, und $R_3$ Carboxyl ist, und Salze von solchen Verbindungen der Formel I.

12. Pharmazeutisch annehmbare Salze von Verbindungen der Formel (I) gemäss Anspruch 2.

13. Pharmazeutisch annehmbare Salze von Verbindungen der Formel (I) gemäss Anspruch 3.

14. Unter physiologischen Bedingungen spaltbare Ester von Verbindungen der Formel I gemäss Anspruch 3.

15. Unter physiologischen Bedingungen spaltbare Ester von Verbindungen der Formel I gemäss Anspruch 2.

16. (5R,6S)-2-(Imidazol-1-yl)-6-hydroxymethyl-2-penem-3-carbonsäure und pharmazeutisch annehmbare Salze davon gemäss Anspruch 3.

17. (5R,6S)-2-(Imidazol-1-yl)-6-[(1'R)-1-hydroxyäthyl]-2-penem-3-carbonsäure und pharmazeutisch annehmbare Salze davon gemäss Anspruch 3.

18. (5R,6S)-2-(4-Methyl-imidazol-1-yl)-6-hydroxymethyl-2-penem-3-carbonsäure und pharmazeutisch annehmbare Salze davon gemäss Anspruch 3.

19. (5R,6S)-2-(4,5-Dimethylimidazol-1-yl)-6-hydroxymethyl-2-penem-3-carbonsäure und pharmazeutisch annehmbare Salze davon gemäss Anspruch 2.

20. (5R,6S)-2-[4-(2-Aminoäthyl)-imidazol-1-yl]-6-hydroxymethyl-2-penem-3-carbonsäure und pharmazeutisch annehmbare Salze davon gemäss Anspruch 2.

21. (5R,6S)-2-[4-Methoxyimidazol-1-yl]-6-hydroxymethyl-2-penem-3-carbonsäure und pharmazeutisch annehmbare Salze davon gemäss Anspruch 2.

22. (5R,6S)-2-[4-Aminomethyl-imidazol-1-yl]-6-hydroxymethyl-2-penem-3-carbonsäure und pharmazeutisch annehmbare Salze davon gemäss Anspruch 2.

23. (5R,6S)-2-(Imidazol-1-yl)-6-[(1R)-1-hydroxyäthyl]-2-penem-3-carbonsäure-acetoxymethylester gemäss Anspruch 2.

24. Eine Verbindung der Formel I gemäss Anspruch 2 ausgewählt aus der Gruppe
(5R,6S)-2-(1,2,4-Triazol-1-yl)-6-hydroxymethyl-2-penem-3-carbonsäure,

(5R,6S)-2-(Pyrazol-1-yl)-6-hydroxymethyl-2-penem-3-carbonsäure, (5R,6S)-2-(Pyrrol-1-yl)-6-hydroxymethyl-2-penem-3-carbonsäure und (5R,6S)-2-(4-Methyl-pyrazol-1-yl)-6-hydroxymethyl-2-penem-3-carbonsäure.

25. Eine Verbindung gemäss Anspruch 3 und ihre pharmazeutisch annehmbaren Salze zur Anwendung in einem Verfahren zur therapeutischen Behandlung des menschlichen und tierischen Körpers.

26. Eine Verbindung gemäss Anspruch 2 und ihre pharmazeutisch annehmbaren Salze zur Anwendung in einem Verfahren zur therapeutischen Behandlung des menschlichen und tierischen Körpers.

27. Pharmazeutisches Präparat enthaltend eine Verbindung der Formel I gemäss Anspruch 3 oder ein pharmazeutisch annehmbares Salz einer solchen Verbindung mit salzbildenden Gruppen.

28. Pharmazeutisches Präparat enthaltend eine Verbindung der Formel I gemäss Anspruch 2 oder ein pharmazeutisch annehmbares Salz einer solchen Verbindung mit salzbildenden Gruppen.

29. Verwendung von Verbindungen der Formel I gemäss Anspruch 2 und von pharmazeutisch annehmbaren Salzen von solchen Verbindungen mit salzbildenden Gruppen zur Herstellung von pharmazeutischen Präparaten.

30. Verwendung von Verbindungen der Formel I gemäss Anspruch 3 und von pharmazeutisch annehmbaren Salzen von solchen Verbindungen mit salzbildenden Gruppen zur Herstellung von pharmazeutischen Präparaten.

31. Verbindungen der Formel I und ihre pharmazeutisch annehmbaren Salze gemäss Anspruch 3 als antibiotische Mittel.

32. Verbindungen der Formel I und ihre pharmazeutisch annehmbaren Salze gemäss Anspruch 2 als antibiotische Mittel.

33. Verfahren zur Herstellung von Verbindungen der Formel I gemäss Anspruch 1, dadurch gekennzeichnet, dass man

a. eine Ylid-Verbindung der Formel

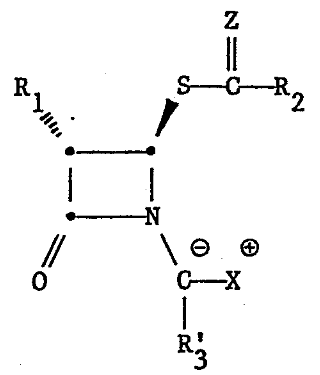

(II),

worin $R_1$ und $R_2$ die unter Formel I angegebenen Bedeutungen haben und $R_3'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b. eine Verbindung der Formel

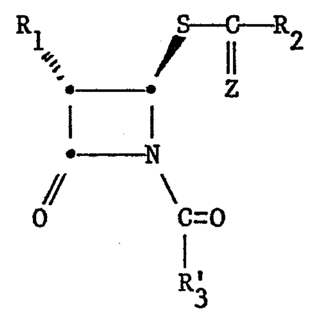

(III),

worin $R_1$ und $R_2$ die unter Formel I angegebenen Bedeutungen haben, Z die unter Formel II angegebene Bedeutung hat und $R_3'$ eine geschützte Carboxylgruppe darstellt, mit einer organischen Verbindung des dreiwertigen Phosphors behandelt, oder

c. eine Verbindung der Formel

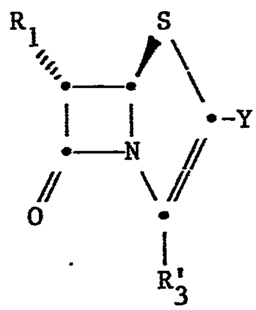

(X),

worin $R_1$ die unter Formel I angegebene Bedeutung hat, $R_3'$ eine geschützte Carboxylgruppe darstellt und Y eine durch nucleophile Reaktion austauschbare Gruppe darstellt, mit einer den Rest $R_2$ einführenden Verbindung umsetzt

und, wenn erwünscht oder notwendig, in einer erhältlichen Verbindung der Formel I eine geschützte Hydroxygruppe im Rest $R_1$ in die freie Hydroxygruppe überführt, und/oder, wenn erwünscht, in einer erhältlichen Verbindung der Formel I eine geschützte Carboxylgruppe $R_3'$ in die freie, in eine unter physiologischen Bedingungen spaltbare veresterte Carboxylgruppe oder in eine andere geschützte Carboxylgruppe $R_3'$ oder eine freie Carboxylgruppe $R_3$ in eine unter physiologischen Bedingungen spaltbare veresterte Carboxylgruppe oder in eine geschützte Carboxylgruppe $R_3'$ überführt, und/oder, wenn erwünscht weitere im Rest $R_2$ enthaltene geschützte funktionelle Gruppen in die freien funktionellen Gruppen überführt, und/oder, wenn erwünscht in einer erhältlichen Verbindung der Formel I einen Rest $R_2$ in einen anderen Rest $R_2$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

34. Verfahren zur Herstellung von Verbindungen der Formel I gemäss Anspruch 1, dadurch gekennzeichnet, dass man

a. eine Ylid-Verbindung der Formel

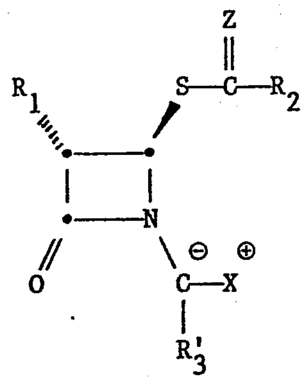

(II),

worin $R_1$ und $R_2$ die in Anspruch 1 angegebenen Bedeutungen haben, $R_3'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b. eine Verbindung der Formel

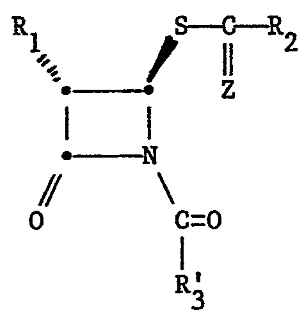

(III),

worin $R_1$ und $R_2$ die in Anspruch 1 angegebenen Bedeutungen haben, Z Schwefel ist und $R_3'$ eine geschützte Carboxylgruppe darstellt, mit einer organischen Verbindung des dreiwertigen Phosphors behandelt, und, wenn erwünscht oder notwendig, in einer erhältlichen Verbindung der Formel I eine geschützte Hydroxygruppe im Rest $R_1$ in die freie Hydroxygruppe überführt, und/oder, wenn erwünscht, in einer erhältlichen Verbindung der Formel I eine geschützte Carboxylgruppe $R_3'$ in die freie, in eine unter physiologischen Bedingungen spaltbare veresterte Carboxylgruppe oder in eine andere geschützte Carboxylgruppe $R_3'$ überführt, und/oder, wenn erwünscht weitere im Rest $R_2$ enthaltene geschützte funktionelle Gruppen in die freien funktionellen Gruppen überführt, und/oder, wenn erwünscht in einer erhältlichen Verbindung der Formel I einen Rest $R_2$ in einen anderen Rest

$R_2$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

35. Die nach dem Verfahren gemäss Anspruch 33 erhältlichen Verbindungen.

36. Die nach dem Verfahren gemäss Anspruch 34 erhältlichen Verbindungen.

Patentansprüche für den Vertragsstaat: AT

1. Verfahren zur Herstellung von Verbindungen der Formel

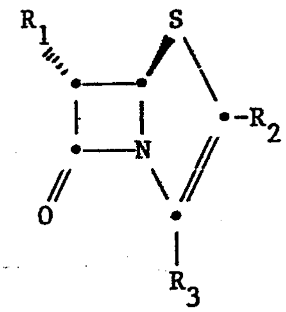

(I),

worin $R_1$ durch Hydroxy oder geschütztes Hydroxy substituiertes Niederalkyl ist, $R_2$ einen über ein Ringstickstoffatom an den Penemrest gebundenen ungesättigten Azaheterocyclylrest darstellt, und $R_3$ Carboxyl oder funktionell abgewandeltes Carboxyl ist, optischen Isomeren von Verbindungen der Formel I, Mischungen dieser optischen Isomeren, und Salzen von solchen Verbindungen der Formel I, die eine salzbildende Gruppe aufweisen, dadurch gekennzeichnet, dass man

a. eine Ylid-Verbindung der Formel

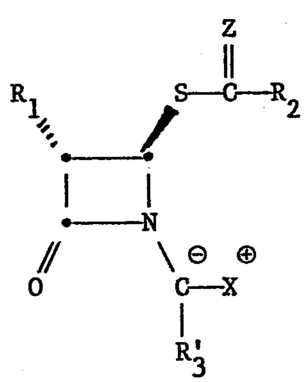

(II),

worin $R_1$ und $R_2$ die unter Formel I angegebenen Bedeutungen haben und $R_3'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b. eine Verbindung der Formel

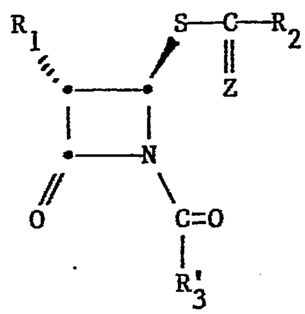

(III),

worin $R_1$ und $R_2$ die unter Formel I angegebenen Bedeutungen haben, Z die unter Formel II angegebene Bedeutung hat und $R_3'$ eine geschützte Carboxylgruppe darstellt, mit einer organischen Verbindung des dreiwertigen Phosphors behandelt, oder

c. eine Verbindung der Formel

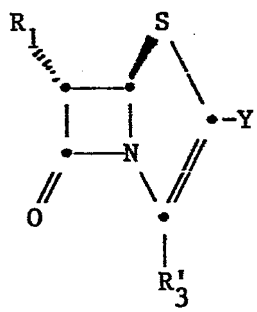

(X),

worin $R_1$ die unter Formel I angegebene Bedeutung hat, $R_3'$ eine geschützte Carboxylgruppe darstellt und Y eine durch nucleophile Reaktion austauschbare Gruppe darstellt, mit einer den Rest $R_2$ einführenden Verbindung umsetzt

und, wenn erwünscht oder notwendig, in einer erhältlichen Verbindung der Formel I eine geschützte Hydroxygruppe im Rest $R_1$ in die freie Hydroxygruppe überführt, und/oder, wenn erwünscht, in einer erhältlichen Verbindung der Formel I eine geschützte Carboxylgruppe $R_3'$ in die freie, in eine unter physiologischen Bedingungen spaltbare veresterte Carboxylgruppe oder in eine andere geschützte Carboxylgruppe $R_3'$ oder eine freie Carboxylgruppe $R_3$ in eine unter physiologischen Bedingungen spaltbare veresterte Carboxylgruppe oder in eine geschützte Carboxylgruppe $R_3'$ überführt, und/oder, wenn erwünscht

weitere im Rest $R_2$ enthaltene geschützte funktionelle Gruppen in die freien funktionellen Gruppen überführt, und/oder, wenn erwünscht in einer erhältlichen Verbindung der Formel I einen Rest $R_2$ in einen anderen Rest $R_2$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

2. Verfahren gemäss Anspruch 1 zur Herstellung von Verbindungen der Formel I, worin $R_1$, $R_2$ und $R_3$ die in Anspruch 1 angegebenen Bedeutungen haben, dadurch gekennzeichnet, dass man

a. eine Ylid-Verbindung der Formel

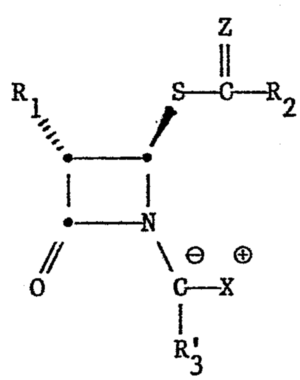

(II),

worin $R_1$ und $R_2$ die in Anspruch 1 angegebenen Bedeutungen haben, $R_3'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b. eine Verbindung der Formel

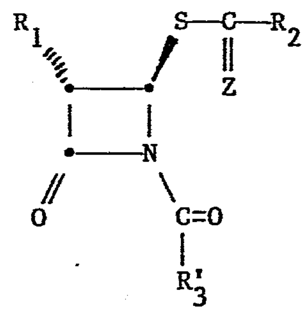

(III),

worin $R_1$ und $R_2$ die in Anspruch 1 angegebenen Bedeutungen haben, Z Schwefel ist und $R_3'$ eine geschützte Carboxylgruppe darstellt, mit einer organischen Verbindung des dreiwertigen Phosphors behandelt, und, wenn erwünscht oder notwendig, in einer erhältlichen Verbindung der Formel I eine geschützte Hydroxygruppe im Rest $R_1$ in die freie Hydroxygruppe überführt, und/oder, wenn erwünscht, in einer erhältlichen Verbindung der Formel I eine geschützte Carboxylgruppe $R_3'$ in die freie, in eine unter physiologischen Bedingungen spaltbare veresterte Carboxylgruppe oder in eine andere geschützte Carboxylgruppe $R_3'$ überführt, und/oder, wenn erwünscht weitere im Rest $R_2$ enthaltene geschützte funktionelle Gruppen in die freien funktionellen Gruppen überführt, und/oder, wenn erwünscht in einer erhältlichen Verbindung der Formel I einen Rest $R_2$ in einen anderen Rest $R_2$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

3. Verfahren zur Herstellung von Verbindungen der Formel I gemäss Anspruch 1, worin $R_1$ durch Hydroxy oder geschütztes Hydroxy substituiertes Niederalkyl ist, $R_2$ ein über ein Ringstickstoffatom an den Penem-Rest gebundener, gegebenenfalls partiell gesättigter monocyclischer 5-gliedriger Heteroaryl-Rest mit 1-4 Ringstickstoffatomen, z.B. ein entsprechender aza-, diaza-, triaza-oder tetrazacyclischer Rest aromatischen Charakters oder ein entsprechender Dihydrorest, ein entsprechender partiell gesättigter monocyclischer 6-gliedriger Heteoaryl-Rest mit 1-3 Ringstickstoffatomen, wie ein

entsprechender aza-, diaza- oder triaza-cyclischer Rest, oder ein entsprechendes gegebenenfalls partiell gesättigtes Benzo-, Dibenzo-, Pyrido- oder Pyrimido-Derivat eines solchen 5- oder 6-gliedrigen Restes ist, wobei diese Reste unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkanoyloxyniederalkyl, Niederalkoxyniederalkyl, Carboxyniederalkyl, Niederalkoxycarbonylniederalkyl, Carbamoylniederalkyl, Carbamoyloxyniederalkyl, Halogenniederalkyl, Niederalkylthioniederalkyl, gegebenenfalls N-niederalkyliertes Aminoniederalkyl, Niederalkanoylaminoniederalkyl, Amino-carboxy-niederalkyl, Amino-niederalkoxycarbonyl-niederalkyl, Sulfoniederalkyl, Amino, Niederalkylamino, Diniederalkylamino, Niederalkylenamino, Niederalkanoylamino, Carboxyl, Niederalkoxycarbonyl, Carbamoyl, N-mono- oder N,N-diniederalkyliertes Carbamoyl, Cyano, Sulfo, Sulfamoyl, gegebenenfalls durch Niederalkyl, Nitro, Niederalkoxy und/oder Halogen substituiertes Phenyl, Cycloalkyl, Nitro, Imino, Oxo und/oder Oxido substituiert sind, $R_3$ Carboxyl, unter physiologischen Bedingungen spaltbares verestertes Carboxyl oder geschütztes Carboxyl $R_3'$ bedeutet, optischen Isomeren von Verbindungen der Formel I, Mischungen dieser optischen Isomeren und Salzen von Verbindungen der Formel I, die eine salzbildende Gruppe aufweisen.

4. Verfahren zur Herstellung von Verbindungen der Formel I gemäss Anspruch 2, worin $R_1$ und $R_3$ die in Anspruch 3 angegebenen Bedeutungen haben und $R_2$ einer der im Anspruch 3 angegebenen Reste ist, welcher unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkoxyniederalkyl, Carboxyniederalkyl, gegebenenfalls N-niederalkyliertes Aminoniederalkyl, Sulfoniederalkyl, Amino, Niederalkylamino, Diniederalkylamino, Niederalkylenamino, Niederalkanoylamino, Carboxyl, Niederalkoxycarbonyl, Carbamoyl, N-mono- oder N,N-diniederalkyliertes Carbamoyl, Cyano, Sulfo, Sulfamoyl, gegebenenfalls durch Niederalkyl, Nitro, Niederalkoxy und/oder Halogen substituiertes Phenyl, Cycloalkyl, Nitro, Imino, Oxo und/oder Oxido substituiert ist, optischen

Isomeren von Verbindungen der Formel I, Mischungen dieser optischen Isomeren und Salzen von Verbindungen der Formel I, die eine salzbildende Gruppe aufweisen.

5. Verfahren zur Herstellung von Verbindungen der Formel I gemäss Anspruch 1, worin $R_1$ durch Hydroxy oder Triniederalkylsilyloxy substituiertes Niederalkyl ist, $R_2$ gegebenenfalls durch Niederalkyl oder Halogen substituiertes 1-Pyrrolyl oder Dihydro-1-pyrrolyl, gegebenenfalls durch Niederalkyl, Niederalkoxy, Aminoniederalkyl, N-niederalkyliertes Aminoniederalkyl, Niederalkanoylaminoniederalkyl, Amino-carboxy-niederalkyl, Amino-niederalkoxycarbonyl-niederalkyl, Carboxyniederalkyl, Niederalkoxycarbonylniederalkyl, Carbamoylniederalkyl, Carbamoyloxyniederalkyl, Hydroxyniederalkyl, Niederalkanoyloxyniederalkyl, Halogenniederalkyl, Sulfoniederalkyl, Niederalkylthioniederalkyl, Amino, Niederalkylamino, Diniederalkylamino, Niederalkanoylamino oder Nitro substituiertes Imidazol-1-yl, gegebenenfalls durch Niederalkyl, Aminoniederalkyl, Amino-carboxyniederalkyl, Amino oder Nitro substituiertes Pyrazol-1-yl, gegebenenfalls durch Niederalkyl, Carboxyniederalkyl oder Phenyl substituiertes 1,2,3-, 1,2,4- oder 1,3,4-Triazol-1-yl, gegebenenfalls durch Niederalkyl, Carboxyniederalkyl, Sulfoniederalkyl, Diniederalkylaminoniederalkyl, Amino oder gegebenenfalls durch Halogen substituiertes Phenyl substituiertes 1-oder 2-Tetrazolyl, unsubstituiertes oder durch Oxo und gegebenenfalls zusätzlich durch Halogen substituiertes Dihydro-1-pyridyl, unsubstituiertes oder durch Oxo und gegebenenfalls zusätzlich durch Niederalkyl, Amino, Diniederalkylamino und/oder Carboxy substituiertes Dihydro- oder Tetrahydro-1-pyrimidyl, gegebenenfalls durch Niederalkyl, Niederalkoxy, Amino und/oder bis zu 2 Oxogruppen substituiertes Dihydro- oder Tetrahydro-1,2,4- oder -1,3,6-triazin-1-yl, Indol-1-yl, Benzimidazol-1-yl, Benzotriazol-1-yl, Pyrido-pyrrol-1-yl, Pyrido-imidazol-1-yl, gegebenenfalls durch Amino, Imino und/oder Oxo substituiertes Pyrimido-, Dihydropyrimido- oder Tetrahydropyrimido-imidazol-1-yl, oder gegebenenfalls durch Amino, Imino und/oder Oxo substituiertes Pyrimido-, Dihydropyrimido- oder Tetrahydropyrimido-1,2,3-triazol-1-yl ist, und $R_3$ Carboxyl, 4-Nitrobenzyloxycarbonyl, Niederalkenyloxycarbonyl, in 2-Stellung durch Niederalkylsulfonyl, Cyano oder

Triniederalkylsilyl substituiertes Aethoxycarbonyl oder eine unter physiologischen Bedingungen spaltbare veresterte Carboxygruppe darstellt, optischen Isomeren von Verbindungen der Formel I, Mischungen dieser optischen Isomeren und Salzen von solchen Verbindungen der Formel I, die eine salzbildende Gruppe aufweisen.

6. Verfahren zur Herstellung von Verbindungen der Formel I gemäss Anspruch 2, worin $R_1$ und $R_3$ die in Anspruch 5 angegebenen Bedeutungen haben und $R_2$ gegebenenfalls durch Niederalkyl oder Halogen substituiertes 1-Pyrrolyl oder Dihydro-1-pyrrolyl, gegebenenfalls durch Niederalkyl, Aminoniederalkyl, Amino oder Nitro substituiertes Imidazol-1-yl oder Pyrazol-1-yl, gegebenenfalls durch Niederalkyl, Carboxyniederalkyl oder Phenyl substituiertes 1,2,3-, 1,2,4- oder 1,3,4-Triazol-1-yl, gegebenenfalls durch Niederalkyl, Carboxyniederalkyl, Sulfoniederalkyl, Diniederalkylaminoniederalkyl, Amino oder gegebenenfalls durch Halogen substituiertes Phenyl substituiertes 1- oder 2-Tetrazolyl, unsubstituiertes oder durch Oxo und gegebenenfalls zusätzlich durch Halogen substituiertes Dihydro-1-pyridyl, unsubstituiertes oder durch Oxo und gegebenenfalls zusätzlich durch Niederalkyl, Amino, Diniederalkylamino und/oder Carboxy substituiertes Dihydro-oder Tetrahydro-1-pyrimidyl, gegebenenfalls durch Niederalkyl, Niederalkoxy, Amino und/oder bis zu 2 Oxogruppen substituiertes Dihydro- oder Tetrahydro-1,2,4- oder -1,3,6-triazin-1-yl; Indol-1-yl, Benzimidazol-1-yl, Benzotriazol-1-yl, Pyridopyrrol-1-yl, Pyrido-imidazol-1-yl, gegebenenfalls durch Amino, Imino und/oder Oxo substituiertes Pyrimido-, Dihydropyrimido-oder Tetrahydropyrimido-imidazol-1-yl, oder gegebenenfalls durch Amino, Imino und/oder Oxo substituiertes Pyrimido-, Dihydropyrimido-oder Tetrahydropyrimido-1,2,3-triazol-1-yl ist, optischen Isomeren von Verbindungen der Formel I, Mischungen dieser optischen Isomeren und Salzen von solchen Verbindungen der Formel I, die eine salzbildende Gruppe aufweisen.

7. Verfahren zur Herstellung von Verbindungen der Formel I gemäss Anspruch 1, worin $R_1$ in $\alpha$-Stellung durch Hydroxy substituiertes Niederalkyl ist, $R_2$ unsubstituiertes oder durch Niederalkyl oder Halogen substituiertes Pyrrol-1-yl, unsubstituiertes oder durch

Niederalkyl oder Aminoniederalkyl substituiertes Pyrazol-1-yl, unsubstituiertes oder durch Niederalkyl, Niederalkoxy, Aminoniederalkyl, Niederalkanoylaminoniederalkyl, Amino-carboxy-niederalkyl, Amino-niederalkoxycarbonyl-niederalkyl, Carboxyniederalkyl, Niederalkoxycarbonylniederalkyl, Carbamoylniederalkyl, Carbamoyloxyniederalkyl, Hydroxyniederalkyl, Niederalkanoyloxyniederalkyl, Halogenniederalkyl, Niederalkylthioniederalkyl, Amino oder Nitro substituiertes Imidazol-1-yl, unsubstituiertes oder durch Niederalkyl substituiertes 1,2,4- oder 1,3,4-Triazol-1-yl, unsubstituiertes oder durch Niederalkyl, Amino oder Phenyl substituiertes 1- oder 2-Tetrazolyl; Indol-1-yl, Benzimidazol-1-yl, Benzotriazol-1-yl, 1H-Pyrrolo(2,3-b)-pyrid-1-yl, 1H-Imidazo(4,5-b)pyrid-1-yl oder Purin-1-yl ist, und $R_3$ Carboxyl oder unter physiologischen Bedingungen spaltbares verestertes Carboxyl, wie Niederalkanoyloxymethoxycarbonyl oder 1-Niederalkoxycarbonyloxy-niederalkoxycarbonyl, bedeutet, optischen Isomeren von Verbindungen der Formel I, Mischungen dieser optischen Isomeren und Salzen von solchen Verbindungen der Formel I, die eine salzbildende Gruppe enthalten.

8. Verfahren zur Herstellung von Verbindungen der Formel I gemäss Anspruch 2, worin $R_1$ in α-Stellung durch Hydroxy oder Triniederalkylsilyloxy substituiertes Niederalkyl ist, $R_2$ unsubstituiertes oder durch Niederalkyl oder Halogen substituiertes Pyrrol-1-yl, unsubstituiertes oder durch Niederalkyl, Aminoniederalkyl, Amino oder Nitro substituiertes Imidazol-1-yl, Pyrazol-1-yl, unsubstituiertes oder durch Niederalkyl substituiertes 1,2,4- oder 1,3,4-Triazol-1-yl, unsubstituiertes oder durch Niederalkyl, Amino oder Phenyl substituiertes 1- oder 2-Tetrazolyl; Indol-1-yl, Benzimidazol-1-yl, Benzotriazol-1-yl, 1H-Pyrrolo(2,3-b)pyrid-1-yl, 1H-Imidazo(4,5-b)pyrid-1-yl oder Purin-1-yl ist, und $R_3$ Carboxyl, Niederalkenyloxycarbonyl oder unter physiologischen Bedingungen spaltbares verestertes Carboxyl, wie Niederalkanoyloxymethoxycarbonyl oder 1-Niederalkoxycarbonyloxy-niederalkoxycarbonyl, bedeutet, optischen Isomeren von Verbindungen der Formel I, Mischungen dieser optischen Isomeren und Salzen von solchen Verbindungen der Formel I, die eine salzbildende Gruppe enthalten.

9. Verfahren zur Herstellung von Verbindungen der Formel I gemäss Anspruch 1, worin $R_1$ Hydroxymethyl oder 1-Hydroxyäthyl ist, $R_2$ Pyrrol-1-yl, unsubstituiertes oder durch Niederalkyl, Niederalkoxy, Amino oder Aminoniederalkyl substituiertes Imidazol-1-yl, unsubstituiertes oder durch Niederalkyl oder Aminoniederalkyl substituiertes Pyrazol-1-yl, 1,2,4-Triazol-1-yl oder unsubstituiertes oder durch Amino substituiertes Tetrazol-1-yl bedeutet, und $R_3$ Carboxyl oder unter physiologischen Bedingungen spaltbares verestertes Carboxyl, wie Niederalkanoyloxymethoxycarbonyl oder 1-Niederalkoxycarbonyloxy-niederalkoxycarbonyl, bedeutet, optischen Isomeren, insbesondere der (1R)-Isomeren von Verbindungen der Formel I, worin $R_1$ 1-Hydroxyäthyl ist, Mischungen dieser optischen Isomeren und Salzen von Verbindungen der Formel I.

10. Verfahren zur Herstellung von Verbindungen der Formel I gemäss Anspruch 2, worin $R_1$ Hydroxymethyl oder 1-Hydroxyäthyl ist, $R_2$ Pyrrol-1-yl, unsubstituiertes oder durch Amino oder Aminoniederalkyl substituiertes Imidazol-1-yl, Pyrazol-1-yl, 1,2,4-Triazol-1-yl, unsubstituiertes oder durch Amino substituiertes Tetrazol-1-yl; Benzimidazol-1-yl oder 1H-Pyrrolo(2,3-b)pyrid-1-yl bedeutet, und $R_3$ Carboxyl ist, optischen Isomeren, insbesondere der (1R)-Isomeren von Verbindungen der Formel I, worin $R_1$ 1-Hydroxyäthyl ist, Mischungen dieser optischen Isomere und Salze von solchen Verbindungen der Formel I.

11. Verfahren zur Herstellung von Verbindungen der Formel I gemäss Anspruch 1, worin $R_1$ Hydroxymethyl oder (1R)-1-Hydroxyäthyl ist, $R_2$ unsubstituiertes oder durch Niederalkyl oder Aminoniederalkyl substituiertes Imidazol-1-yl bedeutet, und $R_3$ Carboxyl oder unter physiologischen Bedingungen spaltbares verestertes Carboxyl, wie Niederalkanoyloxymethoxycarbonyl oder 1-Niederalkoxycarbonyloxy-niederalkoxycarbonyl, ist, und Salzen von Verbindungen der Formel I.

12. Verfahren zur Herstellung von Verbindungen der Formel I gemäss Anspruch 2, worin $R_1$ Hydroxymethyl oder (1R)-1-Hydroxyäthyl ist, $R_2$ Imidazol-1-yl, Pyrazol-1-yl oder 1,2,4-Triazol-1-yl bedeutet, und $R_3$ Carboxyl ist, und Salzen von solchen Verbindungen der Formel I.

13. Verfahren zur Herstellung von pharmazeutisch annehmbaren Salzen von Verbindungen der Formel I gemäss Anspruch 1.

14. Verfahren zur Herstellung von pharmazeutisch annehmbaren Salzen von Verbindungen der Formel I gemäss Anspruch 2.

15. Verfahren zur Herstellung von unter physiologischen Bedingungen spaltbaren Estern von Verbindungen der Formel I gemäss Anspruch 1.

16. Verfahren zur Herstellung von unter physiologischen Bedingungen spaltbaren Estern von Verbindungen der Formel I gemäss Anspruch 2.

17. Verfahren zur Herstellung von (5R,6S)-2-(Imidazol-1-yl)-6-hydroxymethyl-2-penem-3-carbonsäure und pharmazeutisch annehmbaren Salzen davon gemäss Anspruch 2.

18. Verfahren zur Herstellung von (5R,6S)-2-(Imidazol-1-yl)-6-[(1'R)-1-hydroxyäthyl]-2-penem-3-carbonsäure und pharmazeutisch annehmbaren Salzen davon gemäss Anspruch 2.

19. Verfahren zur Herstellung von (5R,6S)-2-(4-Methyl-imidazol-1-yl)-6-hydroxymethyl-2-penem-3-carbonsäure und pharmazeutisch annehmbaren Salzen davon gemäss Anspruch 2.

20. Verfahren zur Herstellung von (5R,6S)-2-(4,5-Dimethylimidazol-1-yl)-6-hydroxymethyl-2-penem-3-carbonsäure und pharmazeutisch annehmbaren Salzen davon gemäss Anspruch 1.

21. Verfahren zur Herstellung von (5R,6S)-2-[4-(2-Aminoäthyl)-imidazol-1-yl]-6-hydroxymethyl-2-penem-3-carbonsäure und pharmazeutisch annehmbaren Salzen davon gemäss Anspruch 1.

22. Verfahren zur Herstellung von (5R,6S)-2-[4-Methoxyimidazol-1-yl]-6-hydroxymethyl-2-penem-3-carbonsäure und pharmazeutisch annehmbaren Salzen davon gemäss Anspruch 1.

23. Verfahren zur Herstellung von (5R,6S)-2-[4-Aminomethyl-imidazol-1-yl]-6-hydroxymethyl-2-penem-3-carbonsäure und pharmazeutisch annehmbaren Salzen davon gemäss Anspruch 1.

24. Verfahren zur Herstellung einer Verbindung der Formel I gemäss Anspruch 1 ausgewählt aus der Gruppe
(5R,6S)-2-(1,2,4-Triazol-1-yl)-6-hydroxymethyl-2-penem-3-carbonsäure,
(5R,6S)-2-(Pyrazol-1-yl)-6-hydroxymethyl-2-penem-3-carbonsäure,
(5R,6S)-2-(Pyrrol-1-yl)-6-hydroxymethyl-2-penem-3-carbonsäure
und (5R,6S)-2-(4-Methyl-pyrazol-1-yl)-6-hydroxymethyl-2-penem-3-carbonsäure.

25. Verfahren zur Herstellung von (5R,6S)-2-(Imidazol-1-yl)-6-[(1R)-1-hydroxyäthyl]-2-penem-3-carbonsäure-acetoxymethylester gemäss Anspruch 1.

26. Verfahren zur Herstellung von pharmazeutischen Präparaten, dadurch gekennzeichnet, dass man eine gemäss Anspruch 1 erhältliche Verbindung der Formel I oder ein pharmazeutisch annehmbares Salz davon mit einem pharmazeutischen Trägermaterial verarbeitet.

27. Verfahren zur Herstellung von pharmazeutischen Präparaten, dadurch gekennzeichnet, dass man eine gemäss Anspruch 2 erhältliche Verbindung der Formel I oder ein pharmazeutisch annehmbares Salz davon mit einem pharmazeutischen Trägermaterial verarbeitet

FO 7.4/UL/gs*/bg*/jt*